# EUROPEAN PATENT APPLICATION

(11) **EP 3 106 520 A1**
(43) Date of publication of application: **21.12.2016**
(21) Application number: 15172495.2
(22) Date of filing: 17.06.2015
(51) Int. Cl.: C12N 15/54, C12N 15/61, C12N 9/12, C12N 9/90, C12P 7/64, C12P 7/48, C12N 1/16, C12N 1/19

(54) **MUTANT YARROWIA STRAIN CAPABLE OF DEGRADING GALACTOSE**

(71) Applicant: INSTITUT NATIONAL DE LA RECHERCHE AGRONOMIQUE, 75007 Paris (FR)
(72) Inventor: The designation of the inventor has not yet been filed
(74) Representative: Gevers & Orès

(57) **Abstract**

The invention relates to a method for obtaining a mutant *Yarrowia* yeast strain capable of growing on D-galactose as sole carbon source, comprising overexpressing in said strain a galactokinase, a galactose-1-phosphate uridyl transferase, an UDP-glucose-4 epimerase and a galactose mutarotase. The invention also relates to a mutant *Yarrowia* strain obtained by said method.

SEQUENCE LISTING

<110>  INSTITUT NATIONAL DE LA RECHERCHE AGRONOMIQUE

<120>  MUTANT YARROWIA STRAIN CAPABLE OF DEGRADING GALACTOSE

<130>  XRN/11-F539_179/EP

<160>  59

<170>  PatentIn version 3.5

<210>  1
<211>  1431
<212>  DNA
<213>  Yarrowia lipolytica

<220>
<221>  CDS
<222>  (1)..(1431)

<400>  1
atg tct atc agc acc ctg ccg ctg gcg aac atc tac gcg gag ccg acc      48
Met Ser Ile Ser Thr Leu Pro Leu Ala Asn Ile Tyr Ala Glu Pro Thr
1               5                   10                  15

aag cag cag ccg cgc tat gat cag ctg act aag cgg ttt tcg gag ctg      96
Lys Gln Gln Pro Arg Tyr Asp Gln Leu Thr Lys Arg Phe Ser Glu Leu
            20                  25                  30

tac gca gtc tcc agc ccg act ttg gtg aga tcg ccg ggc cga gtc aac      144
Tyr Ala Val Ser Ser Pro Thr Leu Val Arg Ser Pro Gly Arg Val Asn
        35                  40                  45

ctc att ggc gag cac att gac tac tcc cac ttc agt gtc atg ccc atg      192
Leu Ile Gly Glu His Ile Asp Tyr Ser His Phe Ser Val Met Pro Met
    50                  55                  60

gcg att gag gat gac gtc atc atg gcc ttc agc gtt ggc ggc gac tcc      240
Ala Ile Glu Asp Asp Val Ile Met Ala Phe Ser Val Gly Gly Asp Ser
65                  70                  75                  80

att gag att gcc aac atg gaa gac gac aag ttc ccg gca gaa aag tac      288
Ile Glu Ile Ala Asn Met Glu Asp Asp Lys Phe Pro Ala Glu Lys Tyr
                85                  90                  95

aac gtt ccg acc gcc gac cag gtg ctc act atc gac cag aca aaa aca      336
Asn Val Pro Thr Ala Asp Gln Val Leu Thr Ile Asp Gln Thr Lys Thr
            100                 105                 110

gtg tgg acc aac tac ttc aaa tgt gga cac aca gtg ggc cga aag cat      384
Val Trp Thr Asn Tyr Phe Lys Cys Gly His Thr Val Gly Arg Lys His
        115                 120                 125

ctg aga gac tct gga gag gag gta aaa ccc gtg gga atc aag gtg ttg      432
Leu Arg Asp Ser Gly Glu Glu Val Lys Pro Val Gly Ile Lys Val Leu
    130                 135                 140

gtc cat gga act gtc ccc gac gcc tgt gga ctg tcc tct tcc gct gcc      480
Val His Gly Thr Val Pro Asp Ala Cys Gly Leu Ser Ser Ser Ala Ala
145                 150                 155                 160

ttt gtg gtg tgc tcc act ctc gtc act ctt ctg tcc cag ggc atg cag 528
Phe Val Val Cys Ser Thr Leu Val Thr Leu Leu Ser Gln Gly Met Gln
                165                 170                 175

gag gtg tca cgt gag ctg ctc acc agg ctc tcc atc acg tgc gag cag 576
Glu Val Ser Arg Glu Leu Leu Thr Arg Leu Ser Ile Thr Cys Glu Gln
            180                 185                 190

ctt att ggt gtc aac acc ggc ggt atg gac cag gct gcc tcc atc tac 624
Leu Ile Gly Val Asn Thr Gly Gly Met Asp Gln Ala Ala Ser Ile Tyr
        195                 200                 205

ggc caa ctc aat cat act ctc ctg gtg gat ttt gct cca acc ctc aag 672
Gly Gln Leu Asn His Thr Leu Leu Val Asp Phe Ala Pro Thr Leu Lys
    210                 215                 220

gcc act cca ttc cag ttc ccc tca acg acc ccc aag acc gtt ttt ctg 720
Ala Thr Pro Phe Gln Phe Pro Ser Thr Thr Pro Lys Thr Val Phe Leu
225                 230                 235                 240

gtt gcc aac acg ctg gtc aag tcc aac aaa cac gaa acc gcg cct cga 768
Val Ala Asn Thr Leu Val Lys Ser Asn Lys His Glu Thr Ala Pro Arg
                245                 250                 255

aac tac aat ctg cgg gtt gtg gag atc act ctg gcc gtc aac gtg ctt 816
Asn Tyr Asn Leu Arg Val Val Glu Ile Thr Leu Ala Val Asn Val Leu
            260                 265                 270

ctg aag agt ctc aag gga gtc acc att cct cag aac gga aac ctc aac 864
Leu Lys Ser Leu Lys Gly Val Thr Ile Pro Gln Asn Gly Asn Leu Asn
        275                 280                 285

aag gga act ctt cac gga ctt atg aag gct gtt gat act gac ctg cac 912
Lys Gly Thr Leu His Gly Leu Met Lys Ala Val Asp Thr Asp Leu His
    290                 295                 300

gga ctt ttg gac ctg gtg gaa act ctg ccc aag gag cct gtg act ctg 960
Gly Leu Leu Asp Leu Val Glu Thr Leu Pro Lys Glu Pro Val Thr Leu
305                 310                 315                 320

caa gag gtg gcc ggc ctg ctg aac gtc tct gag gac gac gtc aag aag 1008
Gln Glu Val Ala Gly Leu Leu Asn Val Ser Glu Asp Asp Val Lys Lys
                325                 330                 335

cag tac ttc att ttc ccc gtc gaa ttc gac act ctg gct ctc tac tcg 1056
Gln Tyr Phe Ile Phe Pro Val Glu Phe Asp Thr Leu Ala Leu Tyr Ser
            340                 345                 350

cgg gcc aag cat gtt gtc aca gag gcc att cga gtc aag gag ttt caa 1104
Arg Ala Lys His Val Val Thr Glu Ala Ile Arg Val Lys Glu Phe Gln
        355                 360                 365

aac ctg ctg tcc aag ggc gag tct gac ccc agg cag ctc ggc aag ctc 1152
Asn Leu Leu Ser Lys Gly Glu Ser Asp Pro Arg Gln Leu Gly Lys Leu
    370                 375                 380

atg aac gag agt caa aag tcg tgc gac cag ctc ttc aac tgc tcc tgt 1200
Met Asn Glu Ser Gln Lys Ser Cys Asp Gln Leu Phe Asn Cys Ser Cys
385                 390                 395                 400

cct gcc atc gac aag gtg tgc gac att gtg ctc aat tct gga ggg ttt 1248
Pro Ala Ile Asp Lys Val Cys Asp Ile Val Leu Asn Ser Gly Gly Phe
                405                 410                 415

gga tcg cgt ctg act ggt gcg ggg tgg gga gga tct act gtc cat ctg          1296
Gly Ser Arg Leu Thr Gly Ala Gly Trp Gly Gly Ser Thr Val His Leu
            420                 425                 430

gtt gag gaa gcc aag ctg aac aac gtg att gac gct ctg aag aag gaa          1344
Val Glu Glu Ala Lys Leu Asn Asn Val Ile Asp Ala Leu Lys Lys Glu
        435                 440                 445

tac tac agc gac atg gat ggt gtc gat tac gat agc gcc atg gtc gtc          1392
Tyr Tyr Ser Asp Met Asp Gly Val Asp Tyr Asp Ser Ala Met Val Val
    450                 455                 460

act gtg cct ggc gag gga tgt gct att gtt gat ttg taa                      1431
Thr Val Pro Gly Glu Gly Cys Ala Ile Val Asp Leu
465                 470                 475


<210>  2
<211>  476
<212>  PRT
<213>  Yarrowia lipolytica

<400>  2

Met Ser Ile Ser Thr Leu Pro Leu Ala Asn Ile Tyr Ala Glu Pro Thr
1               5                   10                  15


Lys Gln Gln Pro Arg Tyr Asp Gln Leu Thr Lys Arg Phe Ser Glu Leu
            20                  25                  30


Tyr Ala Val Ser Ser Pro Thr Leu Val Arg Ser Pro Gly Arg Val Asn
        35                  40                  45


Leu Ile Gly Glu His Ile Asp Tyr Ser His Phe Ser Val Met Pro Met
    50                  55                  60


Ala Ile Glu Asp Asp Val Ile Met Ala Phe Ser Val Gly Gly Asp Ser
65                  70                  75                  80


Ile Glu Ile Ala Asn Met Glu Asp Asp Lys Phe Pro Ala Glu Lys Tyr
                85                  90                  95


Asn Val Pro Thr Ala Asp Gln Val Leu Thr Ile Asp Gln Thr Lys Thr
            100                 105                 110


Val Trp Thr Asn Tyr Phe Lys Cys Gly His Thr Val Gly Arg Lys His
        115                 120                 125


Leu Arg Asp Ser Gly Glu Glu Val Lys Pro Val Gly Ile Lys Val Leu
    130                 135                 140


Val His Gly Thr Val Pro Asp Ala Cys Gly Leu Ser Ser Ser Ala Ala
145                 150                 155                 160

Phe Val Val Cys Ser Thr Leu Val Thr Leu Leu Ser Gln Gly Met Gln
                165                 170                 175

Glu Val Ser Arg Glu Leu Leu Thr Arg Leu Ser Ile Thr Cys Glu Gln
            180                 185                 190

Leu Ile Gly Val Asn Thr Gly Gly Met Asp Gln Ala Ala Ser Ile Tyr
        195                 200                 205

Gly Gln Leu Asn His Thr Leu Leu Val Asp Phe Ala Pro Thr Leu Lys
    210                 215                 220

Ala Thr Pro Phe Gln Phe Pro Ser Thr Thr Pro Lys Thr Val Phe Leu
225                 230                 235                 240

Val Ala Asn Thr Leu Val Lys Ser Asn Lys His Glu Thr Ala Pro Arg
                245                 250                 255

Asn Tyr Asn Leu Arg Val Val Glu Ile Thr Leu Ala Val Asn Val Leu
            260                 265                 270

Leu Lys Ser Leu Lys Gly Val Thr Ile Pro Gln Asn Gly Asn Leu Asn
        275                 280                 285

Lys Gly Thr Leu His Gly Leu Met Lys Ala Val Asp Thr Asp Leu His
    290                 295                 300

Gly Leu Leu Asp Leu Val Glu Thr Leu Pro Lys Glu Pro Val Thr Leu
305                 310                 315                 320

Gln Glu Val Ala Gly Leu Leu Asn Val Ser Glu Asp Asp Val Lys Lys
                325                 330                 335

Gln Tyr Phe Ile Phe Pro Val Glu Phe Asp Thr Leu Ala Leu Tyr Ser
            340                 345                 350

Arg Ala Lys His Val Val Thr Glu Ala Ile Arg Val Lys Glu Phe Gln
        355                 360                 365

Asn Leu Leu Ser Lys Gly Glu Ser Asp Pro Arg Gln Leu Gly Lys Leu
    370                 375                 380

Met Asn Glu Ser Gln Lys Ser Cys Asp Gln Leu Phe Asn Cys Ser Cys
385                 390                 395                 400

Pro Ala Ile Asp Lys Val Cys Asp Ile Val Leu Asn Ser Gly Gly Phe

405                 410                 415

Gly Ser Arg Leu Thr Gly Ala Gly Trp Gly Gly Ser Thr Val His Leu
            420                 425                 430

Val Glu Glu Ala Lys Leu Asn Asn Val Ile Asp Ala Leu Lys Lys Glu
        435                 440                 445

Tyr Tyr Ser Asp Met Asp Gly Val Asp Tyr Asp Ser Ala Met Val Val
    450                 455                 460

Thr Val Pro Gly Glu Gly Cys Ala Ile Val Asp Leu
465                 470                 475

<210>  3
<211>  1059
<212>  DNA
<213>  Yarrowia lipolytica

<220>
<221>  CDS
<222>  (1)..(1059)

<400>  3
atg act ctt gtg gca tcc gac att cct cac aga cga tac aac ccg ctg      48
Met Thr Leu Val Ala Ser Asp Ile Pro His Arg Arg Tyr Asn Pro Leu
1               5                   10                  15

cga caa tca tgg gtg ctg gtg tca cct cat cga gct aag cga cct tgg      96
Arg Gln Ser Trp Val Leu Val Ser Pro His Arg Ala Lys Arg Pro Trp
            20                  25                  30

cag gga cag caa gaa gag ccc acc aag gac acg aag ccc gag tac gac      144
Gln Gly Gln Gln Glu Glu Pro Thr Lys Asp Thr Lys Pro Glu Tyr Asp
        35                  40                  45

gac aag tgc tat ctg tgt cct cga aat gct cga atc aac ggg gag atg      192
Asp Lys Cys Tyr Leu Cys Pro Arg Asn Ala Arg Ile Asn Gly Glu Met
    50                  55                  60

aac ccg gac tac aag gac act ttc gtg ttt gag aac gac ttc agt gct      240
Asn Pro Asp Tyr Lys Asp Thr Phe Val Phe Glu Asn Asp Phe Ser Ala
65                  70                  75                  80

gtt ttt gag aaa act gcg gaa gtg gag aag gtc gtt gag gag gcc caa      288
Val Phe Glu Lys Thr Ala Glu Val Glu Lys Val Val Glu Glu Ala Gln
                85                  90                  95

aag gag cac aca gag tct act gct ctc gac ctg gac ctt ctg cga aaa      336
Lys Glu His Thr Glu Ser Thr Ala Leu Asp Leu Asp Leu Leu Arg Lys
            100                 105                 110

gaa gat gtg ttc gga tac tgt cag gtt att tgc ttt tcg ccc aaa cac      384
Glu Asp Val Phe Gly Tyr Cys Gln Val Ile Cys Phe Ser Pro Lys His
        115                 120                 125

aat gtg act ctt gct aca atg act gtt gag cag atc gaa aag gtg att      432

Asn Val Thr Leu Ala Thr Met Thr Val Glu Gln Ile Glu Lys Val Ile
    130                 135                 140

gag acc tgg cag aaa atg tac aca gag gct cag agt cga gga atc aag     480
Glu Thr Trp Gln Lys Met Tyr Thr Glu Ala Gln Ser Arg Gly Ile Lys
145                 150                 155                 160

tac tcg ctg att ttt gaa aac aag gga gct acc atg ggc tgc tcc aac     528
Tyr Ser Leu Ile Phe Glu Asn Lys Gly Ala Thr Met Gly Cys Ser Asn
                165                 170                 175

cct cat gcc cat ggc cag gtc tgg aac acc agt att gtt cct gaa gag     576
Pro His Ala His Gly Gln Val Trp Asn Thr Ser Ile Val Pro Glu Glu
            180                 185                 190

ccc cag atg gag ttt gat tca ttg aag aaa tac aag gac gaa aag aac     624
Pro Gln Met Glu Phe Asp Ser Leu Lys Lys Tyr Lys Asp Glu Lys Asn
        195                 200                 205

cgt gat ctt ctg gtc gat tac gtc aac ttg gaa atg tcc aaa aac gaa     672
Arg Asp Leu Leu Val Asp Tyr Val Asn Leu Glu Met Ser Lys Asn Glu
    210                 215                 220

cga atc gtc tac cag aat gcc tcg ttt ctc gtg gtg gtt cct tac tgg     720
Arg Ile Val Tyr Gln Asn Ala Ser Phe Leu Val Val Val Pro Tyr Trp
225                 230                 235                 240

gcc gtc tgg cct ttt gag acc atg att gtg tcc aag gaa cgg gtt tct     768
Ala Val Trp Pro Phe Glu Thr Met Ile Val Ser Lys Glu Arg Val Ser
                245                 250                 255

tcc atc aaa aag ctg acc aag gaa caa gtc cag cat ctt gcc gac gcc     816
Ser Ile Lys Lys Leu Thr Lys Glu Gln Val Gln His Leu Ala Asp Ala
            260                 265                 270

atc tcg gtg atc aca aaa aag tac gac aag ctc ttc aac acc tct ttc     864
Ile Ser Val Ile Thr Lys Lys Tyr Asp Lys Leu Phe Asn Thr Ser Phe
        275                 280                 285

ccc tac tca atg ggt att cat caa tca ttt gtg gac gtt gag aac tgt     912
Pro Tyr Ser Met Gly Ile His Gln Ser Phe Val Asp Val Glu Asn Cys
    290                 295                 300

ccc tac gag cat ctt cgg ctc ctg ttc tac cct cct ctt ctg cga tct     960
Pro Tyr Glu His Leu Arg Leu Leu Phe Tyr Pro Pro Leu Leu Arg Ser
305                 310                 315                 320

gcc tcc gtc aag aag ttc cag gtc gga ttc gag atg ctg gga atg ccg     1008
Ala Ser Val Lys Lys Phe Gln Val Gly Phe Glu Met Leu Gly Met Pro
                325                 330                 335

cag cga gac att act gcc gaa ggg gct gcc aag cgg ttg tat gat ttg     1056
Gln Arg Asp Ile Thr Ala Glu Gly Ala Ala Lys Arg Leu Tyr Asp Leu
            340                 345                 350

taa                                                                 1059


<210>  4
<211>  352
<212>  PRT
<213>  Yarrowia lipolytica

<400> 4

Met Thr Leu Val Ala Ser Asp Ile Pro His Arg Arg Tyr Asn Pro Leu
1               5                   10                  15

Arg Gln Ser Trp Val Leu Val Ser Pro His Arg Ala Lys Arg Pro Trp
            20                  25                  30

Gln Gly Gln Gln Glu Glu Pro Thr Lys Asp Thr Lys Pro Glu Tyr Asp
        35                  40                  45

Asp Lys Cys Tyr Leu Cys Pro Arg Asn Ala Arg Ile Asn Gly Glu Met
    50                  55                  60

Asn Pro Asp Tyr Lys Asp Thr Phe Val Phe Glu Asn Asp Phe Ser Ala
65                  70                  75                  80

Val Phe Glu Lys Thr Ala Glu Val Glu Lys Val Val Glu Glu Ala Gln
                85                  90                  95

Lys Glu His Thr Glu Ser Thr Ala Leu Asp Leu Asp Leu Leu Arg Lys
            100                 105                 110

Glu Asp Val Phe Gly Tyr Cys Gln Val Ile Cys Phe Ser Pro Lys His
        115                 120                 125

Asn Val Thr Leu Ala Thr Met Thr Val Glu Gln Ile Glu Lys Val Ile
    130                 135                 140

Glu Thr Trp Gln Lys Met Tyr Thr Glu Ala Gln Ser Arg Gly Ile Lys
145                 150                 155                 160

Tyr Ser Leu Ile Phe Glu Asn Lys Gly Ala Thr Met Gly Cys Ser Asn
                165                 170                 175

Pro His Ala His Gly Gln Val Trp Asn Thr Ser Ile Val Pro Glu Glu
            180                 185                 190

Pro Gln Met Glu Phe Asp Ser Leu Lys Lys Tyr Lys Asp Glu Lys Asn
        195                 200                 205

Arg Asp Leu Leu Val Asp Tyr Val Asn Leu Glu Met Ser Lys Asn Glu
    210                 215                 220

Arg Ile Val Tyr Gln Asn Ala Ser Phe Leu Val Val Val Pro Tyr Trp
225                 230                 235                 240

Ala Val Trp Pro Phe Glu Thr Met Ile Val Ser Lys Glu Arg Val Ser

245                 250                 255

Ser Ile Lys Lys Leu Thr Lys Glu Gln Val Gln His Leu Ala Asp Ala
            260                 265                 270

Ile Ser Val Ile Thr Lys Lys Tyr Asp Lys Leu Phe Asn Thr Ser Phe
        275                 280                 285

Pro Tyr Ser Met Gly Ile His Gln Ser Phe Val Asp Val Glu Asn Cys
    290                 295                 300

Pro Tyr Glu His Leu Arg Leu Leu Phe Tyr Pro Pro Leu Leu Arg Ser
305                 310                 315                 320

Ala Ser Val Lys Lys Phe Gln Val Gly Phe Glu Met Leu Gly Met Pro
                325                 330                 335

Gln Arg Asp Ile Thr Ala Glu Gly Ala Ala Lys Arg Leu Tyr Asp Leu
            340                 345                 350

<210>  5
<211>  1110
<212>  DNA
<213>  Yarrowia lipolytica

<220>
<221>  CDS
<222>  (1)..(1110)

<400>  5
atg acc cag gct gct gct gaa cac gtg ctc gtc acc gga gga gcc gga       48
Met Thr Gln Ala Ala Ala Glu His Val Leu Val Thr Gly Gly Ala Gly
1               5                   10                  15

tac atc ggc tct cac act gtt att gct ctg gtt gag gct gga cga aag       96
Tyr Ile Gly Ser His Thr Val Ile Ala Leu Val Glu Ala Gly Arg Lys
            20                  25                  30

cca gtc att gtc gac aac ctg gac aac tcg tgt gag gag gct gtg cgc      144
Pro Val Ile Val Asp Asn Leu Asp Asn Ser Cys Glu Glu Ala Val Arg
        35                  40                  45

cga atc gaa cag att gtg ggc gag aaa atc ccc ttc ttt gag gtt gat      192
Arg Ile Glu Gln Ile Val Gly Glu Lys Ile Pro Phe Phe Glu Val Asp
    50                  55                  60

atc aga gac act aac ggt ctg gat gaa gtg tac aag cag tac cca acc      240
Ile Arg Asp Thr Asn Gly Leu Asp Glu Val Tyr Lys Gln Tyr Pro Thr
65                  70                  75                  80

atc acc tct gtg ctg cat ttc gct ggt ctc aag gcc gtg gga gaa tct      288
Ile Thr Ser Val Leu His Phe Ala Gly Leu Lys Ala Val Gly Glu Ser
                85                  90                  95

ggc cag att cct ctg gag tat tac aac tgc aac gtg gcg ggt tcc att      336

Gly Gln Ile Pro Leu Glu Tyr Tyr Asn Cys Asn Val Ala Gly Ser Ile
            100                 105                 110

aac ctc ctg cag tgg tgc cag aag cac aac atc cac aac ttt gtc ttc 384
Asn Leu Leu Gln Trp Cys Gln Lys His Asn Ile His Asn Phe Val Phe
        115                 120                 125

tcg tcg tcc gcc acc gtc tac gga gac gtc acc cgg ttt gag ggc atg 432
Ser Ser Ser Ala Thr Val Tyr Gly Asp Val Thr Arg Phe Glu Gly Met
    130                 135                 140

att ccc att cct gag gag tgt ccc acc gga ccc act aac cct tac gga 480
Ile Pro Ile Pro Glu Glu Cys Pro Thr Gly Pro Thr Asn Pro Tyr Gly
145                 150                 155                 160

cga acc aag atg atg atc gag gac atc att cga gat cac gtg cga gcc 528
Arg Thr Lys Met Met Ile Glu Asp Ile Ile Arg Asp His Val Arg Ala
                165                 170                 175

aat gct ccc tgg aac gcc gct att ctg cga tac ttc aat ccg atg ggt 576
Asn Ala Pro Trp Asn Ala Ala Ile Leu Arg Tyr Phe Asn Pro Met Gly
            180                 185                 190

gcc cat ccc tct ggt ctg gtt gga gag gat cct ctt ggt atc ccc aac 624
Ala His Pro Ser Gly Leu Val Gly Glu Asp Pro Leu Gly Ile Pro Asn
        195                 200                 205

aac ctg ctt cct tac ctt gcc cag gtt gcc gtg ggc cga cga gag aag 672
Asn Leu Leu Pro Tyr Leu Ala Gln Val Ala Val Gly Arg Arg Glu Lys
    210                 215                 220

ctc ttt gtg ttt ggt gac gac tac ccc tct cga gat gga act ccc atc 720
Leu Phe Val Phe Gly Asp Asp Tyr Pro Ser Arg Asp Gly Thr Pro Ile
225                 230                 235                 240

cga gac tac atc cac gtg tgc gat ctt gct gct ggc cac gtg gct gcc 768
Arg Asp Tyr Ile His Val Cys Asp Leu Ala Ala Gly His Val Ala Ala
                245                 250                 255

ctt gag aag ctc gag gct gtc gat aaa gag cga aag cag ctg ccc cag 816
Leu Glu Lys Leu Glu Ala Val Asp Lys Glu Arg Lys Gln Leu Pro Gln
            260                 265                 270

ggg tcc ccc gag ctc aag cac ggt ttc tct cga gaa tgg aac cta gga 864
Gly Ser Pro Glu Leu Lys His Gly Phe Ser Arg Glu Trp Asn Leu Gly
        275                 280                 285

acc ggc cac gga tct act gtg ctt gag gtc gtc aag gcc ttt tcc aac 912
Thr Gly His Gly Ser Thr Val Leu Glu Val Val Lys Ala Phe Ser Asn
    290                 295                 300

gcc gtg ggt aag gac cta ccc tac gaa atc acc ggt cga cga gac gga 960
Ala Val Gly Lys Asp Leu Pro Tyr Glu Ile Thr Gly Arg Arg Asp Gly
305                 310                 315                 320

gac gtt ctg gat ctc acc gcg aaa ccc ggc cga gca aac tcc gag ctc 1008
Asp Val Leu Asp Leu Thr Ala Lys Pro Gly Arg Ala Asn Ser Glu Leu
                325                 330                 335

aac tgg aag gca gag aag act ctc gac gaa agc tgc tcc gat ctg tgg 1056
Asn Trp Lys Ala Glu Lys Thr Leu Asp Glu Ser Cys Ser Asp Leu Trp
            340                 345                 350

aga tgg acc agc gag aac ccc tat ggt tac aaa aag gac cgt cat gac     1104
Arg Trp Thr Ser Glu Asn Pro Tyr Gly Tyr Lys Lys Asp Arg His Asp
        355                 360                 365

aag taa                                                             1110
Lys

<210>  6
<211>  369
<212>  PRT
<213>  Yarrowia lipolytica

<400>  6

Met Thr Gln Ala Ala Ala Glu His Val Leu Val Thr Gly Gly Ala Gly
1               5                   10                  15

Tyr Ile Gly Ser His Thr Val Ile Ala Leu Val Glu Ala Gly Arg Lys
            20                  25                  30

Pro Val Ile Val Asp Asn Leu Asp Asn Ser Cys Glu Glu Ala Val Arg
        35                  40                  45

Arg Ile Glu Gln Ile Val Gly Glu Lys Ile Pro Phe Phe Glu Val Asp
    50                  55                  60

Ile Arg Asp Thr Asn Gly Leu Asp Glu Val Tyr Lys Gln Tyr Pro Thr
65                  70                  75                  80

Ile Thr Ser Val Leu His Phe Ala Gly Leu Lys Ala Val Gly Glu Ser
                85                  90                  95

Gly Gln Ile Pro Leu Glu Tyr Tyr Asn Cys Asn Val Ala Gly Ser Ile
            100                 105                 110

Asn Leu Leu Gln Trp Cys Gln Lys His Asn Ile His Asn Phe Val Phe
        115                 120                 125

Ser Ser Ser Ala Thr Val Tyr Gly Asp Val Thr Arg Phe Glu Gly Met
    130                 135                 140

Ile Pro Ile Pro Glu Glu Cys Pro Thr Gly Pro Thr Asn Pro Tyr Gly
145                 150                 155                 160

Arg Thr Lys Met Met Ile Glu Asp Ile Ile Arg Asp His Val Arg Ala
                165                 170                 175

Asn Ala Pro Trp Asn Ala Ala Ile Leu Arg Tyr Phe Asn Pro Met Gly
            180                 185                 190

Ala His Pro Ser Gly Leu Val Gly Glu Asp Pro Leu Gly Ile Pro Asn
        195                 200                 205

Asn Leu Leu Pro Tyr Leu Ala Gln Val Ala Val Gly Arg Arg Glu Lys
    210                 215                 220

Leu Phe Val Phe Gly Asp Asp Tyr Pro Ser Arg Asp Gly Thr Pro Ile
225                 230                 235                 240

Arg Asp Tyr Ile His Val Cys Asp Leu Ala Ala Gly His Val Ala Ala
                245                 250                 255

Leu Glu Lys Leu Glu Ala Val Asp Lys Glu Arg Lys Gln Leu Pro Gln
            260                 265                 270

Gly Ser Pro Glu Leu Lys His Gly Phe Ser Arg Glu Trp Asn Leu Gly
        275                 280                 285

Thr Gly His Gly Ser Thr Val Leu Glu Val Val Lys Ala Phe Ser Asn
    290                 295                 300

Ala Val Gly Lys Asp Leu Pro Tyr Glu Ile Thr Gly Arg Arg Asp Gly
305                 310                 315                 320

Asp Val Leu Asp Leu Thr Ala Lys Pro Gly Arg Ala Asn Ser Glu Leu
                325                 330                 335

Asn Trp Lys Ala Glu Lys Thr Leu Asp Glu Ser Cys Ser Asp Leu Trp
            340                 345                 350

Arg Trp Thr Ser Glu Asn Pro Tyr Gly Tyr Lys Lys Asp Arg His Asp
        355                 360                 365

Lys

<210>  7
<211>  984
<212>  DNA
<213>  Yarrowia lipolytica

<220>
<221>  CDS
<222>  (1)..(984)

<400>  7
atg acc ttt tgc aac acg gga gca acg ctc cag agc tgg gaa aat agc      48
Met Thr Phe Cys Asn Thr Gly Ala Thr Leu Gln Ser Trp Glu Asn Ser
1               5                   10                  15

atc ctc ggg ttt gaa aac cac aca aat tac ctg aac cga caa aac ccc     96
Ile Leu Gly Phe Glu Asn His Thr Asn Tyr Leu Asn Arg Gln Asn Pro
            20                  25                  30

tac ttt gga gct aca att ggc ccg gta gct aac cgg cta tac caa cca     144
Tyr Phe Gly Ala Thr Ile Gly Pro Val Ala Asn Arg Leu Tyr Gln Pro
        35                  40                  45

aac ggt atc ctg ttg cac ggc gga aaa gaa ggc tgg tcc tgg aag aaa     192
Asn Gly Ile Leu Leu His Gly Gly Lys Glu Gly Trp Ser Trp Lys Lys
    50                  55                  60

tgg aac aag agc tcc ccc gcc gct gct gaa aat agt aac gtg acc aaa     240
Trp Asn Lys Ser Ser Pro Ala Ala Ala Glu Asn Ser Asn Val Thr Lys
65                  70                  75                  80

ttt gag ctc aga gat acc cac atc acc gcc cac gtg aca tat acg ctg     288
Phe Glu Leu Arg Asp Thr His Ile Thr Ala His Val Thr Tyr Thr Leu
                85                  90                  95

act aag tcg ccc gac aag aag acg tcc caa ctg gaa atc gaa tac tcc     336
Thr Lys Ser Pro Asp Lys Lys Thr Ser Gln Leu Glu Ile Glu Tyr Ser
            100                 105                 110

gcc aac agt aat gag ctg ggt aag agc atg ccg att agc atg aca aat     384
Ala Asn Ser Asn Glu Leu Gly Lys Ser Met Pro Ile Ser Met Thr Asn
        115                 120                 125

cac agc tac ttc tgc ttg gac aaa gac ggt gcc gaa ggt gag ggt cac     432
His Ser Tyr Phe Cys Leu Asp Lys Asp Gly Ala Glu Gly Glu Gly His
    130                 135                 140

gtg acc gtc gac gac cac gag ctc tgg ctg tgt aac gat cat gtg att     480
Val Thr Val Asp Asp His Glu Leu Trp Leu Cys Asn Asp His Val Ile
145                 150                 155                 160

gaa cat aag gac ggt ctg caa tcg gag cct cct acg ggc gag ctt gtc     528
Glu His Lys Asp Gly Leu Gln Ser Glu Pro Pro Thr Gly Glu Leu Val
                165                 170                 175

cga tgc gag gca ttg ttg aag gcg ggg gcg ggt gag tca cgt gac aca     576
Arg Cys Glu Ala Leu Leu Lys Ala Gly Ala Gly Glu Ser Arg Asp Thr
            180                 185                 190

ttc tcg tca ctt ggt cgc ttt tcg gac ttg tct tca ctt ggt ggg acg     624
Phe Ser Ser Leu Gly Arg Phe Ser Asp Leu Ser Ser Leu Gly Gly Thr
        195                 200                 205

tcc atc tcc ctc aat gga act gtt ctc gac cac act ttt gct gtc gat     672
Ser Ile Ser Leu Asn Gly Thr Val Leu Asp His Thr Phe Ala Val Asp
    210                 215                 220

aac gac agg ctt tca cgt gat gcc aag ctc gat acc cgt gat gat cca     720
Asn Asp Arg Leu Ser Arg Asp Ala Lys Leu Asp Thr Arg Asp Asp Pro
225                 230                 235                 240

tta gtc agc gtt gca att ttg tca cgt gga aag acc cgt ctg acc gtc     768
Leu Val Ser Val Ala Ile Leu Ser Arg Gly Lys Thr Arg Leu Thr Val
                245                 250                 255

aag acc acc gag ccc tgt ttc cag gtc tat acc ggc gac tac aac aat     816
Lys Thr Thr Glu Pro Cys Phe Gln Val Tyr Thr Gly Asp Tyr Asn Asn

260                 265                 270

gtg gaa ctg ctt ccc ggc gag tca cgt gac ttt ggt cga cgt tct ggg         864
Val Glu Leu Leu Pro Gly Glu Ser Arg Asp Phe Gly Arg Arg Ser Gly
        275                 280                 285

gtt gcg att gag tgt tct cgc ccc acg aat gct tgg aat gtt cct ggg         912
Val Ala Ile Glu Cys Ser Arg Pro Thr Asn Ala Trp Asn Val Pro Gly
    290                 295                 300

tgg cag ccg tgg gtt gat ttg ggt cag ggg gag tat ggt gct aag att         960
Trp Gln Pro Trp Val Asp Leu Gly Gln Gly Glu Tyr Gly Ala Lys Ile
305                 310                 315                 320

atc tat gtt ttg gag cgg gag tga                                         984
Ile Tyr Val Leu Glu Arg Glu
                325

<210>  8
<211>  327
<212>  PRT
<213>  Yarrowia lipolytica

<400>  8

Met Thr Phe Cys Asn Thr Gly Ala Thr Leu Gln Ser Trp Glu Asn Ser
1               5                   10                  15

Ile Leu Gly Phe Glu Asn His Thr Asn Tyr Leu Asn Arg Gln Asn Pro
            20                  25                  30

Tyr Phe Gly Ala Thr Ile Gly Pro Val Ala Asn Arg Leu Tyr Gln Pro
        35                  40                  45

Asn Gly Ile Leu Leu His Gly Gly Lys Glu Gly Trp Ser Trp Lys Lys
    50                  55                  60

Trp Asn Lys Ser Ser Pro Ala Ala Ala Glu Asn Ser Asn Val Thr Lys
65                  70                  75                  80

Phe Glu Leu Arg Asp Thr His Ile Thr Ala His Val Thr Tyr Thr Leu
                85                  90                  95

Thr Lys Ser Pro Asp Lys Lys Thr Ser Gln Leu Glu Ile Glu Tyr Ser
            100                 105                 110

Ala Asn Ser Asn Glu Leu Gly Lys Ser Met Pro Ile Ser Met Thr Asn
        115                 120                 125

His Ser Tyr Phe Cys Leu Asp Lys Asp Gly Ala Glu Gly Glu Gly His
    130                 135                 140

Val Thr Val Asp Asp His Glu Leu Trp Leu Cys Asn Asp His Val Ile

145                 150                 155                 160

Glu His Lys Asp Gly Leu Gln Ser Glu Pro Pro Thr Gly Glu Leu Val
                165                 170                 175

Arg Cys Glu Ala Leu Leu Lys Ala Gly Ala Gly Glu Ser Arg Asp Thr
            180                 185                 190

Phe Ser Ser Leu Gly Arg Phe Ser Asp Leu Ser Ser Leu Gly Gly Thr
        195                 200                 205

Ser Ile Ser Leu Asn Gly Thr Val Leu Asp His Thr Phe Ala Val Asp
    210                 215                 220

Asn Asp Arg Leu Ser Arg Asp Ala Lys Leu Asp Thr Arg Asp Asp Pro
225                 230                 235                 240

Leu Val Ser Val Ala Ile Leu Ser Arg Gly Lys Thr Arg Leu Thr Val
                245                 250                 255

Lys Thr Thr Glu Pro Cys Phe Gln Val Tyr Thr Gly Asp Tyr Asn Asn
            260                 265                 270

Val Glu Leu Leu Pro Gly Glu Ser Arg Asp Phe Gly Arg Arg Ser Gly
        275                 280                 285

Val Ala Ile Glu Cys Ser Arg Pro Thr Asn Ala Trp Asn Val Pro Gly
    290                 295                 300

Trp Gln Pro Trp Val Asp Leu Gly Gln Gly Glu Tyr Gly Ala Lys Ile
305                 310                 315                 320

Ile Tyr Val Leu Glu Arg Glu
                325

<210>  9
<211>  483
<212>  PRT
<213>  Artificial Sequence

<220>
<223>  GAL1 consensus sequence


<220>
<221>  VARIANT
<222>  (2)..(2)
<223>  Replace = Ser

<220>
<221>  VARIANT

<222>  (3)..(3)
<223>  Replace = Ile

<220>
<221>  VARIANT
<222>  (4)..(4)
<223>  Replace = Ser or Thr

<220>
<221>  VARIANT
<222>  (5)..(5)
<223>  Replace = Thr

<220>
<221>  VARIANT
<222>  (7)..(7)
<223>  Replace = Pro

<220>
<221>  VARIANT
<222>  (9)..(9)
<223>  Replace = Ala or Ser

<220>
<221>  VARIANT
<222>  (10)..(10)
<223>  Replace = Asn

<220>
<221>  VARIANT
<222>  (13)..(13)
<223>  Replace = Ser

<220>
<221>  VARIANT
<222>  (14)..(14)
<223>  Replace = Glu or Ser

<220>
<221>  VARIANT
<222>  (16)..(16)
<223>  Replace = Thr, Ser or Glu

<220>
<221>  VARIANT
<222>  (17)..(17)
<223>  Replace = Arg or Thr

<220>
<221>  VARIANT
<222>  (18)..(18)
<223>  Replace = His or Ala

<220>
<221>  VARIANT
<222>  (19)..(19)
<223>  Replace = Gln or Arg

<220>
<221>  VARIANT
<222>  (20)..(20)
<223>  Replace = Pro, Thr or Lys

<220>
<221>  VARIANT
<222>  (23)..(23)
<223>  Replace = Glu or Val

<220>
<221>  VARIANT
<222>  (24)..(24)
<223>  Replace = Gln, Glu or Asn

<220>
<221>  VARIANT
<222>  (25)..(25)
<223>  Replace = Leu

<220>
<221>  VARIANT
<222>  (26)..(26)
<223>  Replace = Val

<220>
<221>  VARIANT
<222>  (27)..(27)
<223>  Replace = Lys or Thr

<220>
<221>  VARIANT
<222>  (30)..(30)
<223>  Replace = Ser, Thr or Arg

<220>
<221>  VARIANT
<222>  (31)..(31)
<223>  Replace = Glu

<220>
<221>  VARIANT
<222>  (34)..(34)
<223>  Replace = Pro or nothing

<220>
<221>  VARIANT
<222>  (35)..(35)
<223>  Replace = Val

<220>
<221>  VARIANT
<222>  (36)..(36)
<223>  Replace = Ser, Ala, Cys

<220>
<221>  VARIANT
<222>  (37)..(37)
<223>  Replace = Ser or Lys

<220>
<221>  VARIANT
<222>  (38)..(38)
<223>  Replace = Ala

<220>
<221>  VARIANT
<222>  (40)..(40)

<223>  Replace = Leu or Val

<220>
<221>  VARIANT
<222>  (43)..(43)
<223>  Replace = Ala

<220>
<221>  VARIANT
<222>  (67)..(67)
<223>  Replace = Gly

<220>
<221>  VARIANT
<222>  (71)..(71)
<223>  Replace = Ile

<220>
<221>  VARIANT
<222>  (75)..(75)
<223>  Replace = Ser

<220>
<221>  VARIANT
<222>  (77)..(77)
<223>  Replace = Gly or Glu

<220>
<221>  VARIANT
<222>  (80)..(80)
<223>  Replace = Ser or Lys

<220>
<221>  VARIANT
<222>  (82)..(82)
<223>  Replace = Glu or Arg

<220>
<221>  VARIANT
<222>  (83)..(83)
<223>  Replace = Ile

<220>
<221>  VARIANT
<222>  (86)..(86)
<223>  Replace = Met or Ile

<220>
<221>  VARIANT
<222>  (89)..(89)
<223>  Replace = Glu

<220>
<221>  VARIANT
<222>  (93)..(93)
<223>  Replace = Ser

<220>
<221>  VARIANT
<222>  (95)..(95)
<223>  Replace = Lys or Ile

<220>

<221>  VARIANT
<222>  (97)..(97)
<223>  Replace = Asn or His

<220>
<221>  VARIANT
<222>  (98)..(98)
<223>  Replace = Val or Cys

<220>
<221>  VARIANT
<222>  (100)..(100)
<223>  Replace = Thr, Ala or Asp

<220>
<221>  VARIANT
<222>  (101)..(101)
<223>  Replace = Ala or Pro

<220>
<221>  VARIANT
<222>  (102)..(102)
<223>  Replace = Asp, Thr or Asn

<220>
<221>  VARIANT
<222>  (103)..(103)
<223>  Replace = Gln or Cys

<220>
<221>  VARIANT
<222>  (104)..(104)
<223>  Replace = Glu

<220>
<221>  VARIANT
<222>  (106)..(106)
<223>  Replace = Thr

<220>
<221>  VARIANT
<222>  (109)..(109)
<223>  Replace = Gln

<220>
<221>  VARIANT
<222>  (110)..(110)
<223>  Replace = Thr or Lys

<220>
<221>  VARIANT
<222>  (122)..(122)
<223>  Replace = Phe

<220>
<221>  VARIANT
<222>  (125)..(125)
<223>  Replace = Gly

<220>
<221>  VARIANT
<222>  (127)..(127)
<223>  Replace = Lys

<220>
<221>  VARIANT
<222>  (128)..(128)
<223>  Replace = Tyr

<220>
<221>  VARIANT
<222>  (129)..(129)
<223>  Replace = Phe or Val

<220>
<221>  VARIANT
<222>  (130)..(130)
<223>  Replace = Arg

<220>
<221>  VARIANT
<222>  (131)..(131)
<223>  Replace = Asp or Glu

<220>
<221>  VARIANT
<222>  (132)..(132)
<223>  Replace = Thr

<220>
<221>  VARIANT
<222>  (133)..(133)
<223>  Replace = His

<220>
<221>  VARIANT
<222>  (134)..(134)
<223>  Replace = Glu, Asp, or Gly

<220>
<221>  VARIANT
<222>  (135)..(135)
<223>  Replace = Glu or Asp

<220>
<221>  VARIANT
<222>  (136)..(136)
<223>  Replace = Leu or nothing

<220>
<221>  VARIANT
<222>  (137)..(137)
<223>  Replace = Lys or Gln

<220>
<221>  VARIANT
<222>  (138)..(138)
<223>  Replace = Pro

<220>
<221>  VARIANT
<222>  (139)..(139)
<223>  Replace = Leu

<220>
<221>  VARIANT

<222>  (143)..(143)
<223>  Replace = Ile

<220>
<221>  VARIANT
<222>  (169)..(169)
<223>  Replace = Thr

<220>
<221>  VARIANT
<222>  (171)..(171)
<223>  Replace = Leu or Met

<220>
<221>  VARIANT
<222>  (172)..(172)
<223>  Replace = Ser

<220>
<221>  VARIANT
<222>  (173)..(173)
<223>  Replace = Gln or Tyr

<220>
<221>  VARIANT
<222>  (175)..(175)
<223>  Replace = nothing

<220>
<221>  VARIANT
<222>  (176)..(176)
<223>  Replace = nothing

<220>
<221>  VARIANT
<222>  (177)..(177)
<223>  Replace = nothing

<220>
<221>  VARIANT
<222>  (178)..(178)
<223>  Replace = Met or Leu

<220>
<221>  VARIANT
<222>  (179)..(179)
<223>  Replace = Gln, Lys or Thr

<220>
<221>  VARIANT
<222>  (180)..(180)
<223>  Replace = Glu or Gln

<220>
<221>  VARIANT
<222>  (181)..(181)
<223>  Replace = Val

<220>
<221>  VARIANT
<222>  (184)..(184)
<223>  Replace = Glu

<220>
<221>  VARIANT
<222>  (188)..(188)
<223>  Replace = Arg or Thr

<220>
<221>  VARIANT
<222>  (197)..(197)
<223>  Replace = Val

<220>
<221>  VARIANT
<222>  (211)..(211)
<223>  Replace = Tyr

<220>
<221>  VARIANT
<222>  (213)..(213)
<223>  Replace = Val

<220>
<221>  VARIANT
<222>  (223)..(223)
<223>  Replace = Ala

<220>
<221>  VARIANT
<222>  (225)..(225)
<223>  Replace = Val or Lys

<220>
<221>  VARIANT
<222>  (227)..(227)
<223>  Replace = Arg

<220>
<221>  VARIANT
<222>  (230)..(230)
<223>  Replace = Pro

<220>
<221>  VARIANT
<222>  (232)..(232)
<223>  Replace = Gln or Leu

<220>
<221>  VARIANT
<222>  (235)..(235)
<223>  Replace = Ser or Ala

<220>
<221>  VARIANT
<222>  (237)..(237)
<223>  Replace = Thr, Val or Ser

<220>
<221>  VARIANT
<222>  (239)..(239)
<223>  Replace = Lys or Ser

<220>
<221>  VARIANT
<222>  (240)..(240)

<223>  Replace = Ala

<220>
<221>  VARIANT
<222>  (249)..(249)
<223>  Replace = Ile

<220>
<221>  VARIANT
<222>  (250)..(250)
<223>  Replace = Arg

<220>
<221>  VARIANT
<222>  (270)..(270)
<223>  Replace = Leu

<220>
<221>  VARIANT
<222>  (272)..(272)
<223>  Replace = Val

<220>
<221>  VARIANT
<222>  (274)..(274)
<223>  Replace = Ile

<220>
<221>  VARIANT
<222>  (275)..(275)
<223>  Replace = Leu

<220>
<221>  VARIANT
<222>  (276)..(276)
<223>  Replace = Leu

<220>
<221>  VARIANT
<222>  (277)..(277)
<223>  Replace = His

<220>
<221>  VARIANT
<222>  (278)..(278)
<223>  Replace = Ser or Gly

<220>
<221>  VARIANT
<222>  (280)..(280)
<223>  Replace = Asn or nothing

<220>
<221>  VARIANT
<222>  (281)..(281)
<223>  Replace = Asp

<220>
<221>  VARIANT
<222>  (282)..(282)
<223>  Replace = Val

<220>

<221>  VARIANT
<222>  (283)..(283)
<223>  Replace = Thr, Ser or Lys

<220>
<221>  VARIANT
<222>  (284)..(284)
<223>  Replace = Ile

<220>
<221>  VARIANT
<222>  (285)..(285)
<223>  Replace = Pro

<220>
<221>  VARIANT
<222>  (287)..(287)
<223>  Replace = Asp

<220>
<221>  VARIANT
<222>  (295)..(295)
<223>  Replace = Leu

<220>
<221>  VARIANT
<222>  (296)..(296)
<223>  Replace = His or Arg

<220>
<221>  VARIANT
<222>  (299)..(299)
<223>  Replace = Leu

<220>
<221>  VARIANT
<222>  (300)..(300)
<223>  Replace = Lys or Glu

<220>
<221>  VARIANT
<222>  (302)..(302)
<223>  Replace = Val or Cys

<220>
<221>  VARIANT
<222>  (303)..(303)
<223>  Replace = Asp or Asn

<220>
<221>  VARIANT
<222>  (307)..(307)
<223>  Replace =  Asn, His or Thr

<220>
<221>  VARIANT
<222>  (310)..(310)
<223>  Replace =  Leu

<220>
<221>  VARIANT
<222>  (311)..(311)
<223>  Replace =  Asp or Lys

<220>
<221>  VARIANT
<222>  (313)..(313)
<223>  Replace =  Val

<220>
<221>  VARIANT
<222>  (314)..(314)
<223>  Replace =  nothing

<220>
<221>  VARIANT
<222>  (315)..(315)
<223>  Replace =  Phe

<220>
<221>  VARIANT
<222>  (316)..(316)
<223>  Replace =  Thr

<220>
<221>  VARIANT
<222>  (319)..(319)
<223>  Replace =  Lys

<220>
<221>  VARIANT
<222>  (320)..(320)
<223>  Replace =  Asp

<220>
<221>  VARIANT
<222>  (321)..(321)
<223>  Replace =  Pro

<220>
<221>  VARIANT
<222>  (322)..(322)
<223>  Replace =  Leu

<220>
<221>  VARIANT
<222>  (323)..(323)
<223>  Replace =  Thr

<220>
<221>  VARIANT
<222>  (325)..(325)
<223>  Replace = Gln

<220>
<221>  VARIANT
<222>  (327)..(327)
<223>  Replace = Val

<220>
<221>  VARIANT
<222>  (328)..(328)
<223>  Replace = Ala

<220>
<221>  VARIANT

<222>  (329)..(329)
<223>  Replace = Gly, Lys or Thr

<220>
<221>  VARIANT
<222>  (332)..(332)
<223>  Replace = Asn, Asp or Glu

<220>
<221>  VARIANT
<222>  (333)..(333)
<223>  Replace = Val or Ile

<220>
<221>  VARIANT
<222>  (335)..(335)
<223>  Replace = Glu

<220>
<221>  VARIANT
<222>  (336)..(336)
<223>  Replace = Asp or Glu

<220>
<221>  VARIANT
<222>  (337)..(337)
<223>  Replace = Asp

<220>
<221>  VARIANT
<222>  (338)..(338)
<223>  Replace = Ile

<220>
<221>  VARIANT
<222>  (339)..(339)
<223>  Replace = Arg

<220>
<221>  VARIANT
<222>  (340)..(340)
<223>  Replace = Lys

<220>
<221>  VARIANT
<222>  (341)..(341)
<223>  Replace = Gln or Glu

<220>
<221>  VARIANT
<222>  (344)..(344)
<223>  Replace = Ile

<220>
<221>  VARIANT
<222>  (346)..(346)
<223>  Replace = Pro

<220>
<221>  VARIANT
<222>  (350)..(350)
<223>  Replace = Lys

<220>
<221>  VARIANT
<222>  (351)..(351)
<223>  Replace = Thr or Ser

<220>
<221>  VARIANT
<222>  (353)..(353)
<223>  Replace = Ala

<220>
<221>  VARIANT
<222>  (356)..(356)
<223>  Replace = Ser or Ala

<220>
<221>  VARIANT
<222>  (363)..(363)
<223>  Replace = Ser

<220>
<221>  VARIANT
<222>  (366)..(366)
<223>  Replace = Leu

<220>
<221>  VARIANT
<222>  (369)..(369)
<223>  Replace = Lys or Met

<220>
<221>  VARIANT
<222>  (370)..(370)
<223>  Replace = Lys

<220>
<221>  VARIANT
<222>  (372)..(372)
<223>  Replace = Arg

<220>
<221>  VARIANT
<222>  (373)..(373)
<223>  Replace = Asn or Lys

<220>
<221>  VARIANT
<222>  (374)..(374)
<223>  Replace = Leu or Lys

<220>
<221>  VARIANT
<222>  (377)..(377)
<223>  Replace = Lys, Leu or Glu

<220>
<221>  VARIANT
<222>  (379)..(379)
<223>  Replace = Glu or Val

<220>
<221>  VARIANT
<222>  (381)..(381)

<223>  Replace = Asn

<220>
<221>  VARIANT
<222>  (383)..(383)
<223>  Replace = Arg, Lys or Ile

<220>
<221>  VARIANT
<222>  (384)..(384)
<223>  Replace = Gln or Glu

<220>
<221>  VARIANT
<222>  (387)..(387)
<223>  Replace = Lys

<220>
<221>  VARIANT
<222>  (391)..(391)
<223>  Replace = Glu

<220>
<221>  VARIANT
<222>  (394)..(394)
<223>  Replace = Lys or Glu

<220>
<221>  VARIANT
<222>  (397)..(397)
<223>  Replace = Asp

<220>
<221>  VARIANT
<222>  (398)..(398)
<223>  Replace = Gln or Glu

<220>
<221>  VARIANT
<222>  (401)..(401)
<223>  Replace = Asn or Asp

<220>
<221>  VARIANT
<222>  (405)..(405)
<223>  Replace = Pro

<220>
<221>  VARIANT
<222>  (406)..(406)
<223>  Replace = Glu

<220>
<221>  VARIANT
<222>  (408)..(408)
<223>  Replace = Glu

<220>
<221>  VARIANT
<222>  (409)..(409)
<223>  Replace = Lys or Glu

<220>

<221>  VARIANT
<222>  (412)..(412)
<223>  Replace = Asp or Glu

<220>
<221>  VARIANT
<222>  (416)..(416)
<223>  Replace = Asn or Lys

<220>
<221>  VARIANT
<222>  (417)..(417)
<223>  Replace = Ala

<220>
<221>  VARIANT
<222>  (420)..(420)
<223>  Replace = Phe

<220>
<221>  VARIANT
<222>  (438)..(438)
<223>  Replace = Gln

<220>
<221>  VARIANT
<222>  (439)..(439)
<223>  Replace = Glu or Arg

<220>
<221>  VARIANT
<222>  (440)..(440)
<223>  Replace = Ala or Asp

<220>
<221>  VARIANT
<222>  (442)..(442)
<223>  Replace = Val

<220>
<221>  VARIANT
<222>  (443)..(443)
<223>  Replace = Asn, Gln or Asp

<220>
<221>  VARIANT
<222>  (444)..(444)
<223>  Replace = Asn or Tyr

<220>
<221>  VARIANT
<222>  (447)..(447)
<223>  Replace = Asp or Gly

<220>
<221>  VARIANT
<222>  (448)..(448)
<223>  Replace = Ala or Thr

<220>
<221>  VARIANT
<222>  (451)..(451)
<223>  Replace = Ser

<220>
<221>  VARIANT
<222>  (455)..(455)
<223>  Replace = Glu

<220>
<221>  VARIANT
<222>  (456)..(456)
<223>  Replace = Asp or Arg

<220>
<221>  VARIANT
<222>  (457)..(457)
<223>  Replace = Met or Leu

<220>
<221>  VARIANT
<222>  (458)..(458)
<223>  Replace = Asp, Glu or Asn

<220>
<221>  VARIANT
<222>  (459)..(459)
<223>  Replace = Gly or Lys

<220>
<221>  VARIANT
<222>  (460)..(460)
<223>  Replace = nothing

<220>
<221>  VARIANT
<222>  (461)..(461)
<223>  Replace = Lys

<220>
<221>  VARIANT
<222>  (462)..(462)
<223>  Replace = Tyr or Ala

<220>
<221>  VARIANT
<222>  (464)..(464)
<223>  Replace = Ser or Asn

<220>
<221>  VARIANT
<222>  (466)..(466)
<223>  Replace = Ile

<220>
<221>  VARIANT
<222>  (467)..(467)
<223>  Replace = Leu

<220>
<221>  VARIANT
<222>  (473)..(473)
<223>  Replace = Gln

<220>
<221>  VARIANT

<222>  (476)..(476)
<223>  Replace = Cys

<220>
<221>  VARIANT
<222>  (478)..(478)
<223>  Replace = Ile or Val

<220>
<221>  VARIANT
<222>  (480)..(480)
<223>  Replace = Leu, Cys or Phe

<220>
<221>  VARIANT
<222>  (481)..(481)
<223>  Replace = Lys, Ser or nothing

<220>
<221>  VARIANT
<222>  (482)..(482)
<223>  Replace = nothing

<220>
<221>  VARIANT
<222>  (483)..(483)
<223>  Replace = nothing

<400>  9

Met Asn Val Ala Lys Leu Gly Leu Glu Lys Ile Tyr Ala Asp Pro Ala
1               5                   10                  15

Lys Gln Ser Ala Arg Tyr Asp Asp Ile Thr Ala Arg Phe Lys Gln Leu
            20                  25                  30

Tyr Ala Gly Gln Thr Pro Thr Ile Val Arg Ser Pro Gly Arg Val Asn
        35                  40                  45

Leu Ile Gly Glu His Ile Asp Tyr Ser His Phe Ser Val Met Pro Met
    50                  55                  60

Ala Ile Glu Asp Asp Val Val Met Ala Phe Cys Val Ala Gly Asp Thr
65                  70                  75                  80

Ile Asp Val Ala Asn Leu Glu Asp Asp Lys Phe Pro Ala Glu Ser Tyr
                85                  90                  95

Asp Ile Pro Ser Gly Glu Glu Val Leu Glu Ile Asp Thr Gln Lys Thr
            100                 105                 110

Val Trp Thr Asn Tyr Phe Lys Cys Gly His Thr Val Ala Arg Arg His
        115                 120                 125

Leu Asn Leu Ser Gly Lys Ser Val Arg Asp Val Gly Ile Lys Val Leu

130                 135                 140

Val His Gly Thr Val Pro Asp Ala Cys Gly Leu Ser Ser Ser Ala Ala
145                 150                 155                 160

Phe Val Val Cys Ser Thr Leu Val Ser Leu Ile Ala Cys Gly Gly Gly
                165                 170                 175

Ala Glu Asp Ser Ile Ser Arg Asp Leu Leu Thr Lys Leu Ser Ile Thr
            180                 185                 190

Cys Glu Gln Leu Ile Gly Val Asn Thr Gly Gly Met Asp Gln Ala Ala
        195                 200                 205

Ser Ile Phe Gly Gln Leu Asn His Thr Leu Leu Val Asp Phe Ser Pro
    210                 215                 220

Thr Leu Lys Ala Thr Ser Phe Lys Phe Pro Glu Thr Arg Pro Glu Thr
225                 230                 235                 240

Val Phe Leu Val Ala Asn Thr Leu Val Lys Ser Asn Lys His Glu Thr
                245                 250                 255

Ala Pro Arg Asn Tyr Asn Leu Arg Val Val Glu Ile Thr Ile Ala Ala
            260                 265                 270

Asn Val Ile Ala Lys Lys Leu Lys Gly Leu Gln Leu Val Gln Asn Gly
        275                 280                 285

Asn Leu Asn Lys Gly Thr Phe Asn Gly Leu Met Gln Ala Ala Gly Thr
    290                 295                 300

Asp Leu Ala Gly Leu Glu Gly Leu Ile Asp Glu His Leu Pro Arg Glu
305                 310                 315                 320

Gly Val Ser Leu Glu Glu Ala Ser Glu Leu Leu Gly Ala Ser Val Ala
                325                 330                 335

Glu Val Lys Glu Ser Tyr Phe Val Phe His Val Glu Phe Asp Lys Leu
            340                 345                 350

Gln Leu Tyr Lys Arg Ala Lys His Val Val Thr Glu Ala Ile Arg Val
        355                 360                 365

Leu Glu Phe Gln Ser Met Leu Ser Ser Gly Thr Ser Asp Pro Ser Lys
    370                 375                 380

Leu Gly Asn Leu Met Asn Leu Ser Gln Gln Ser Cys Arg Asp Leu Phe
385                 390                 395                 400

Glu Cys Ser Cys Asp Ala Ile Asp Arg Val Cys Lys Ile Val Leu Ala
                405                 410                 415

Ser Gly Gly Leu Gly Ser Arg Leu Thr Gly Ala Gly Trp Gly Gly Ser
            420                 425                 430

Thr Val His Leu Val Glu Lys Glu Lys Leu Ser Ser Val Ile Glu Ser
        435                 440                 445

Leu Lys Lys Glu Tyr Tyr Ser Gly Ile Gly Glu Val Asp Phe Asp Thr
    450                 455                 460

Ala Met Val Val Thr Val Pro Gly Glu Gly Thr Ala Phe Val Asp Ile
465                 470                 475                 480

Gln Leu His

<210>  10
<211>  476
<212>  PRT
<213>  Yarrowia galli

<400>  10

Met Ser Ile Ser Thr Leu Pro Leu Ser Lys Ile Tyr Ala Asp Pro Ser
1               5                   10                  15

Lys His Gln Thr Arg Tyr Glu Glu Leu Thr Lys Arg Phe Ser Gln Leu
            20                  25                  30

Tyr Pro Gly Ser Thr Ala Thr Leu Val Arg Ser Pro Gly Arg Val Asn
        35                  40                  45

Leu Ile Gly Glu His Ile Asp Tyr Ser His Phe Ser Val Met Pro Met
    50                  55                  60

Ala Ile Glu Asp Asp Val Ile Met Ala Phe Ser Val Gly Gly Asp Ser
65                  70                  75                  80

Ile Glu Ile Ala Asn Met Glu Asp Glu Lys Phe Pro Ala Glu Lys Tyr
                85                  90                  95

Asp Ile Pro Thr Ala Thr Glu Val Leu Thr Ile Asp Gln Thr Lys Thr
            100                 105                 110

Val Trp Thr Asn Tyr Phe Lys Cys Gly His Thr Val Gly Arg Lys Tyr
        115                 120                 125

Leu Arg Glu Ser Gly Glu Glu Val Lys Pro Val Gly Ile Lys Val Leu
    130                 135                 140

Val His Gly Thr Val Pro Asp Ala Cys Gly Leu Ser Ser Ser Ala Ala
145                 150                 155                 160

Phe Val Val Cys Ser Thr Leu Val Thr Leu Leu Ser Gln Gly Met Lys
                165                 170                 175

Glu Val Ser Arg Asp Leu Leu Thr Lys Leu Ser Ile Thr Cys Glu Gln
            180                 185                 190

Leu Ile Gly Val Asn Thr Gly Gly Met Asp Gln Ala Ala Ser Ile Tyr
        195                 200                 205

Gly Gln Leu Asn His Thr Leu Leu Val Asp Phe Ala Pro Thr Leu Lys
    210                 215                 220

Ala Thr Pro Phe Gln Phe Pro Ala Thr Thr Pro Lys Thr Val Phe Leu
225                 230                 235                 240

Val Ala Asn Thr Leu Val Lys Ser Asn Lys His Glu Thr Ala Pro Arg
                245                 250                 255

Asn Tyr Asn Leu Arg Val Val Glu Ile Thr Leu Ala Val Asn Val Leu
            260                 265                 270

Leu Lys Ser Leu Asn Gly Val Ser Ile Pro Gln Asn Gly Asn Leu Asn
        275                 280                 285

Lys Gly Thr Leu His Gly Leu Met Lys Ala Val Gly Thr Asp Leu Asn
    290                 295                 300

Gly Leu Leu Asp Leu Val Glu Thr Leu Pro Lys Glu Pro Val Thr Leu
305                 310                 315                 320

Gln Glu Val Ala Lys Leu Leu Asp Ile Ser Glu Glu Asp Val Lys Lys
                325                 330                 335

Gln Tyr Phe Ile Phe Pro Val Glu Phe Asp Thr Leu Ala Leu Tyr Ala
            340                 345                 350

Arg Ala Lys His Val Val Thr Glu Ala Ile Arg Val Leu Glu Phe Gln
        355                 360                 365

Ser Leu Leu Ser Lys Gly Glu Ser Asp Pro Lys Glu Leu Gly Lys Leu
    370                 375                 380

Met Asn Glu Ser Gln Lys Ser Cys Asp Gln Leu Phe Asn Cys Ser Cys
385                 390                 395                 400

Pro Ala Ile Glu Lys Val Cys Glu Ile Val Leu Lys Ser Gly Gly Phe
                405                 410                 415

Gly Ser Arg Leu Thr Gly Ala Gly Trp Gly Gly Ser Thr Val His Leu
            420                 425                 430

Val Glu Glu Ala Lys Leu Asn Asn Val Ile Gly Ala Leu Lys Lys Glu
        435                 440                 445

Tyr Tyr Ser Asp Met Glu Gly Val Asp Tyr Asp Ser Ala Met Val Val
    450                 455                 460

Thr Val Pro Gly Glu Gly Cys Ala Ile Val Asp Leu
465                 470                 475

<210>  11
<211>  476
<212>  PRT
<213>  Yarrowia yakushimensis

<400>  11

Met Ser Ile Ala Thr Leu Pro Leu Ser Lys Ile Tyr Ala Asp Pro Ala
1               5                   10                  15

Arg His Gln Ala Arg Tyr Glu Gln Leu Thr Lys Arg Phe Thr Glu Leu
            20                  25                  30

Tyr Gly Ala Lys Pro Thr Val Val Arg Ser Pro Gly Arg Val Asn Leu
        35                  40                  45

Ile Gly Glu His Ile Asp Tyr Ser His Phe Ser Val Met Pro Met Ala
    50                  55                  60

Ile Glu Asp Asp Val Ile Met Ala Phe Ser Val Gly Gly Asp Ser Ile
65                  70                  75                  80

Glu Ile Ala Asn Ile Glu Asp Glu Lys Phe Pro Ala Glu Lys Tyr Asp
                85                  90                  95

Ile Pro Ala Pro Asn Glu Val Leu Thr Ile Asp Gln Lys Lys Thr Val
            100                 105                 110

Trp Thr Asn Tyr Phe Lys Cys Gly His Thr Val Ala Arg Lys His Phe
        115                 120                 125

Arg Asp Ser Gly Asp Asp Val Lys Pro Val Gly Ile Lys Ile Leu Val
    130                 135                 140

His Gly Thr Val Pro Asp Ala Cys Gly Leu Ser Ser Ser Ala Ala Phe
145                 150                 155                 160

Val Val Cys Ser Thr Leu Val Thr Leu Leu Ser Gln Gly Leu Thr Glu
                165                 170                 175

Val Ser Arg Asp Leu Leu Thr Thr Leu Ser Ile Thr Cys Glu Gln Leu
            180                 185                 190

Ile Gly Val Asn Thr Gly Gly Met Asp Gln Ala Ala Ser Ile Tyr Gly
        195                 200                 205

Gln Leu Asn His Thr Leu Leu Val Asp Phe Ala Pro Val Leu Lys Ala
    210                 215                 220

Thr Pro Phe Leu Phe Pro Ser Thr Val Pro Lys Thr Val Phe Leu Val
225                 230                 235                 240

Ala Asn Thr Leu Val Lys Ser Asn Lys His Glu Thr Ala Pro Arg Asn
                245                 250                 255

Tyr Asn Leu Arg Val Val Glu Ile Thr Leu Ala Val Asn Val Leu Leu
            260                 265                 270

Lys Gly Leu Lys Asp Val Thr Ile Pro Gln Asn Gly Asn Leu Asn Lys
        275                 280                 285

Gly Thr Leu Arg Gly Leu Met Glu Ala Val Gly Thr Asp Leu His Gly
    290                 295                 300

Leu Leu Asp Leu Val Glu Thr Leu Pro Lys Glu Pro Val Thr Leu Gln
305                 310                 315                 320

Glu Val Ala Lys Leu Leu Asp Ile Ser Glu Glu Asp Val Lys Lys Gln
                325                 330                 335

Tyr Phe Ile Phe Pro Val Glu Phe Asp Thr Leu Ala Leu Tyr Ser Arg
            340                 345                 350

Ala Lys His Val Val Thr Glu Ala Leu Arg Val Leu Glu Phe Gln Lys
        355                 360                 365

Lys Leu Ser Leu Gly Glu Ser Asp Pro Lys Gln Leu Gly Lys Leu Met
    370                 375                 380

Asn Glu Ser Gln Lys Ser Cys Asp Gln Leu Phe Asn Cys Ser Cys Pro
385                 390                 395                 400

Ala Ile Glu Glu Val Cys Glu Ile Val Leu Lys Ser Gly Gly Leu Gly
                405                 410                 415

Ser Arg Leu Thr Gly Ala Gly Trp Gly Gly Ser Thr Val His Leu Val
            420                 425                 430

Glu Glu Ala Lys Leu Gln Asn Val Ile Asp Ala Leu Lys Lys Glu Tyr
        435                 440                 445

Tyr Ser Asp Met Glu Gly Val Asp Tyr Asp Asn Ala Met Val Val Thr
    450                 455                 460

Val Pro Gly Glu Gly Cys Ala Val Val Asp Cys Lys
465                 470                 475

<210>  12
<211>  478
<212>  PRT
<213>  Yarrowia alimentaria

<400>  12

Met Asn Val Ala Lys Leu Gly Leu Glu Lys Ile Tyr Ala Asp Pro Ala
1               5                   10                  15

Lys Gln Ser Ala Arg Tyr Asp Asp Ile Thr Ala Arg Phe Lys Gln Leu
            20                  25                  30

Tyr Gly Gln Thr Pro Thr Ile Val Arg Ser Pro Gly Arg Val Asn Leu
        35                  40                  45

Ile Gly Glu His Ile Asp Tyr Ser His Phe Ser Val Met Pro Met Ala
    50                  55                  60

Ile Glu Asp Asp Val Val Met Ala Phe Cys Val Ala Gly Asp Thr Ile
65                  70                  75                  80

Asp Val Ala Asn Leu Glu Asp Asp Lys Phe Pro Ala Glu Ser Tyr Asp
                85                  90                  95

Ile Pro Ser Gly Glu Glu Val Leu Glu Ile Asp Thr Gln Lys Thr Val
            100                 105                 110

Trp Thr Asn Tyr Phe Lys Cys Gly His Thr Val Ala Arg Arg His Leu
        115                 120                 125

Asn Leu Ser Gly Lys Ser Arg Asp Val Gly Ile Lys Val Leu Val His
    130                 135                 140

Gly Thr Val Pro Asp Ala Cys Gly Leu Ser Ser Ser Ala Ala Phe Val
145                 150                 155                 160

Val Cys Ser Thr Leu Val Ser Leu Ile Ala Cys Gly Gly Gly Ala Glu
                165                 170                 175

Asp Ser Ile Ser Arg Asp Leu Leu Thr Lys Leu Ser Ile Thr Cys Glu
            180                 185                 190

Gln Leu Ile Gly Val Asn Thr Gly Gly Met Asp Gln Ala Ala Ser Ile
        195                 200                 205

Phe Gly Gln Leu Asn His Thr Leu Leu Val Asp Phe Ser Pro Thr Leu
    210                 215                 220

Lys Ala Thr Ser Phe Lys Phe Pro Glu Thr Arg Pro Glu Thr Val Phe
225                 230                 235                 240

Leu Val Ala Asn Thr Leu Val Lys Ser Asn Lys His Glu Thr Ala Pro
                245                 250                 255

Arg Asn Tyr Asn Leu Arg Val Val Glu Ile Thr Ile Ala Ala Asn Val
            260                 265                 270

Ile Ala Lys Lys Leu Gly Leu Gln Leu Val Gln Asn Gly Asn Leu Asn
        275                 280                 285

Lys Gly Thr Phe Asn Gly Leu Met Gln Ala Ala Gly Thr Asp Leu Ala
    290                 295                 300

Gly Leu Glu Gly Leu Ile Asp Glu His Leu Pro Arg Glu Gly Val Ser
305                 310                 315                 320

Leu Glu Glu Ala Ser Glu Leu Leu Gly Ala Ser Val Ala Glu Val Lys
                325                 330                 335

Glu Ser Tyr Phe Val Phe His Val Glu Phe Asp Lys Leu Gln Leu Tyr
            340                 345                 350

Lys Arg Ala Lys His Val Val Thr Glu Ala Ile Arg Val Leu Glu Phe

355                 360                 365

Gln Ser Met Leu Ser Ser Gly Thr Ser Asp Pro Ser Lys Leu Gly Asn
    370                 375                 380

Leu Met Asn Leu Ser Gln Gln Ser Cys Arg Asp Leu Phe Glu Cys Ser
385                 390                 395                 400

Cys Asp Ala Ile Asp Arg Val Cys Lys Ile Val Leu Ala Ser Gly Gly
                405                 410                 415

Leu Gly Ser Arg Leu Thr Gly Ala Gly Trp Gly Gly Ser Thr Val His
            420                 425                 430

Leu Val Glu Lys Glu Lys Leu Ser Ser Val Ile Glu Ser Leu Lys Lys
        435                 440                 445

Glu Tyr Tyr Ser Gly Ile Gly Glu Val Asp Phe Asp Thr Ala Met Val
    450                 455                 460

Val Thr Val Pro Gly Glu Gly Thr Ala Phe Val Asp Ile Gln
465                 470                 475

<210>  13
<211>  477
<212>  PRT
<213>  Yarrowia phangngensis

<400>  13

Met Ser Ile Thr Lys Leu Pro Leu Glu Lys Ile Tyr Ser Ser Pro Glu
1               5                   10                  15

Thr Ala Arg Lys Arg Tyr Val Asn Ile Val Thr Arg Phe Arg Glu Leu
            20                  25                  30

Tyr Gly Cys Thr Pro Thr Leu Val Arg Ala Pro Gly Arg Val Asn Leu
        35                  40                  45

Ile Gly Glu His Ile Asp Tyr Ser His Phe Ser Val Met Pro Met Ala
    50                  55                  60

Ile Gly Asp Asp Val Ile Met Ala Phe Cys Val Glu Gly Asp Lys Ile
65                  70                  75                  80

Arg Ile Ala Asn Leu Glu Asp Glu Lys Phe Pro Ser Glu Ile Tyr His
                85                  90                  95

Cys Pro Asp Ala Asp Cys Glu Leu Glu Ile Asp Gln Thr Lys Thr Val

            100                 105                 110

Trp Thr Asn Tyr Phe Lys Cys Gly Phe Thr Val Gly Arg Lys Tyr Val
        115                 120                 125

Arg Glu Thr His Gly Ser Leu Gln Pro Leu Gly Ile Lys Ile Leu Val
    130                 135                 140

His Gly Thr Val Pro Asp Ala Cys Gly Leu Ser Ser Ser Ala Ala Phe
145                 150                 155                 160

Val Val Cys Ser Thr Leu Val Thr Leu Met Ala Tyr Gly Glu Thr Gln
                165                 170                 175

Val Ser Arg Glu Leu Leu Thr Lys Leu Ser Ile Thr Cys Glu Gln Leu
            180                 185                 190

Val Gly Val Asn Thr Gly Gly Met Asp Gln Ala Ala Ser Ile Tyr Gly
        195                 200                 205

Val Leu Asn His Thr Leu Leu Val Asp Phe Ala Pro Lys Leu Arg Ala
    210                 215                 220

Thr Pro Phe Leu Phe Pro Glu Thr Ser Pro Ser Ala Val Phe Leu Val
225                 230                 235                 240

Ala Asn Thr Leu Ile Arg Ser Asn Lys His Glu Thr Ala Pro Arg Asn
                245                 250                 255

Tyr Asn Leu Arg Val Val Glu Ile Thr Leu Ala Val Asn Ile Leu Ala
            260                 265                 270

His Lys Leu Gly Leu Lys Ile Pro Gln Asp Gly Asn Leu Asn Lys Gly
        275                 280                 285

Thr Phe His Gly Leu Leu Glu Ala Cys Asn Thr Asp Leu Thr Gly Leu
    290                 295                 300

Leu Lys Leu Val Asp Phe His Leu Pro Arg Asp Pro Leu Thr Leu Glu
305                 310                 315                 320

Glu Val Ser Thr Leu Leu Glu Ile Ser Glu Asp Glu Ile Arg Lys Glu
                325                 330                 335

Tyr Phe Val Phe Pro Val Glu Phe Lys Ser Leu Ala Leu Tyr Ser Arg
            340                 345                 350

Ala Lys His Val Val Ser Glu Ala Ile Arg Val Met Lys Phe Arg Lys
        355                 360                 365

Lys Leu Ser Glu Gly Val Ser Asn Pro Ile Glu Leu Gly Lys Leu Met
    370                 375                 380

Asn Glu Ser Gln Glu Ser Cys Arg Glu Leu Phe Asp Cys Ser Cys Pro
385                 390                 395                 400

Glu Ile Asp Arg Val Cys Glu Ile Val Leu Lys Ala Gly Gly Phe Gly
                405                 410                 415

Ser Arg Leu Thr Gly Ala Gly Trp Gly Gly Ser Thr Val His Leu Val
            420                 425                 430

Gln Arg Asp Lys Val Asp Tyr Val Ile Asp Thr Leu Lys Ser Glu Tyr
        435                 440                 445

Tyr Glu Arg Leu Asn Lys Lys Ala Asp Ser Ala Ile Leu Val Thr Val
    450                 455                 460

Pro Gly Gln Gly Cys Ala Ile Val Asp Phe Ser Leu His
465                 470                 475

<210>  14
<211>  355
<212>  PRT
<213>  Artificial Sequence

<220>
<223>  GAL7 consensus sequence


<220>
<221>  VARIANT
<222>  (4)..(4)
<223>  Replace = Val or Glu

<220>
<221>  VARIANT
<222>  (5)..(5)
<223>  Replace = Ala

<220>
<221>  VARIANT
<222>  (6)..(6)
<223>  Replace = Ser or Phe

<220>
<221>  VARIANT
<222>  (18)..(18)
<223>  Replace = Gln

<220>
<221>  VARIANT

<222>  (40)..(40)
<223>  Replace = Thr or Phe

<220>
<221>  VARIANT
<222>  (43)..(43)
<223>  Replace = Thr or Asp

<220>
<221>  VARIANT
<222>  (49)..(49)
<223>  Replace = Glu

<220>
<221>  VARIANT
<222>  (56)..(56)
<223>  Replace = Cys

<220>
<221>  VARIANT
<222>  (58)..(58)
<223>  Replace = Ala or Glu

<220>
<221>  VARIANT
<222>  (60)..(60)
<223>  Replace = Val

<220>
<221>  VARIANT
<222>  (64)..(64)
<223>  Replace = Met or Lys

<220>
<221>  VARIANT
<222>  (69)..(69)
<223>  Replace = Lys

<220>
<221>  VARIANT
<222>  (70)..(70)
<223>  Replace = Asp

<220>
<221>  VARIANT
<222>  (72)..(72)
<223>  Replace = Tyr

<220>
<221>  VARIANT
<222>  (75)..(75)
<223>  Replace = Asn

<220>
<221>  VARIANT
<222>  (83)..(83)
<223>  Replace = Glu

<220>
<221>  VARIANT
<222>  (84)..(84)
<223>  Replace = Arg

<220>
<221>  VARIANT
<222>  (86)..(86)
<223>  Replace = Ala or Asp

<220>
<221>  VARIANT
<222>  (88)..(88)
<223>  Replace = Val

<220>
<221>  VARIANT
<222>  (90)..(90)
<223>  Replace = Lys or Glu

<220>
<221>  VARIANT
<222>  (91)..(91)
<223>  Replace = Val

<220>
<221>  VARIANT
<222>  (92)..(92)
<223>  Replace = Val, Ile or Leu

<220>
<221>  VARIANT
<222>  (93)..(93)
<223>  Replace = Glu or Arg

<220>
<221>  VARIANT
<222>  (95)..(95)
<223>  Replace = nothing

<220>
<221>  VARIANT
<222>  (96)..(96)
<223>  Replace = nothing

<220>
<221>  VARIANT
<222>  (97)..(97)
<223>  Replace = Ala

<220>
<221>  VARIANT
<222>  (98)..(98)
<223>  Replace = Gln, Ser or Asn

<220>
<221>  VARIANT
<222>  (99)..(99)
<223>  Replace = Lys or Glu

<220>
<221>  VARIANT
<222>  (100)..(100)
<223>  Replace = nothing

<220>
<221>  VARIANT
<222>  (101)..(101)

<223>  Replace = Glu or Lys

<220>
<221>  VARIANT
<222>  (102)..(102)
<223>  Replace = His, Glu, Gln or Asn

<220>
<221>  VARIANT
<222>  (103)..(103)
<223>  Replace = Thr

<220>
<221>  VARIANT
<222>  (104)..(104)
<223>  Replace = Glu, Lys or Gly

<220>
<221>  VARIANT
<222>  (105)..(105)
<223>  Replace = Ser, Pro or Phe

<220>
<221>  VARIANT
<222>  (106)..(106)
<223>  Replace = Thr, Ser or His

<220>
<221>  VARIANT
<222>  (107)..(107)
<223>  Replace = Ala or Glu

<220>
<221>  VARIANT
<222>  (108)..(108)
<223>  Replace = Leu

<220>
<221>  VARIANT
<222>  (109)..(109)
<223>  Replace = Asp

<220>
<221>  VARIANT
<222>  (110)..(110)
<223>  Replace = Leu, Arg or Ser

<220>
<221>  VARIANT
<222>  (111)..(111)
<223>  Replace = Leu or Asn

<220>
<221>  VARIANT
<222>  (114)..(114)
<223>  Replace = Gln or Asn

<220>
<221>  VARIANT
<222>  (115)..(115)
<223>  Replace = Lys or Arg

<220>

<221>  VARIANT
<222>  (116)..(116)
<223>  Replace = Glu

<220>
<221>  VARIANT
<222>  (117)..(117)
<223>  Replace = Asp or Glu

<220>
<221>  VARIANT
<222>  (118)..(118)
<223>  Replace = Val

<220>
<221>  VARIANT
<222>  (137)..(137)
<223>  Replace = Ser

<220>
<221>  VARIANT
<222>  (140)..(140)
<223>  Replace = Pro

<220>
<221>  VARIANT
<222>  (141)..(141)
<223>  Replace = Glu or Thr

<220>
<221>  VARIANT
<222>  (144)..(144)
<223>  Replace = Gln

<220>
<221>  VARIANT
<222>  (145)..(145)
<223>  Replace = Lys, Arg or Ala

<220>
<221>  VARIANT
<222>  (147)..(147)
<223>  Replace = Val

<220>
<221>  VARIANT
<222>  (148)..(148)
<223>  Replace = Glu, Ala or Asn

<220>
<221>  VARIANT
<222>  (151)..(151)
<223>  Replace = Arg

<220>
<221>  VARIANT
<222>  (152)..(152)
<223>  Replace = Lys

<220>
<221>  VARIANT
<222>  (153)..(153)
<223>  Replace = Ile

<220>
<221>  VARIANT
<222>  (155)..(155)
<223>  Replace = Thr or Glu

<220>
<221>  VARIANT
<222>  (159)..(159)
<223>  Replace = Ser or Arg

<220>
<221>  VARIANT
<222>  (160)..(160)
<223>  Replace = Lys

<220>
<221>  VARIANT
<222>  (161)..(161)
<223>  Replace = Glu

<220>
<221>  VARIANT
<222>  (173)..(173)
<223>  Replace = Ser

<220>
<221>  VARIANT
<222>  (174)..(174)
<223>  Replace = Ala

<220>
<221>  VARIANT
<222>  (184)..(184)
<223>  Replace = Cys

<220>
<221>  VARIANT
<222>  (191)..(191)
<223>  Replace = Ile

<220>
<221>  VARIANT
<222>  (194)..(194)
<223>  Replace = Asp

<220>
<221>  VARIANT
<222>  (198)..(198)
<223>  Replace = Leu

<220>
<221>  VARIANT
<222>  (200)..(200)
<223>  Replace = Phe

<220>
<221>  VARIANT
<222>  (201)..(201)
<223>  Replace = Asp

<220>
<221>  VARIANT

<222>  (202)..(202)
<223>  Replace = Ser

<220>
<221>  VARIANT
<222>  (204)..(204)
<223>  Replace = Lys

<220>
<221>  VARIANT
<222>  (205)..(205)
<223>  Replace = Lys

<220>
<221>  VARIANT
<222>  (208)..(208)
<223>  Replace = Glu

<220>
<221>  VARIANT
<222>  (209)..(209)
<223>  Replace = Glu

<220>
<221>  VARIANT
<222>  (210)..(210)
<223>  Replace = Asn

<220>
<221>  VARIANT
<222>  (211)..(211)
<223>  Replace = Asn or Lys

<220>
<221>  VARIANT
<222>  (219)..(219)
<223>  Replace = Val

<220>
<221>  VARIANT
<222>  (220)..(220)
<223>  Replace = Asn, His, Gln or Glu

<220>
<221>  VARIANT
<222>  (223)..(223)
<223>  Replace = Met

<220>
<221>  VARIANT
<222>  (224)..(224)
<223>  Replace = Ser or Gln

<220>
<221>  VARIANT
<222>  (226)..(226)
<223>  Replace = Asn or Glu

<220>
<221>  VARIANT
<222>  (229)..(229)
<223>  Replace = Ile

<220>
<221>  VARIANT
<222>  (232)..(232)
<223>  Replace = Gln

<220>
<221>  VARIANT
<222>  (234)..(234)
<223>  Replace = Ala or Glu

<220>
<221>  VARIANT
<222>  (253)..(253)
<223>  Replace = Val

<220>
<221>  VARIANT
<222>  (256)..(256)
<223>  Replace = Glu

<220>
<221>  VARIANT
<222>  (258)..(258)
<223>  Replace = Val

<220>
<221>  VARIANT
<222>  (259)..(259)
<223>  Replace = Ser

<220>
<221>  VARIANT
<222>  (261)..(261)
<223>  Replace = Ile

<220>
<221>  VARIANT
<222>  (262)..(262)
<223>  Replace = Lys

<220>
<221>  VARIANT
<222>  (265)..(265)
<223>  Replace = Ser

<220>
<221>  VARIANT
<222>  (266)..(266)
<223>  Replace = Gln or Asn

<220>
<221>  VARIANT
<222>  (267)..(267)
<223>  Replace = His or Glu

<220>
<221>  VARIANT
<222>  (269)..(269)
<223>  Replace = Ala

<220>
<221>  VARIANT
<222>  (270)..(270)

<223>  Replace = Gln, Glu or Ser

<220>
<221>  VARIANT
<222>  (271)..(271)
<223>  Replace = His

<220>
<221>  VARIANT
<222>  (275)..(275)
<223>  Replace = Ser

<220>
<221>  VARIANT
<222>  (279)..(279)
<223>  Replace = Ile

<220>
<221>  VARIANT
<222>  (281)..(281)
<223>  Replace = Lys

<220>
<221>  VARIANT
<222>  (285)..(285)
<223>  Replace = Asp

<220>
<221>  VARIANT
<222>  (288)..(288)
<223>  Replace = Lys

<220>
<221>  VARIANT
<222>  (304)..(304)
<223>  Replace = Val

<220>
<221>  VARIANT
<222>  (305)..(305)
<223>  Replace = Glu

<220>
<221>  VARIANT
<222>  (306)..(306)
<223>  Replace = Asp

<220>
<221>  VARIANT
<222>  (308)..(308)
<223>  Replace = Pro

<220>
<221>  VARIANT
<222>  (345)..(345)
<223>  Replace = Thr

<220>
<221>  VARIANT
<222>  (347)..(347)
<223>  Replace = Glu

<220>

<221>  VARIANT
<222>  (350)..(350)
<223>  Replace = Lys

<220>
<221>  VARIANT
<222>  (353)..(353)
<223>  Replace = Tyr, Phe or Arg

<220>
<221>  VARIANT
<222>  (354)..(354)
<223>  Replace = Asp or Glu

<400>  14

Met Thr Leu Ser Pro Ala Asp Ile Pro His Arg Arg Tyr Asn Pro Leu
1               5                   10                  15

Arg Lys Ser Trp Val Leu Val Ser Pro His Arg Ala Lys Arg Pro Trp
            20                  25                  30

Gln Gly Gln Gln Glu Glu Pro Val Lys Asp Ser Lys Pro Glu Tyr Asp
        35                  40                  45

Asp Lys Cys Tyr Leu Cys Pro Arg Asn Lys Arg Ile Asn Gly Glu Leu
    50                  55                  60

Asn Pro Asp Tyr Gln Ser Thr Phe Val Phe Glu Asn Asp Phe Ser Ala
65                  70                  75                  80

Val Phe Pro Lys Thr Glu Glu Ile Glu Ser Leu Met Lys Glu Glu Lys
                85                  90                  95

Ser Glu Gln Lys Thr Ser Ser Ser Leu Ala Gly Ile Glu Ala Asp Leu
            100                 105                 110

Leu Arg Gln Asp Pro Thr Phe Gly Tyr Cys Gln Val Ile Cys Phe Ser
        115                 120                 125

Pro Lys His Asn Val Thr Leu Ala Thr Met Thr Val Pro Gln Ile Glu
    130                 135                 140

Gln Val Ile Tyr Thr Trp Gln Asp Met Tyr Arg Glu Ala Gln Thr Arg
145                 150                 155                 160

Gln Ile Lys Tyr Ser Leu Ile Phe Glu Asn Lys Gly Ala Thr Met Gly
                165                 170                 175

Cys Ser Asn Pro His Ala His Gly Gln Val Trp Asn Thr Ser Val Val
            180                 185                 190

Pro Glu Glu Pro Gln Met Glu Tyr Asn Ala Leu Arg Glu Tyr Lys Asp
        195                 200                 205

Ser Lys Ser Arg Asp Leu Leu Val Asp Tyr Ala Ala Leu Glu Leu Glu
    210                 215                 220

Lys Lys Glu Arg Val Val Tyr Glu Asn Ser Ser Phe Leu Val Val Val
225                 230                 235                 240

Pro Tyr Trp Ala Val Trp Pro Phe Glu Thr Met Ile Phe Ser Lys Gln
                245                 250                 255

Arg Ile Ala Ser Val Ile Lys Leu Thr Lys Pro Gln Val Ala Asp Leu
            260                 265                 270

Ala Asp Ala Ile Ser Val Leu Thr Arg Lys Tyr Asp Lys Leu Phe Asn
        275                 280                 285

Thr Ser Phe Pro Tyr Ser Met Gly Ile His Gln Ser Phe Val Asp Glu
    290                 295                 300

Pro Asn Cys Glu Tyr Glu His Leu Arg Leu Leu Phe Tyr Pro Pro Leu
305                 310                 315                 320

Leu Arg Ser Ala Ser Val Lys Lys Phe Gln Val Gly Phe Glu Met Leu
                325                 330                 335

Gly Met Pro Gln Arg Asp Ile Thr Ala Glu Gly Ala Ala Gln Arg Leu
            340                 345                 350

Lys Gly Leu
        355

<210>  15
<211>  353
<212>  PRT
<213>  Yarrowia galli

<400>  15

Met Thr Leu Val Ala Ser Asp Ile Pro His Arg Arg Tyr Asn Pro Leu
1               5                   10                  15

Arg Gln Ser Trp Val Leu Val Ser Pro His Arg Ala Lys Arg Pro Trp
            20                  25                  30

Gln Gly Gln Gln Glu Glu Pro Thr Lys Asp Thr Lys Pro Glu Tyr Asp
        35                  40                  45

Asp Lys Cys Tyr Leu Cys Pro Arg Asn Ala Arg Ile Asn Gly Glu Met
    50                  55                  60

Asn Pro Asp Tyr Lys Asp Thr Phe Val Phe Glu Asn Asp Phe Ser Ala
65                  70                  75                  80

Val Phe Glu Lys Thr Ala Glu Val Glu Lys Val Ile Glu Glu Ala Gln
                85                  90                  95

Lys Lys Glu Glu Ser Lys Pro Ser Glu Leu Glu Leu Asp Leu Leu Arg
            100                 105                 110

Lys Asp Asp Val Phe Gly Tyr Cys Gln Val Ile Cys Phe Ser Pro Lys
        115                 120                 125

His Asn Val Thr Leu Ala Thr Met Thr Val Glu Gln Ile Gln Arg Val
    130                 135                 140

Ile Glu Thr Trp Gln Lys Met Tyr Thr Glu Ala Gln Ser Arg Gly Ile
145                 150                 155                 160

Lys Tyr Ser Leu Ile Phe Glu Asn Lys Gly Ala Thr Met Gly Cys Ser
                165                 170                 175

Asn Pro His Ala His Gly Gln Val Trp Asn Thr Ser Val Val Pro Glu
            180                 185                 190

Glu Pro Gln Met Glu Phe Asp Ser Leu Lys Lys Tyr Lys Asp Glu Lys
        195                 200                 205

Lys Arg Asp Leu Leu Val Asp Tyr Val His Leu Glu Met Ser Lys Asn
    210                 215                 220

Glu Arg Ile Val Tyr Gln Asn Ala Ser Phe Leu Val Val Val Pro Tyr
225                 230                 235                 240

Trp Ala Val Trp Pro Phe Glu Thr Met Ile Val Ser Lys Glu Arg Val
                245                 250                 255

Ser Ser Ile Lys Lys Leu Thr Gln Glu Gln Val Glu His Leu Ala Asp
            260                 265                 270

Ala Ile Ser Val Leu Thr Lys Lys Tyr Asp Lys Leu Phe Asn Thr Ser
        275                 280                 285

Phe Pro Tyr Ser Met Gly Ile His Gln Ser Phe Val Asp Val Glu Asp
    290                 295                 300

Cys Pro Tyr Glu His Leu Arg Leu Leu Phe Tyr Pro Pro Leu Leu Arg
305                 310                 315                 320

Ser Ala Ser Val Lys Lys Phe Gln Val Gly Phe Glu Met Leu Gly Met
                325                 330                 335

Pro Gln Arg Asp Ile Thr Ala Glu Gly Ala Ala Lys Arg Leu Phe Asp
            340                 345                 350

Leu

<210>  16
<211>  352
<212>  PRT
<213>  Yarrowia yakushimensis

<400>  16

Met Thr Leu Val Ala Ser Asp Ile Pro His Arg Arg Tyr Asn Pro Leu
1               5                   10                  15

Arg Gln Ser Trp Val Leu Val Ser Pro His Arg Ala Lys Arg Pro Trp
            20                  25                  30

Gln Gly Gln Gln Glu Glu Pro Thr Lys Asp Thr Lys Pro Glu Tyr Asp
        35                  40                  45

Glu Lys Cys Tyr Leu Cys Pro Arg Asn Ala Arg Ile Asn Gly Glu Met
    50                  55                  60

Asn Pro Asp Tyr Lys Asp Thr Phe Val Phe Glu Asn Asp Phe Ser Ala
65                  70                  75                  80

Val Phe Glu Lys Thr Ala Glu Val Glu Lys Val Ile Glu Glu Ala Ser
                85                  90                  95

Lys Lys Gln Ser Glu Ser Ser Glu Leu Glu Arg Leu Leu Leu Gln Lys
            100                 105                 110

Glu Asp Val Phe Gly Tyr Cys Gln Val Ile Cys Phe Ser Pro Lys His
        115                 120                 125

Asn Val Thr Leu Ala Thr Met Thr Val Glu Gln Ile Glu Lys Val Ile
    130                 135                 140

Ala Thr Trp Gln Lys Met Tyr Thr Glu Ala Gln Ser Arg Gly Ile Lys
145                 150                 155                 160

Tyr Ser Leu Ile Phe Glu Asn Lys Gly Ala Thr Met Gly Cys Ser Asn
                165                 170                 175

Pro His Ala His Gly Gln Val Trp Asn Thr Ser Val Val Pro Glu Glu
            180                 185                 190

Pro Gln Met Glu Tyr Asp Ser Leu Lys Lys Tyr Lys Glu Glu Asn Lys
        195                 200                 205

Arg Asp Leu Leu Val Asp Tyr Val Gln Leu Glu Leu Ser Lys Asn Glu
    210                 215                 220

Arg Ile Val Tyr Gln Asn Ala Ser Phe Leu Val Val Val Pro Tyr Trp
225                 230                 235                 240

Ala Val Trp Pro Phe Glu Thr Met Ile Val Ser Lys Glu Arg Val Ser
                245                 250                 255

Ser Ile Lys Lys Leu Thr Lys Glu Gln Ala Glu His Leu Ala Asp Ala
            260                 265                 270

Ile Ser Val Leu Thr Lys Lys Tyr Asp Lys Leu Phe Asn Thr Ser Phe
        275                 280                 285

Pro Tyr Ser Met Gly Ile His Gln Ser Phe Val Asp Val Glu Asp Cys
    290                 295                 300

Pro Tyr Glu His Leu Arg Leu Leu Phe Tyr Pro Pro Leu Leu Arg Ser
305                 310                 315                 320

Ala Ser Val Lys Lys Phe Gln Val Gly Phe Glu Met Leu Gly Met Pro
                325                 330                 335

Gln Arg Asp Ile Thr Ala Glu Gly Ala Ala Lys Arg Leu Tyr Glu Leu
            340                 345                 350

<210>  17
<211>  352
<212>  PRT
<213>  Yarrowia phangngensis

<400>  17

Met Thr Leu Glu Ala Phe Asp Ile Pro His Arg Arg Tyr Asn Pro Leu
1               5                   10                  15

Arg Gln Ser Trp Val Leu Val Ser Pro His Arg Ala Lys Arg Pro Trp
            20                  25                  30

Gln Gly Gln Gln Glu Glu Pro Phe Lys Asp Asp Lys Pro Glu Tyr Asp
        35                  40                  45

Glu Lys Cys Tyr Leu Cys Pro Cys Asn Glu Arg Val Asn Gly Glu Lys
    50                  55                  60

Asn Pro Asp Tyr Gln Asp Thr Tyr Val Phe Asn Asn Asp Phe Ser Ala
65                  70                  75                  80

Val Phe Glu Arg Thr Asp Glu Val Glu Glu Leu Leu Arg Glu Ser Asn
                85                  90                  95

Glu Thr Asn Ser Gly Phe His Glu Ile Glu Ser Asn Leu Leu Asn Arg
            100                 105                 110

Glu Glu Val Phe Gly Tyr Cys Gln Val Ile Cys Phe Ser Pro Lys His
        115                 120                 125

Asn Val Thr Leu Ala Ser Met Thr Pro Thr Gln Ile Glu Ala Val Val
    130                 135                 140

Asn Thr Trp Arg Lys Ile Tyr Glu Glu Ala Gln Arg Lys Gly Ile Lys
145                 150                 155                 160

Tyr Ser Leu Ile Phe Glu Asn Lys Gly Ser Ala Met Gly Cys Ser Asn
                165                 170                 175

Pro His Ala His Cys Gln Val Trp Asn Thr Ser Val Val Pro Asp Glu
            180                 185                 190

Pro Gln Leu Glu Phe Asn Ser Leu Lys Lys Tyr Lys Asp Glu Lys Lys
        195                 200                 205

Arg Asp Leu Leu Val Asp Tyr Val Gly Leu Glu Leu Gln Lys Glu Glu
    210                 215                 220

Arg Ile Val Tyr Gln Asn Glu Ser Phe Leu Val Val Val Pro Tyr Trp
225                 230                 235                 240

Ala Val Trp Pro Phe Glu Thr Met Ile Val Ser Lys Glu Arg Ile Ser
                245                 250                 255

Ser Ile Lys Lys Leu Ser Asn His Gln Val Ser Asp Leu Ala Asp Ser
            260                 265                 270

Ile Ser Val Leu Thr Arg Lys Tyr Asp Asp Leu Phe Lys Thr Ser Phe

275                 280                 285

Pro Tyr Ser Met Gly Ile His Gln Ser Phe Val Asp Val Glu Asp Cys
    290                 295                 300

Pro Tyr Glu His Leu Arg Leu Leu Phe Tyr Pro Pro Leu Leu Arg Ser
305                 310                 315                 320

Ala Ser Val Lys Lys Phe Gln Val Gly Phe Glu Met Leu Gly Met Pro
                325                 330                 335

Gln Arg Asp Ile Thr Thr Glu Glu Ala Ala Lys Arg Leu Arg Asp Leu
            340                 345                 350

<210>  18
<211>  355
<212>  PRT
<213>  Yarrowia alimentaria

<400>  18

Met Thr Leu Ser Pro Ala Asp Ile Pro His Arg Arg Tyr Asn Pro Leu
1               5                   10                  15

Arg Lys Ser Trp Val Leu Val Ser Pro His Arg Ala Lys Arg Pro Trp
            20                  25                  30

Gln Gly Gln Gln Glu Glu Pro Val Lys Asp Ser Lys Pro Glu Tyr Asp
        35                  40                  45

Asp Lys Cys Tyr Leu Cys Pro Arg Asn Lys Arg Ile Asn Gly Glu Leu
    50                  55                  60

Asn Pro Asp Tyr Gln Ser Thr Phe Val Phe Glu Asn Asp Phe Ser Ala
65                  70                  75                  80

Val Phe Pro Lys Thr Glu Glu Ile Glu Ser Leu Met Lys Glu Glu Lys
                85                  90                  95

Ser Glu Gln Lys Thr Ser Ser Ser Leu Ala Gly Ile Glu Ala Asp Leu
            100                 105                 110

Leu Arg Gln Asp Pro Thr Phe Gly Tyr Cys Gln Val Ile Cys Phe Ser
        115                 120                 125

Pro Lys His Asn Val Thr Leu Ala Thr Met Thr Val Pro Gln Ile Glu
    130                 135                 140

Gln Val Ile Tyr Thr Trp Gln Asp Met Tyr Arg Glu Ala Gln Thr Arg

145                 150                 155                 160

Gln Ile Lys Tyr Ser Leu Ile Phe Glu Asn Lys Gly Ala Thr Met Gly
                165                 170                 175

Cys Ser Asn Pro His Ala His Gly Gln Val Trp Asn Thr Ser Val Val
            180                 185                 190

Pro Glu Glu Pro Gln Met Glu Tyr Asn Ala Leu Arg Glu Tyr Lys Asp
        195                 200                 205

Ser Lys Ser Arg Asp Leu Leu Val Asp Tyr Ala Ala Leu Glu Leu Glu
    210                 215                 220

Lys Lys Glu Arg Val Val Tyr Glu Asn Ser Ser Phe Leu Val Val Val
225                 230                 235                 240

Pro Tyr Trp Ala Val Trp Pro Phe Glu Thr Met Ile Phe Ser Lys Gln
                245                 250                 255

Arg Ile Ala Ser Val Ile Lys Leu Thr Lys Pro Gln Val Ala Asp Leu
            260                 265                 270

Ala Asp Ala Ile Ser Val Leu Thr Arg Lys Tyr Asp Lys Leu Phe Asn
        275                 280                 285

Thr Ser Phe Pro Tyr Ser Met Gly Ile His Gln Ser Phe Val Asp Glu
    290                 295                 300

Pro Asn Cys Glu Tyr Glu His Leu Arg Leu Leu Phe Tyr Pro Pro Leu
305                 310                 315                 320

Leu Arg Ser Ala Ser Val Lys Lys Phe Gln Val Gly Phe Glu Met Leu
                325                 330                 335

Gly Met Pro Gln Arg Asp Ile Thr Ala Glu Gly Ala Ala Gln Arg Leu
            340                 345                 350

Lys Gly Leu
        355

<210>  19
<211>  369
<212>  PRT
<213>  Artificial Sequence

<220>
<223>  GAL10E consensus sequence

<220>
<221>  VARIANT
<222>  (4)..(4)
<223>  Replace = Thr

<220>
<221>  VARIANT
<222>  (5)..(5)
<223>  Replace = Thr or nothing

<220>
<221>  VARIANT
<222>  (6)..(6)
<223>  Replace = nothing

<220>
<221>  VARIANT
<222>  (8)..(8)
<223>  Replace = Tyr

<220>
<221>  VARIANT
<222>  (25)..(25)
<223>  Replace = Ser

<220>
<221>  VARIANT
<222>  (27)..(27)
<223>  Replace = Ile

<220>
<221>  VARIANT
<222>  (34)..(34)
<223>  Replace = Ile

<220>
<221>  VARIANT
<222>  (44)..(44)
<223>  Replace = Ile or Val

<220>
<221>  VARIANT
<222>  (45)..(45)
<223>  Replace = Asp or Gln

<220>
<221>  VARIANT
<222>  (48)..(48)
<223>  Replace = Ser or Gln

<220>
<221>  VARIANT
<222>  (52)..(52)
<223>  Replace = His or Glu

<220>
<221>  VARIANT
<222>  (57)..(57)
<223>  Replace = Thr

<220>
<221>  VARIANT

<222>  (58)..(58)
<223>  Replace = Val

<220>
<221>  VARIANT
<222>  (62)..(62)
<223>  Replace = Lys

<220>
<221>  VARIANT
<222>  (65)..(65)
<223>  Replace = Val

<220>
<221>  VARIANT
<222>  (69)..(69)
<223>  Replace = Lys

<220>
<221>  VARIANT
<222>  (73)..(73)
<223>  Replace = Ala

<220>
<221>  VARIANT
<222>  (77)..(77)
<223>  Replace = Glu

<220>
<221>  VARIANT
<222>  (78)..(78)
<223>  Replace = Phe

<220>
<221>  VARIANT
<222>  (98)..(98)
<223>  Replace = Glu

<220>
<221>  VARIANT
<222>  (99)..(99)
<223>  Replace = Lys

<220>
<221>  VARIANT
<222>  (105)..(105)
<223>  Replace = Phe

<220>
<221>  VARIANT
<222>  (109)..(109)
<223>  Replace = Thr

<220>
<221>  VARIANT
<222>  (111)..(111)
<223>  Replace = Thr

<220>
<221>  VARIANT
<222>  (119)..(119)
<223>  Replace = His

<220>
<221>  VARIANT
<222>  (120)..(120)
<223>  Replace = Ser

<220>
<221>  VARIANT
<222>  (122)..(122)
<223>  Replace = Asp

<220>
<221>  VARIANT
<222>  (154)..(154)
<223>  Replace = Asp

<220>
<221>  VARIANT
<222>  (166)..(166)
<223>  Replace = Val

<220>
<221>  VARIANT
<222>  (169)..(169)
<223>  Replace = Val

<220>
<221>  VARIANT
<222>  (178)..(178)
<223>  Replace = Val or Pro

<220>
<221>  VARIANT
<222>  (179)..(179)
<223>  Replace = Ala or Ser

<220>
<221>  VARIANT
<222>  (184)..(184)
<223>  Replace = Leu

<220>
<221>  VARIANT
<222>  (213)..(213)
<223>  Replace = Phe

<220>
<221>  VARIANT
<222>  (230)..(230)
<223>  Replace = Asn

<220>
<221>  VARIANT
<222>  (235)..(235)
<223>  Replace = Lys

<220>
<221>  VARIANT
<222>  (258)..(258)
<223>  Replace = Asn, Asp or Lys

<220>
<221>  VARIANT
<222>  (262)..(262)

<223>  Replace = Ser

<220>
<221>  VARIANT
<222>  (264)..(264)
<223>  Replace = Glu or Gln

<220>
<221>  VARIANT
<222>  (265)..(265)
<223>  Replace = His

<220>
<221>  VARIANT
<222>  (266)..(266)
<223>  Replace = Gln

<220>
<221>  VARIANT
<222>  (268)..(268)
<223>  Replace = Gln

<220>
<221>  VARIANT
<222>  (269)..(269)
<223>  Replace = Glu

<220>
<221>  VARIANT
<222>  (272)..(272)
<223>  Replace = Glu or Lys

<220>
<221>  VARIANT
<222>  (273)..(273)
<223>  Replace = Asp

<220>
<221>  VARIANT
<222>  (275)..(275)
<223>  Replace = Ala

<220>
<221>  VARIANT
<222>  (291)..(291)
<223>  Replace = Leu

<220>
<221>  VARIANT
<222>  (296)..(296)
<223>  Replace = Met

<220>
<221>  VARIANT
<222>  (299)..(299)
<223>  Replace = Ile

<220>
<221>  VARIANT
<222>  (300)..(300)
<223>  Replace = Gln or Asp

<220>

<221>  VARIANT
<222>  (303)..(303)
<223>  Replace = Cys

<220>
<221>  VARIANT
<222>  (304)..(304)
<223>  Replace = Lys

<220>
<221>  VARIANT
<222>  (309)..(309)
<223>  Replace = Thr

<220>
<221>  VARIANT
<222>  (314)..(314)
<223>  Replace = Val

<220>
<221>  VARIANT
<222>  (315)..(315)
<223>  Replace = Ala

<220>
<221>  VARIANT
<222>  (330)..(330)
<223>  Replace = Asp

<220>
<221>  VARIANT
<222>  (334)..(334)
<223>  Replace = Thr

<220>
<221>  VARIANT
<222>  (337)..(337)
<223>  Replace = Glu, Ser or Gln

<220>
<221>  VARIANT
<222>  (339)..(339)
<223>  Replace = Arg

<220>
<221>  VARIANT
<222>  (341)..(341)
<223>  Replace = Lys

<220>
<221>  VARIANT
<222>  (342)..(342)
<223>  Replace = Gln

<220>
<221>  VARIANT
<222>  (344)..(344)
<223>  Replace = Met

<220>
<221>  VARIANT
<222>  (346)..(346)
<223>  Replace = Lys

<220>
<221>  VARIANT
<222>  (349)..(349)
<223>  Replace = Ala, Thr or Arg

<220>
<221>  VARIANT
<222>  (353)..(353)
<223>  Replace = Lys

<220>
<221>  VARIANT
<222>  (357)..(357)
<223>  Replace = Gln

<220>
<221>  VARIANT
<222>  (364)..(364)
<223>  Replace = Glu

<220>
<221>  VARIANT
<222>  (365)..(365)
<223>  Replace = Glu

<220>
<221>  VARIANT
<222>  (366)..(366)
<223>  Replace = Glu

<220>
<221>  VARIANT
<222>  (367)..(367)
<223>  Replace = Lys

<220>
<221>  VARIANT
<222>  (368)..(368)
<223>  Replace = nothing

<220>
<221>  VARIANT
<222>  (369)..(369)
<223>  Replace = Glu or nothing

<400>  19

Met Thr Gln Ala Ala Ala Glu His Val Leu Val Thr Gly Gly Ala Gly
1               5                   10                  15

Tyr Ile Gly Ser His Thr Val Ile Ala Leu Val Glu Ala Gly Arg Lys
            20                  25                  30

Pro Val Ile Val Asp Asn Leu Asp Asn Ser Cys Glu Glu Ala Val Arg
        35                  40                  45

Arg Ile Glu Gln Ile Val Gly Glu Lys Ile Pro Phe Phe Glu Val Asp
    50                  55                  60

Ile Arg Asp Thr Asn Gly Leu Asp Glu Val Tyr Lys Gln Tyr Pro Thr
65                  70                  75                  80

Ile Thr Ser Val Leu His Phe Ala Gly Leu Lys Ala Val Gly Glu Ser
                85                  90                  95

Gly Gln Ile Pro Leu Glu Tyr Tyr Asn Cys Asn Val Ala Gly Ser Ile
            100                 105                 110

Asn Leu Leu Gln Trp Cys Gln Lys His Asn Ile His Asn Phe Val Phe
        115                 120                 125

Ser Ser Ser Ala Thr Val Tyr Gly Asp Val Thr Arg Phe Glu Gly Met
    130                 135                 140

Ile Pro Ile Pro Glu Glu Cys Pro Thr Gly Pro Thr Asn Pro Tyr Gly
145                 150                 155                 160

Arg Thr Lys Met Met Ile Glu Asp Ile Ile Arg Asp His Val Arg Ala
                165                 170                 175

Asn Ala Pro Trp Asn Ala Ala Ile Leu Arg Tyr Phe Asn Pro Met Gly
            180                 185                 190

Ala His Pro Ser Gly Leu Val Gly Glu Asp Pro Leu Gly Ile Pro Asn
        195                 200                 205

Asn Leu Leu Pro Tyr Leu Ala Gln Val Ala Val Gly Arg Arg Glu Lys
    210                 215                 220

Leu Phe Val Phe Gly Asp Asp Tyr Pro Ser Arg Asp Gly Thr Pro Ile
225                 230                 235                 240

Arg Asp Tyr Ile His Val Cys Asp Leu Ala Ala Gly His Val Ala Ala
                245                 250                 255

Leu Glu Lys Leu Glu Ala Val Asp Lys Glu Arg Lys Gln Leu Pro Gln
            260                 265                 270

Gly Ser Pro Glu Leu Lys His Gly Phe Ser Arg Glu Trp Asn Leu Gly
        275                 280                 285

Thr Gly His Gly Ser Thr Val Leu Glu Val Val Lys Ala Phe Ser Asn
    290                 295                 300

Ala Val Gly Lys Asp Leu Pro Tyr Glu Ile Thr Gly Arg Arg Asp Gly
305                 310                 315                 320

Asp Val Leu Asp Leu Thr Ala Lys Pro Gly Arg Ala Asn Ser Glu Leu
                325                 330                 335

Asn Trp Lys Ala Glu Lys Thr Leu Asp Glu Ser Cys Ser Asp Leu Trp
            340                 345                 350

Arg Trp Thr Ser Glu Asn Pro Tyr Gly Tyr Lys Lys Asp Arg His Asp
        355                 360                 365

Lys

<210>  20
<211>  369
<212>  PRT
<213>  Yarrowia galli

<400>  20

Met Thr Gln Ala Ala Ala Glu His Val Leu Val Thr Gly Gly Ala Gly
1               5                   10                  15

Tyr Ile Gly Ser His Thr Val Ile Ala Leu Val Glu Ala Gly Arg Lys
            20                  25                  30

Pro Val Ile Val Asp Asn Leu Asp Asn Ser Cys Glu Glu Ala Val Arg
        35                  40                  45

Arg Ile Glu Gln Ile Val Gly Glu Lys Ile Pro Phe Phe Glu Val Asp
    50                  55                  60

Ile Arg Asp Thr Asn Gly Leu Asp Glu Val Tyr Lys Gln Tyr Pro Thr
65                  70                  75                  80

Ile Thr Ser Val Leu His Phe Ala Gly Leu Lys Ala Val Gly Glu Ser
                85                  90                  95

Gly Gln Ile Pro Leu Glu Tyr Tyr Phe Cys Asn Val Ala Gly Ser Ile
            100                 105                 110

Asn Leu Leu Gln Trp Cys Gln Lys His Asp Ile His Asn Phe Val Phe
        115                 120                 125

Ser Ser Ser Ala Thr Val Tyr Gly Asp Val Thr Arg Phe Glu Gly Met
    130                 135                 140

Ile Pro Ile Pro Glu Glu Cys Pro Thr Gly Pro Thr Asn Pro Tyr Gly
145                 150                 155                 160

Arg Thr Lys Met Met Ile Glu Asp Ile Ile Arg Asp His Val Arg Ala
                165                 170                 175

Asn Ala Pro Trp Asn Ala Ala Ile Leu Arg Tyr Phe Asn Pro Met Gly
            180                 185                 190

Ala His Pro Ser Gly Leu Val Gly Glu Asp Pro Leu Gly Ile Pro Asn
        195                 200                 205

Asn Leu Leu Pro Tyr Leu Ala Gln Val Ala Val Gly Arg Arg Glu Lys
    210                 215                 220

Leu Phe Val Phe Gly Asp Asp Tyr Pro Ser Arg Asp Gly Thr Pro Ile
225                 230                 235                 240

Arg Asp Tyr Ile His Val Cys Asp Leu Ala Ala Gly His Val Ala Ala
                245                 250                 255

Leu Asn Lys Leu Glu Ala Val Asp Lys Glu Arg Lys Gln Leu Pro Glu
            260                 265                 270

Asp Ser Ala Glu Leu Lys His Gly Phe Ser Arg Glu Trp Asn Leu Gly
        275                 280                 285

Thr Gly His Gly Ser Thr Val Leu Glu Val Val Lys Ala Phe Ser Lys
    290                 295                 300

Ala Val Gly Lys Asp Leu Pro Tyr Glu Ile Ala Gly Arg Arg Asp Gly
305                 310                 315                 320

Asp Val Leu Asp Leu Thr Ala Lys Pro Gly Arg Ala Asn Ser Glu Leu
                325                 330                 335

Glu Trp Lys Ala Glu Lys Thr Leu Asp Glu Ser Cys Ser Asp Leu Trp
            340                 345                 350

Arg Trp Thr Ser Glu Asn Pro Tyr Gly Tyr Lys Lys Asp Arg His Asp
        355                 360                 365

Lys

<210>  21
<211>  368
<212>  PRT
<213>  Yarrowia yakushimensis

<400>  21

Met Thr Gln Ala Thr Glu Tyr Val Leu Val Thr Gly Gly Ala Gly Tyr
1               5                   10                  15

Ile Gly Ser His Thr Val Ile Ala Leu Val Glu Ala Gly Arg Lys Pro
            20                  25                  30

Ile Ile Val Asp Asn Leu Asp Asn Ser Cys Glu Asp Ala Val Ser Arg
        35                  40                  45

Ile Glu His Ile Val Gly Glu Thr Ile Pro Phe Phe Lys Val Asp Ile
    50                  55                  60

Arg Asp Thr Asn Gly Leu Asp Glu Val Tyr Lys Gln Phe Pro Thr Ile
65                  70                  75                  80

Thr Ser Val Leu His Phe Ala Gly Leu Lys Ala Val Gly Glu Ser Gly
                85                  90                  95

Gln Ile Pro Leu Glu Tyr Tyr Asn Cys Asn Val Thr Gly Ser Ile Asn
            100                 105                 110

Leu Leu Gln Trp Cys Gln Lys His Asn Ile His Asn Phe Val Phe Ser
        115                 120                 125

Ser Ser Ala Thr Val Tyr Gly Asp Val Thr Arg Phe Glu Gly Met Ile
    130                 135                 140

Pro Ile Pro Glu Glu Cys Pro Thr Gly Pro Thr Asn Pro Tyr Gly Arg
145                 150                 155                 160

Thr Lys Met Met Val Glu Asp Ile Ile Arg Asp His Val Arg Ala Asn
                165                 170                 175

Val Pro Trp Asn Ala Ala Ile Leu Arg Tyr Phe Asn Pro Met Gly Ala
            180                 185                 190

His Pro Ser Gly Leu Val Gly Glu Asp Pro Leu Gly Ile Pro Asn Asn
        195                 200                 205

Leu Leu Pro Tyr Leu Ala Gln Val Ala Val Gly Arg Arg Glu Lys Leu
    210                 215                 220

Phe Val Phe Gly Asn Asp Tyr Pro Ser Arg Asp Gly Thr Pro Ile Arg
225                 230                 235                 240

Asp Tyr Ile His Val Cys Asp Leu Ala Ala Gly His Val Ala Ala Leu

245                 250                 255

Asp Lys Leu Glu Ala Val Asp Lys Glu Arg Lys Gln Leu Pro Glu Gly
            260                 265                 270

Ser Pro Glu Leu Lys His Gly Phe Ser Arg Glu Trp Asn Leu Gly Thr
        275                 280                 285

Gly His Gly Ser Thr Val Leu Glu Val Val Gln Ala Phe Ser Lys Ala
    290                 295                 300

Val Gly Lys Asp Leu Pro Tyr Glu Ile Thr Gly Arg Arg Asp Gly Asp
305                 310                 315                 320

Val Leu Asp Leu Thr Ala Lys Pro Gly Arg Ala Asn Thr Glu Leu Asn
                325                 330                 335

Trp Arg Ala Lys Gln Thr Leu Asp Lys Ser Cys Ala Asp Leu Trp Arg
            340                 345                 350

Trp Thr Ser Glu Asn Pro Tyr Gly Tyr Lys Lys Asp Arg His Asp Glu
        355                 360                 365

<210>  22
<211>  365
<212>  PRT
<213>  Yarrowia phangngensis

<400>  22

Met Thr Gln Thr Glu His Val Leu Val Thr Gly Gly Ala Gly Tyr Ile
1               5                   10                  15

Gly Ser His Thr Val Ile Ser Leu Val Glu Ala Gly Arg Lys Pro Ile
            20                  25                  30

Ile Val Asp Asn Leu Asp Asn Ser Cys Ile Gln Ala Val Gln Arg Ile
        35                  40                  45

Glu Glu Ile Val Gly Glu Lys Ile Pro Phe Phe Glu Val Asp Val Arg
    50                  55                  60

Asp Thr Lys Gly Leu Asp Glu Val Tyr Lys Gln Tyr Pro Thr Ile Thr
65                  70                  75                  80

Ser Val Leu His Phe Ala Gly Leu Lys Ala Val Gly Glu Ser Gly Glu
                85                  90                  95

Ile Pro Leu Glu Tyr Tyr Phe Cys Asn Val Ala Gly Ser Ile Asn Leu

            100                 105                 110

Leu Gln Trp Cys His Lys His Asn Ile His Asn Phe Val Phe Ser Ser
        115                 120                 125

Ser Ala Thr Val Tyr Gly Asp Val Thr Arg Phe Glu Gly Met Ile Pro
    130                 135                 140

Ile Pro Glu Glu Cys Pro Thr Gly Pro Thr Asn Pro Tyr Gly Arg Thr
145                 150                 155                 160

Lys Met Met Ile Glu Asp Val Ile Arg Asp His Val Arg Ala Asn Pro
                165                 170                 175

Ala Trp Asn Ala Ala Ile Leu Arg Tyr Phe Asn Pro Met Gly Ala His
            180                 185                 190

Pro Ser Gly Leu Val Gly Glu Asp Pro Leu Gly Ile Pro Asn Asn Leu
        195                 200                 205

Leu Pro Tyr Leu Ala Gln Val Ala Val Gly Arg Arg Glu Lys Leu Phe
    210                 215                 220

Val Phe Gly Asn Asp Tyr Pro Ser Arg Asp Gly Thr Pro Ile Arg Asp
225                 230                 235                 240

Tyr Ile His Val Cys Asp Leu Ala Ala Gly His Val Ala Ala Leu Asn
                245                 250                 255

Lys Leu Glu Ala Val Glu Lys Gln Arg Lys Glu Leu Pro Lys Asp Ser
            260                 265                 270

Ala Glu Leu Lys His Gly Phe Ser Arg Glu Trp Asn Leu Gly Thr Gly
        275                 280                 285

His Gly Ser Thr Val Leu Glu Val Val Asp Ala Phe Cys Lys Ala Val
    290                 295                 300

Gly Lys Thr Leu Pro Tyr Glu Ile Thr Gly Arg Arg Asp Gly Asp Val
305                 310                 315                 320

Leu Asp Leu Thr Ala Lys Pro Gly Arg Ala Asn Ser Glu Leu Ser Trp
                325                 330                 335

Lys Ala Glu Lys Thr Leu Asp Glu Ser Cys Thr Asp Leu Trp Arg Trp
            340                 345                 350

Thr Ser Gln Asn Pro Tyr Gly Tyr Lys Lys Glu Glu Lys
        355                 360                 365

<210>  23
<211>  366
<212>  PRT
<213>  Yarrowia alimentaria

<400>  23

Met Thr Gln Thr Glu His Val Leu Val Thr Gly Gly Ala Gly Tyr Ile
1               5                   10                  15

Gly Ser His Thr Val Ile Ala Leu Ile Glu Ala Gly Arg Lys Pro Val
            20                  25                  30

Ile Val Asp Asn Leu Asp Asn Ser Cys Val Glu Ala Val Arg Arg Ile
        35                  40                  45

Glu Gln Ile Val Gly Glu Lys Val Pro Phe Phe Glu Val Asp Ile Arg
    50                  55                  60

Asp Thr Asn Gly Leu Asp Ala Val Tyr Lys Glu Phe Pro Thr Ile Thr
65                  70                  75                  80

Ser Val Leu His Phe Ala Gly Leu Lys Ala Val Gly Glu Ser Gly Gln
                85                  90                  95

Lys Pro Leu Glu Tyr Tyr Phe Cys Asn Val Ala Gly Thr Ile Asn Leu
            100                 105                 110

Leu Gln Trp Cys His Ser His Asn Ile His Asn Phe Val Phe Ser Ser
        115                 120                 125

Ser Ala Thr Val Tyr Gly Asp Val Thr Arg Phe Glu Gly Met Ile Pro
    130                 135                 140

Ile Pro Glu Glu Cys Pro Thr Asp Pro Thr Asn Pro Tyr Gly Arg Thr
145                 150                 155                 160

Lys Met Met Ile Glu Asp Ile Ile Arg Asp His Val Arg Ala Asn Pro
                165                 170                 175

Ser Trp Asn Ala Ala Leu Leu Arg Tyr Phe Asn Pro Met Gly Ala His
            180                 185                 190

Pro Ser Gly Leu Val Gly Glu Asp Pro Leu Gly Ile Pro Asn Asn Leu
        195                 200                 205

Leu Pro Phe Leu Ala Gln Val Ala Val Gly Arg Arg Glu Lys Leu Phe
    210                 215                 220

Val Phe Gly Asn Asp Tyr Pro Ser Lys Asp Gly Thr Pro Ile Arg Asp
225                 230                 235                 240

Tyr Ile His Val Cys Asp Leu Ala Ala Gly His Val Ala Ala Leu Lys
                245                 250                 255

Lys Leu Glu Ser Val Gln His Glu Arg Gln Glu Leu Pro Lys Asp Ser
            260                 265                 270

Pro Glu Leu Lys His Gly Phe Ser Arg Glu Trp Asn Leu Gly Thr Gly
        275                 280                 285

Leu Gly Ser Thr Val Met Glu Val Ile Lys Ala Phe Ser Lys Ala Val
    290                 295                 300

Gly Lys Asp Leu Pro Tyr Glu Val Ala Gly Arg Arg Asp Gly Asp Val
305                 310                 315                 320

Leu Asp Leu Thr Ala Lys Pro Asp Arg Ala Asn Thr Glu Leu Gln Trp
                325                 330                 335

Lys Ala Glu Lys Thr Met Asp Glu Ser Cys Arg Asp Leu Trp Lys Trp
            340                 345                 350

Thr Ser Glu Asn Pro Tyr Gly Tyr Lys Glu Asp Arg His Asp
        355                 360                 365

<210>  24
<211>  26
<212>  PRT
<213>  Artificial Sequence

<220>
<223>  Fragment of GAL10M

<220>
<221>  VARIANT
<222>  (3)..(3)
<223>  Replace = Phe

<220>
<221>  VARIANT
<222>  (17)..(17)
<223>  Replace = Asp or Asn

<220>
<221>  VARIANT
<222>  (19)..(19)
<223>  Replace = Glu, Asp or Gln

<220>
<221>  VARIANT
<222>  (22)..(22)
<223>  Replace = Val or Thr

<400>  24

Asn Pro Tyr Phe Gly Ala Thr Ile Gly Pro Val Ala Asn Arg Leu Tyr
1               5                   10                  15

Gln Pro Asn Gly Ile Leu Leu His Gly Gly
            20                  25

<210>  25
<211>  24
<212>  PRT
<213>  Artificial Sequence

<220>
<223>  Fragment of GAL10M

<220>
<221>  VARIANT
<222>  (3)..(3)
<223>  Replace = Leu

<220>
<221>  VARIANT
<222>  (4)..(4)
<223>  Replace = Asn

<220>
<221>  VARIANT
<222>  (6)..(6)
<223>  Replace = Pro

<220>
<221>  VARIANT
<222>  (12)..(12)
<223>  Replace = Glu

<220>
<221>  VARIANT
<222>  (17)..(17)
<223>  Replace = Thr or Lys

<220>
<221>  VARIANT
<222>  (20)..(20)
<223>  Replace = Asp or Val

<220>
<221>  VARIANT
<222>  (23)..(23)
<223>  Replace = Lys

<220>
<221>  VARIANT
<222>  (24)..(24)

<223>  Replace = Asp

<400>  25

Arg Leu Ser Arg Asp Ala Lys Leu Asp Thr Arg Asp Asp Pro Leu Val
1               5                   10                  15

Ser Val Ala Ile Leu Ser Arg Gly
            20

<210>  26
<211>  33
<212>  PRT
<213>  Artificial Sequence

<220>
<223>  Fragment of GAL10M

<220>
<221>  VARIANT
<222>  (2)..(2)
<223>  Replace = Asn

<220>
<221>  VARIANT
<222>  (4)..(4)
<223>  Replace = Ser

<220>
<221>  VARIANT
<222>  (7)..(7)
<223>  Replace = Ala or Ser

<220>
<221>  VARIANT
<222>  (9)..(9)
<223>  Replace = Ala

<220>
<221>  VARIANT
<222>  (15)..(15)
<223>  Replace = Ala

<220>
<221>  VARIANT
<222>  (16)..(16)
<223>  Replace = Ala

<220>
<221>  VARIANT
<222>  (23)..(23)
<223>  Replace = His or Tyr

<220>
<221>  VARIANT
<222>  (26)..(26)
<223>  Replace = Gln or Glu

<220>
<221>  VARIANT

<222>  (28)..(28)
<223>  Replace = Lys or Arg

<220>
<221>  VARIANT
<222>  (29)..(29)
<223>  Replace = Asn, Asp or Lys

<220>
<221>  VARIANT
<222>  (31)..(31)
<223>  Replace = Thr

<220>
<221>  VARIANT
<222>  (33)..(33)
<223>  Replace = Met

<400>  26

Glu Ser Arg Asp Phe Gly Arg Arg Ser Gly Val Ala Ile Glu Cys Ser
1               5                   10                  15

Arg Pro Thr Asn Ala Trp Asn Val Pro Gly Trp Gln Pro Trp Val Asp
            20                  25                  30

Leu

<210>  27
<211>  329
<212>  PRT
<213>  Yarrowia galli

<400>  27

Met Thr Leu Cys Asn Thr Gly Ala Thr Leu Gln Ser Trp Glu Asn Ser
1               5                   10                  15

Val Leu Gly Phe Ser Asn Ala Asp Asn Tyr Val Ser Ser Glu Asn Pro
            20                  25                  30

Phe Phe Gly Ala Thr Ile Gly Pro Val Ala Asn Arg Leu Tyr Gln Pro
        35                  40                  45

Glu Gly Ile Val Leu His Gly Gly Lys Glu Ser Trp Ala Trp Lys Lys
    50                  55                  60

Trp Thr Arg Thr Glu Glu Glu Gly His Val Thr Lys Phe Glu Leu Arg
65                  70                  75                  80

Asp Thr Glu Ile Thr Ala His Val Thr Tyr Thr Leu Thr Lys Pro Asp
                85                  90                  95

Ser Thr Thr Thr Gln Leu Glu Ile Glu Tyr Ser Ala Lys Ser Met Asp
            100                 105                 110

Gly Arg Ser Thr Pro Ile Ser Met Thr Asn His Ser Tyr Phe Cys Leu
        115                 120                 125

Asp Gln Asp Ser Glu Gly His Val Thr Val Asp Asp His Glu Leu Trp
    130                 135                 140

Leu Cys Asn Asp His Val Ile Glu His Lys Asp Gly Leu Gln Ser Glu
145                 150                 155                 160

Ala Pro Thr Gly Glu Leu Val Cys Cys Glu Ala Leu Glu Arg Ala Glu
                165                 170                 175

Leu Val Lys Ser Arg Asp Gln Ile Glu Ser Arg Glu Ile Lys Gln Ser
            180                 185                 190

Arg Asp Ile Ser Asn Asp Ser Thr Ile Pro Leu Pro Gly Ser Ser Arg
        195                 200                 205

Ala Phe Ser Leu Ala Gly Leu Ser Leu Asp His Thr Phe Ala Val Asn
    210                 215                 220

Asn Gln Arg Leu Ser Arg Asp Pro Lys Leu Asp Thr Arg Asp Asp Pro
225                 230                 235                 240

Leu Val Thr Val Ala Asp Leu Ser Arg Asp Lys Thr Arg Leu Ile Val
                245                 250                 255

Lys Thr Thr Glu Pro Cys Phe Gln Val Tyr Thr Gly Asp Tyr Asn Asn
            260                 265                 270

Val Ser Gln Leu Pro Gly Glu Ser Arg Asp Phe Gly Ala Arg Ser Gly
        275                 280                 285

Val Ala Ile Glu Cys Ser Arg Pro Thr Asn Ala Trp Asn Val Pro Gln
    290                 295                 300

Trp Lys Asn Trp Val Asp Leu Gly Gln Gly Glu Tyr Gly Ala Lys Ile
305                 310                 315                 320

Val Tyr Val Leu Glu Lys Arg Gly Asp
                325

<210>  28
<211>  319
<212>  PRT

<213>  Yarrowia yakushimensis

<400>  28

Met Thr Phe Ser Ala Thr Gly Ala Thr Leu Gln Ser Trp Glu Asn Cys
1               5                   10                  15

Ile Leu Gly Phe Glu Asn Asp Ala Asn Tyr Leu Asn Asn Gln Asn Pro
            20                  25                  30

Tyr Phe Gly Ala Thr Ile Gly Pro Val Ala Asn Arg Leu Tyr Asp Pro
        35                  40                  45

Asp Gly Ile Val Leu His Gly Gly Glu Glu Gly Trp Ser Trp Lys Asn
    50                  55                  60

Trp Lys Lys Ile Glu Glu Thr Glu Lys Val Thr Lys Phe Glu Leu Asn
65                  70                  75                  80

Asp Asp Arg Ile Thr Ala His Val Ile Tyr Thr Leu Thr Asn Pro His
                85                  90                  95

Pro Asn Thr Ser Gln Leu Glu Ile Glu Tyr Ser Ala Leu Ala Lys Glu
            100                 105                 110

Lys Asp Ala Asn Leu Pro Ile Asn Met Thr Asn His Ser Tyr Phe Cys
        115                 120                 125

Leu Pro Pro Gln Ser Gly His Val Thr Val Asp Asp His Asp Leu Trp
    130                 135                 140

Leu Cys Asn Thr Gln Val Leu Glu His Lys Asp Gly Leu Gln Ser Glu
145                 150                 155                 160

Pro Pro Thr Gly Lys Leu Ile Val Cys Asp Pro Leu Lys Glu Val Arg
                165                 170                 175

Ser Arg Asp Glu Asn Asn His Asp Asp Val Gly Ser His Asp Ser Asn
            180                 185                 190

Ser Pro Ser His Ala Val Gln Met Ser Leu Asn Gly Met Ala Leu Asp
        195                 200                 205

His Thr Phe Ala Val Asp Asn Glu Arg Leu Ser Arg Asp Ala Lys Leu
    210                 215                 220

Asp Thr Arg Asp Asp Pro Leu Val Thr Val Ala Val Leu Ser Arg Asp
225                 230                 235                 240

Ser Lys Thr Arg Leu Thr Val Lys Thr Thr Glu Pro Cys Phe Gln Val
                245                 250                 255

Tyr Thr Gly Asp Phe Ile Ser Val Asp Gln Leu Ser Gly Glu Ser Arg
            260                 265                 270

Asp Phe Gly Ser Arg Ala Gly Val Ala Ile Glu Cys Ser Arg Pro Thr
        275                 280                 285

Asn Ala Trp His Val Pro Glu Trp Arg Asp Trp Val Asp Leu Ser Lys
    290                 295                 300

Gly Val Tyr Gly Ser Lys Ile Val Tyr Ile Leu Glu Arg Glu Glu
305                 310                 315

<210>  29
<211>  282
<212>  PRT
<213>  Yarrowia alimentaria

<400>  29

Met Tyr Ser Asp Ile Gly Ala Ala Val Lys Ala Leu Asp Gly Val Val
1               5                   10                  15

Leu Gly Phe Ser Asn Asp Glu Asn Tyr Leu Lys Pro Glu Asn Pro Phe
            20                  25                  30

Phe Gly Ala Thr Ile Gly Pro Val Ala Asn Arg Leu Tyr Asn Pro Gln
        35                  40                  45

Gly Ile Thr Leu His Gly Gly Ala Asn Ser Trp Ala Phe Lys Thr Trp
    50                  55                  60

Ser Arg Ser Glu Asn Gly Asn Val Thr Thr Phe Asp Leu Val Asp Gly
65                  70                  75                  80

Asp Val Arg Ala Gln Val Lys Tyr Thr Leu Val Lys Thr Lys Thr Ala
                85                  90                  95

Thr Thr Leu Glu Ile Glu Phe Trp Ala Asn Ser Ser Gly Lys Ser Ile
            100                 105                 110

Pro Leu Ser Met Ala Asn His Cys Tyr Phe Ser Leu Asn Gly Glu Ser
        115                 120                 125

Thr Ile Asp Asp Ser Asp Leu Trp Leu Cys Ser Asp Lys Val Ile Glu
    130                 135                 140

His Gly Asn Asp Ser Gln Asn Glu Ala Pro Thr Gly Gln Leu Lys Thr
145                 150                 155                 160

Phe Lys Arg Ala Ser Asn Ala Leu Thr Thr Pro Ile Asp His Thr Phe
                165                 170                 175

Val Ile Asp Glu Gln Arg Leu Leu Asn Asp Ala Lys Leu Asp Thr Arg
            180                 185                 190

Glu Asp Pro Leu Val Lys Val Ala Val Leu Ser Lys Gly Asn Val Ser
        195                 200                 205

Val Thr Val Glu Thr Thr Glu Pro Val Phe Gln Val Tyr Thr Gly Asp
    210                 215                 220

Tyr Asn Asn Val Pro Arg Phe Glu Asn Glu Asn Arg Ser Phe Gly Ala
225                 230                 235                 240

Arg Ser Gly Val Ala Ile Glu Ala Ala Arg Pro Thr Asn Ala Trp Tyr
                245                 250                 255

Val Pro Gln Trp Lys Lys Trp Thr Asp Met Ala Gln Gly Asp Tyr Gly
            260                 265                 270

Ser Arg Thr Val Tyr Thr Val Lys Arg Gly
        275                 280

<210>  30
<211>  41
<212>  DNA
<213>  Artificial Sequence

<220>
<223>  Primer

<400>  30
gcgcggatcc atgactaaat ctcattcaga agaagtgatt g                          41

<210>  31
<211>  40
<212>  DNA
<213>  Artificial Sequence

<220>
<223>  Primer

<400>  31
gcgccctagg ttataattca tatagacagc tgcccaatgc                            40

<210>  32
<211>  29

<212>  DNA
<213>  Artificial Sequence

<220>
<223>  Primer

<400>  32
gcgcggatcc atgactgctg aagaatttg                                          29


<210>  33
<211>  35
<212>  DNA
<213>  Artificial Sequence

<220>
<223>  Primer

<400>  33
gcgccctagg ttacagtctt tgtagataat gaatc                                   35


<210>  34
<211>  30
<212>  DNA
<213>  Artificial Sequence

<220>
<223>  Primer

<400>  34
gcgcggatcc atgacagctc agttacaaag                                         30


<210>  35
<211>  32
<212>  DNA
<213>  Artificial Sequence

<220>
<223>  Primer

<400>  35
gcgccctagg tcaggaaaat ctgtagacaa tc                                      32


<210>  36
<211>  30
<212>  DNA
<213>  Artificial Sequence

<220>
<223>  Primer

<400>  36
gtgaggatcc atgtctatca gcaccctgcc                                         30


<210>  37
<211>  35
<212>  DNA
<213>  Artificial Sequence

<220>
<223>  Primer

<400>  37
gcgccctagg ttacaaatca acaatagcac atccc                                35


<210>  38
<211>  29
<212>  DNA
<213>  Artificial Sequence

<220>
<223>  Primer

<400>  38
gagaggatcc atgactcttg tggcatccg                                       29


<210>  39
<211>  33
<212>  DNA
<213>  Artificial Sequence

<220>
<223>  Primer

<400>  39
gcgccctagg ttacaaatca tacaaccgct tgg                                  33


<210>  40
<211>  32
<212>  DNA
<213>  Artificial Sequence

<220>
<223>  Primer

<400>  40
gagaagatct atgacccagg ctgctgctga ac                                   32


<210>  41
<211>  40
<212>  DNA
<213>  Artificial Sequence

<220>
<223>  Primer

<400>  41
gcgcactagt ttacttgtca tgacggtcct ttttgtaacc                           40


<210>  42
<211>  36
<212>  DNA
<213>  Artificial Sequence

<220>
<223>  Primer

<400>  42
gagaggatcc atgacctttt gcaacacggg agcaac                                36

<210>  43
<211>  43
<212>  DNA
<213>  Artificial Sequence

<220>
<223>  Primer

<400>  43
gcgccctagg tcactcccgc tccaaaacat agataatctt agc                        43

<210>  44
<211>  30
<212>  DNA
<213>  Artificial Sequence

<220>
<223>  Primer

<400>  44
gcgcactagt atgtctatca gcaccctgcc                                       30

<210>  45
<211>  37
<212>  DNA
<213>  Artificial Sequence

<220>
<223>  Primer

<400>  45
gcgccccggg ttacaaatca acaatagcac atccctc                               37

<210>  46
<211>  29
<212>  DNA
<213>  Artificial Sequence

<220>
<223>  Primer

<400>  46
gagaactagt atgactcttg tggcatccg                                        29

<210>  47
<211>  34
<212>  DNA
<213>  Artificial Sequence

<220>
<223>  Primer

<400>  47
gcgacccggg ttacaaatca tacaaccgct tggc                                  34

<210>  48
<211>  29
<212>  DNA
<213>  Artificial Sequence

<220>
<223>  Primer

<400>  48
gagaactagt atgacccagg ctgctgctg                                          29


<210>  49
<211>  35
<212>  DNA
<213>  Artificial Sequence

<220>
<223>  Primer

<400>  49
gagacccggg ttacttgtca tgacggtcct ttttg                                   35


<210>  50
<211>  36
<212>  DNA
<213>  Artificial Sequence

<220>
<223>  Primer

<400>  50
gcgcactagt atgacctttt gcaacacggg agcaac                                  36


<210>  51
<211>  42
<212>  DNA
<213>  Artificial Sequence

<220>
<223>  Primer

<400>  51
gcgcgaattc tcactcccgc tccaaaacat agataatctt ag                           42


<210>  52
<211>  20
<212>  DNA
<213>  Artificial Sequence

<220>
<223>  Primer

<400>  52
tcttcaactg ctcctgtcct                                                    20


<210>  53
<211>  21

<212>  DNA
<213>  Artificial Sequence

<220>
<223>  Primer

<400>  53
ctatcgtaat cgacaccatc c                                                 21


<210>  54
<211>  20
<212>  DNA
<213>  Artificial Sequence

<220>
<223>  Primer

<400>  54
ccatcaaaaa gctgaccaag                                                   20


<210>  55
<211>  20
<212>  DNA
<213>  Artificial Sequence

<220>
<223>  Primer

<400>  55
atcgcagaag aggagggtag                                                   20


<210>  56
<211>  19
<212>  DNA
<213>  Artificial Sequence

<220>
<223>  Primer

<400>  56
tggtgacgac taccccctct                                                   19


<210>  57
<211>  20
<212>  DNA
<213>  Artificial Sequence

<220>
<223>  Primer

<400>  57
cggttcctag gttccattct                                                   20


<210>  58
<211>  20
<212>  DNA
<213>  Artificial Sequence

<220>
<223>  Primer

<400>  58
tccatctccc tcaatggaac                                                    20


<210>  59
<211>  20
<212>  DNA
<213>  Artificial Sequence

<220>
<223>  Primer

<400>  59
gccggtatag acctggaaac                                                    20

## Description

The present invention relates to mutant *Yarrowia* strains capable of growing on galactose as carbon source and means for obtaining such mutant strains.

Compounds such as citric acid, lipids, or sugar alcohols are of industrial interest. As a consequence, using microorganisms such as *Yarrowia lipolytica* to produce them has spurred research around the world (Beopoulos *et al.,* 2012; Tai and Stephanopoulos, 2013; Liu *et al.,* 2014; Mirończuk *et al.,* 2014). From an economic and social point of view, it is very important to identify raw materials that could serve as cheap and renewable substrates and that are not already in demand for food production. Therefore, at present, research efforts are focused on using plant biomass, which contains a diversity of sugars, for the production of these valuable compounds (Blazeck *et al.,* 2014; Wang *et al.,* 2014). One such sugar is D-galactose, a monosaccharide that is a C4 epimer of glucose. The polysaccharides found in plant cell walls (e.g., galactomannans), gums, hemicelluloses, and pectins are rich sources of galactose (Schädel *et al.,* 2010; Christensen *et al.,* 2011). Galactose also occurs naturally in milk: lactose is made up of galactose and glucose. Both milk and plant biomass are readily exploited by many microorganisms, including bacteria, yeasts, and fungi. These diverse species utilize only a few pathways to break down galactose. For example, the bacterium *Azotobacter vinelandii* uses the non-phosphorylative DeLey-Doudoroff pathway to metabolize galactose. Galactose is oxidized, forming galactonate, which is ultimately broken down into pyruvate and glyceraldehyde 3-phosphate (Wong and Yao, 1994). Another pathway, the oxido-reductive pathway, exists in filamentous fungi such as *Aspergillus niger* or *Hypocrea jecorina.* It involves a series of redox reactions that convert galactose to D-fructose (Gruben *et al.,* 2012; Koivistoinen *et al.,* 2012; Mojzita *et al.,* 2012). However, the best-known and best-studied pathway is the Leloir pathway, which occurs in many organisms, including yeasts such as *Saccharomyces cerevisiae* and *Kluyveromyces lactis* (Meyer *et al.,* 1991; Frey, 1996; Holden *et al.,* 2003; Sellick *et al.,* 2008). Via this phosphorylative pathway, D-galactose is converted to glucose-6-phosphate. In *S. cerevisiae,* ß-D-galactose is first converted to its α-anomer by galactose mutarotase (*scGAL10,* YBR019C); only this anomeric form can be utilized by cells. Subsequently, α-D-galactose is phosphorylated by galactokinase (*scGAL1,* YBR020W), releasing galactose-1-phosphate. Then, galactose-1-phosphate uridyltrans-ferase (*scGAL7,* YBR018C) converts this intermediate compound into UDP-galactose, simultaneously releasing glucose-1-phosphate. In the last step of the pathway, UDP-galactose is epimerized into UDP-glucose by UDP-galactose 4-epimerase (*scGAL10,* YBR019C). It is worth noting that, in *S. cerevisiae* and other ascomycetes, the epimerase and mutarotase domains are fused together but act independently (Slot and Rokas, 2010). Additionally, the mutarotase domain is not essential to galactose metabolism because the sugar anomers interconvert spontaneously in water (Bouffard *et al.,* 1994). Phosphoglucomutase converts the glucose-1-phosphate released by the pathway to glucose-6-phosphate, an intermediate compound in glycolysis. However, because phosphorrglucomutase is also involved in glycogenolysis, it is not formally considered to be part of the Leloir pathway. In addition, the form of Leloir pathway regulation that exists *in S. cerevisiae* may be restricted to yeasts and not conserved in other ascomycetes (Hartl *et al.,* 2012).

*Y. lipolytica* is a dimorphic ascomycete yeast that belongs to the subphylum Saccharomycotina (van der Walt and von Arx, 1980). *Y. lipolytica* is able to use a few monosaccharides as carbon sources, namely glucose, fructose, and mannose (Coelho *et al.,* 2010; Michely *et al.,* 2013). All the genes that encode putative structural Leloir pathway proteins are present in its genome, but the wild type strains of this species appear to be unable to use galactose as its sole carbon source (Slot and Rokas, 2010). The genes are not clustered as they are in *S. cerevisiae* and other ascomycetes and, furthermore, are located on different chromosomes (Slot and Rokas, 2010).

The inventors have found that *Y. lipolytica* carry and express all the Leloir pathway genes, which encode fully functional proteins that are involved in galactose utilization. When *Y. lipolytica* was grown on mixed media, containing both galactose and varying concentrations of glucose, it was able to metabolize galactose, including when glucose concentrations were higher than 4 g.L⁻¹. However, in such mixed media, glucose was still the preferred carbon source.

Surprisingly, a *Y. lipolytica* strain in which the four *ylGAL* genes were overexpressed, namely:
- Yl*GAL1* (YALI0C13090g) coding for a galactokinase,
- Yl*GAL7* (YALI0F23947g) coding for a galactose-1-phosphate uridyl transferase,
- Yl*GAL10e* (YALI0E26829g) coding for a UDP-glucose-4 epimerase, and
- Y1*GAL10m* (YALI0C09570g) coding for a galactose mutarotase,
was able to efficiently use galactose as its sole carbon source. This quadruple mutant was used to successfully produce citric acid and lipids from galactose; the yield of these compounds was comparable or greater than that obtained for the parental strain (W29) grown on glucose media.

These results are surprising because efforts at engineering *Y. lipolytica* able to degrading sugars which are not reported as being naturally consumed by *Y. lipolytica,* such as xylose, by overexpressing endogenous putative genes involved in the sugar *(e.g.,* xylose) degrading pathway, were not successful (see International Application WO 2013/192520).

The effective activation of the galactose utilization pathway in *Y. lipolylica* can therefore serve as an excellent starting point for further improving citric acid and lipid production from renewable substrates.

Accordingly, the present invention provides a method for obtaining a *Yarrowia* strain, preferably a *Y. lipolytica* strain, capable of growing on D-galactose as sole carbon source, wherein said method comprises overexpressing in said strain
- a galactokinase (E.C 2.7.1.6) having at least 75% identity, or by order of increasing preference at least 78%, 80%, 85%, 90%, 92%, 95%, 96%, 97%, 98% or 99% identity, with the polypeptide of sequence SEQ ID NO: 2 (YALI_GAL1),
- a galactose-1-phosphate uridyl transferase (E.C 2.7.7.12) having at least 65% identity, or by order of increasing preference at least 70%, 75%, 80%, 85%, 90%, 92%, 95%, 96%, 97%, 98% or 99% identity, with the polypeptide of sequence SEQ ID NO: 4 (YALI_GAL7),
- an UDP-glucose-4 epimerase (E.C 5.1.3.2) having at least 85% identity, or by order of increasing preference at least 88%, 90%, 92%, 95%, 96%, 97%, 98% or 99% identity, with the polypeptide of sequence SEQ ID NO: 6 (YALI_GAL10E) and
- a galactose mutarotase (E.C 5.1.3.3) having at least 45% identity, or by order of increasing preference at least 48%, 50%, 55%, 60%, 65%, 70%, 75%, 80%, 85%, 90%, 92%, 95%, 96%, 97%, 98% or 99% identity, with the polypeptide of sequence SEQ ID NO: 8 (YALI_GAL10M).

Unless otherwise specified, the percent of identity between two protein sequences which are mentioned herein is calculated from the best alignment of the two sequences using the BLAST program (Tatusova and Madden, 1999) with the default parameters (open gap penalty = 2; extension gap penalty = 5; matrix = BLOSUM 62).

The term overexpressing an enzyme in a *Yarrowia* strain, herein refers to artificially increasing the quantity of said enzyme produced in a *Yarrowia* strain compared to a reference (control) *Yarrowia* strain wherein said enzyme is not overexpressed. This term also encompasses expression of an enzyme in a *Yarrowia* strain which does not naturally contain a gene encoding said enzyme.

An advantageous method for overexpressing an enzyme in a *Yarrowia* strain comprises introducing into the genome of said *Yarrowia* strain a DNA construct comprising a nucleotide sequence encoding said enzyme, placed under the control of a promoter.

Nucleotide sequences encoding YALI_GAL1, YALI_GAL7, YALI_GAL10E and YALI_GAL10M are provided in SEQ ID NO: 1, SEQ ID NO: 3, SEQ ID NO: 5 and SEQ ID NO: 7 respectively.

Methods for determining whether an enzyme has a galactokinase (E.C2.7.1.6) activity are known in the art. By way of example, galactokinase activity can be measured by quantifying formation of galactose1-phosphate from C14-galactose, as described in Schell and Wilson (1977).

Methods for determining whether an enzyme has a galactose-1-phosphate uridyl transferase (E.C 2.7.7.12) activity are known in the art. By way of example, galactose-1-phosphate uridyl transferase activity can be measured by quantifying the release of glucose-1-phosphate from UDP-Glucose, as described in Segawa and Fukasawa (1979).

Methods for determining whether an enzyme has an UDP-glucose-4 epimerase (E.C 5.1.3.2) activity are known in the art. By way of example, UDP-glucose-4 epimerase activity can be measured by quantifying the release of UDP-Glucuronic acid from UDP-glucose which was previously formed from UDP-galactose, as described in Majumdar *et al.* (2004).

Methods for determining whether an enzyme has a galactose mutarotase (E.C5.1.3.3) activity are known in the art. By way of example, galactose mutarotase activity can be measured polarimetrically by the formation of β-D-glucose or β-D-galactose from α-D-glucose or α-D-galactose, as described in Majumdar *et al.* (2004).

Advantageously, at least one, or by order of increasing preference at least 2, 3 or 4 of the enzymes as defined above *(i.e.,* galactokinase, galactose-1-phosphate uridyl transferase, UDP-glucose-4 epimerase and galactose mutarotase), preferably the 4 enzymes as defined above, are from a *Yarrowia* strain, preferably selected from the group consisting of *Y. lipolytica, Y. galli, Y*. *yakushimensis, Y*. *alimentaria* and *Y*. *phangngensis,* provided that said strain naturally comprises the enzyme as defined above, more preferably from a *Y. lipolytica* strain.

At least one, or by order of increasing preference at least 2, 3 or 4 of the enzymes as defined are endogenous enzymes, *i.e.,* are from the *Yarrowia* strain in which the overexpression is performed.

In a preferred embodiment, the galactokinase having at least 75% identity with the polypeptide of sequence SEQ ID NO: 2 (YALI_GAL1) has the consensus amino acid sequence SEQ ID NO: 9. This sequence SEQ ID NO: 9 corresponds to the consensus amino acid sequence obtained by aligning the galactokinase from the strains *Y. lipolytica* CLIB122 (YALI_GAL1 of SEQ ID NO: 2), *Y. galli* CBS 9722 (YAGA_GAL1 of SEQ ID NO: 10), *Y*. *yakushimensis* CBS 10253 (YAYA_GAL1 of SEQ ID NO: 11), *Y alimentaria* CBS 10151 (YAAL_GAL1 of SEQ ID NO: 12) and *Y. phangngensis* CBS 10407 (YAPH_GAL1 of SEQ ID NO: 13).

Advantageously, the galactokinase having at least 75% identity with the polypeptide of sequence SEQ ID NO: 2 is selected from the group consisting of SEQ ID NO: 2, 10, 11, 12 and 13, preferably SEQ ID NO: 2.

The galactokinase enzymes of SEQ ID NO: 2 (YALI_GAL1), SEQ ID NO: 10 (YAGA_GAL1), SEQ ID NO: 11 (YAYA_GAL1), SEQ ID NO: 12 (YAAL_GAL1) and SEQ ID NO: 13 (YAPH_GAL1) have respectively 100%, 94.33%, 92.89%, 78.15% and 78.97% identity with the polypeptide of sequence SEQ ID NO: 2 (YALI_GAL1).

In another preferred embodiment, the galactose-1-phosphate uridyl transferase having at least 65% identity with the polypeptide of sequence SEQ ID NO: 4 has the consensus amino acid sequence SEQ ID NO: 14. This sequence SEQ ID NO: 14 corresponds to the consensus amino acid sequence obtained by aligning the galactose-1-phosphate uridyl transferase from the strains *Y. lipolytica* CLIB122 (YALI_GAL7 of SEQ ID NO: 4), *Y. galli* CBS 9722 (YAGA_GAL7 of SEQ ID NO: 15), *Y. yakushimensis* CBS 10253 (YAYA_GAL7 of SEQ ID NO: 16), *Y. alimentaria* CBS 10151 (YAAL_GAL7 of SEQ ID NO: 17) and *Y. phangngensis* CBS 10407 (YAPH_GAL7 of SEQ ID NO: 18).

Advantageously, the galactose-1-phosphate uridyl transferase having at least 65% identity with the polypeptide of sequence SEQ ID NO: 4 is selected from the group consisting of SEQ ID NO: 4, 15, 16, 17 and 18, preferably SEQ ID NO: 4.

The galactose-1-phosphate uridyl transferase enzymes of SEQ ID NO: 4 (YALI_GAL7), SEQ ID NO: 15 (YAGA_GAL7), SEQ ID NO: 16 (YAYA_GAL7), SEQ ID NO: 17 (YAAL_GAL7) and SEQ ID NO: 18 (YAPH_GAL7) have respectively 100%, 91.17%, 87.36%, 70.65% and 70.83% identity with the polypeptide of sequence SEQ ID NO: 4 (YALI_GAL7).

In another preferred embodiment, the UDP-glucose-4 epimerase having at least 85% identity with the polypeptide of sequence SEQ ID NO: 6 (YALI_GAL10E) has the consensus amino acid sequence SEQ ID NO: 19. This sequence SEQ ID NO: 19 corresponds to the consensus amino acid sequence obtained by aligning the UDP-glucose-4 epimerase from the strains *Y. lipolytica* CLIB122 (YALI_GAL10E of SEQ ID NO: 6), *Y. galli* CBS 9722 (YAGA_GAL10E of SEQ ID NO: 20), *Y. yakushimensis* CBS 10253 (YAYA_GAL10E of SEQ ID NO: 21), *Y. alimentaria* CBS 10151 (YAAL_GAL10E of SEQ ID NO: 22) and *Y. phangngensis* CBS 10407 (YAPH_GAL10E of SEQ ID NO: 23).

Advantageously, the UDP-glucose-4 epimerase having at least 85% identity with the polypeptide of sequence SEQ ID NO: 6 is selected from the group consisting of SEQ ID NO: 6, 20, 21, 22 and 23, preferably SEQ ID NO: 6.

The UDP-glucose-4 epimerase enzymes of SEQ ID NO: 6 (YALI_GAL10E), SEQ ID NO: 21 (YAGA_GAL10E), SEQ ID NO: 22 (YAYA_GAE10E), SEQ ID NO: 23 (YAAL_GAL10E) and SEQ ID NO: 24 (YAPH_GAL10E) have respectively 100%, 97.56%, 93.22%, 90.96% and 88.59% identity with the polypeptide of sequence SEQ ID NO: 6 (YALI_GAL10E).

In another preferred embodiment, the amino acid sequence of the galactose mutarotase having at least 45% identity with the polypeptide of sequence SEQ ID NO: 8 (YALI_GAL10M) comprises, from the N-terminus to the C-terminus, the polypeptide fragments of SEQ ID NO: 24, 25 and 26. These fragments of SEQ ID NO: 24, 26 and 26 have been obtained by aligning the galactose mutarotase from the strains *Y. lipolytica* CLIB122 (YALI_GAL10M of SEQ ID NO: 8), *Y*. *galli* CBS 9722 (YAGA_GAL10M of SEQ ID NO: 27), *Y. yakushimensis* CBS 10253 (YAYA_GAL10M of SEQ ID NO: 28) and *Y. alimentaria* CBS 10151 (YAAL_GAL10M of SEQ ID NO: 29).

Advantageously, the galactose mutarotase having at least 45% identity with the polypeptide of sequence SEQ ID NO: 8 (YALI_GAL10M) is selected from the group consisting of SEQ ID NO: 8, 27, 28 and 29, preferably SEQ ID NO: 8.

The galactose mutarotase enzymes of SEQ ID NO: 8 (YALI_GAL10M), SEQ ID NO: 27 (YAGA_GAL10M), SEQ ID NO: 28 (YAYA_GAL10M) and SEQ ID NO: 29 (YAAL_GAL10M) have respectively 100%, 73.43%, 67.38% and 48.76% identity with the polypeptide of sequence SEQ ID NO: 8 (YALI_GAL10M).

Advantageously, said *Yarrowia* strain is auxotrophic for leucine (Leu) and optionally for the decarboxylase orotidine-5'-phosphate (Ura-).

Said *Yarrowia* strain can also be a mutant *Yarrowia* strain wherein the expression or activity of the endogenous isoforms of acyl-coenzymeA oxidases (AOX, EC 1.3.3.6) involved, at least partially, in the β-oxidation of fatty acids, is inhibited. In *Yarrowia,* 6 genes (*POX1, POX2, POX3, POX4, POX5* and *POX6*) encode these isoforms. Said inhibition of the expression or activity can be total or partial. Total or partial inhibition of the expression or activity of these enzymes leads to accumulation by yeast of dodecanedioic acid without use of accumulated fat. More particularly, the coding sequence of the genes *POX1-6* and the peptide sequence of AOX1-6 from *Y. lipolytica* CLIB122 are available in either at Génolevures database (http://genolevures.org/) or at GRYC database (http://gryc.inra.fr/) or in GenBank database under the following accession numbers or names: *POX1* / AOX1 = YALI0E32835g / YALI0E32835p, *POX2* / AOX2 = YAL10F10857g / YALI0F10857p; *POX3* / AOX3 = YALI0D24750g / YALI0D24750p; *POX4* / AOX4 = YALI0E27654g / YALI0E27654p; *POX5* / AOX5 = YALI0C23859g / YALI0C23859p; *POX6* / AOX6 = YALI0E06567g / YALI0E06567p. The peptide sequences of the acyl-CoA oxidases of *Y. lipolytica* have 45% identity or 50% similarity with those from other yeasts. The degree of identity between the acyl-CoA oxidases varies from 55% to 70% (or from 65 to 76% similarity) (see International Application WO 2006/064131). A method of inhibiting the expression of the 6 endogenous AOX in a *Y. lipolytica* strain is described in Beopoulos *et al.,* 2008 and International Applications WO 2006/064131, WO 2010/004141 and WO 2012/001144.

The *Yarrowia* strain can further comprise other mutations such as those described in International Applications WO 2006/064131, WO 2010/004141, WO 2012/001144, WO 2014/178014 and WO 2014/136028 which are useful for obtaining a fatty acids or citric acid producing yeast strain.

In particular, the *Yarrowia* strain can be genetically modified to improve lipid accumulation. Said *Yarrowia* strain having improved properties for lipid accumulation can be a mutant *Yarrowia* strain, preferably a *Y. lipolytica* mutant strain, wherein at least one protein, preferably at least one endogenous protein, selected from the group consisting of an acyl-CoA:diacylglycerol acyltransferase 2 (encoded by *DGA1*)*,* an acyl-CoA:diacylglycerol acyltransferase 1 (encoded by *DGA2*)*,* a glycerol-3-phosphate dehydrogenase NAD+ (encoded by *GPD1*)*,* an acetyl-CoA carboxylase (encoded by *ACC1*) and a hexokinase (encoded by *HXK1*; EC number: 2.7.1.1; YALI0B22308g in *Y. lipolytica*) is overexpressed, and/or the expression or activity of at least one endogenous protein selected from the group consisting of the glycerol 3-phosphate dehydrogenase (encoded by *GUT2),* the triglyceride lipase (encoded by *TGL4)* and the peroxin 10 (encoded by *PEX10*) is inhibited.

Advantageously, the protein hexokinase (encoded by *HXK1*; EC number: 2.7.1.1; YALI0B22308g in *Y. lipolytica)* is overexpressed in said mutant yeast strain, preferably in said mutant *Y. lipolytica* strain.

Advantageously, the 5 proteins, acyl-CoA:diacylglycerol acyltransferase 2, acyl-CoA:diacylglycerol acyltransferase 1, glycerol-3-phosphate dehydrogenase NAD+, acetyl-CoA carboxylase and hexokinase, are overexpressed, and the expression or activity of the 3 endogenous proteins, glycerol 3-phosphate dehydrogenase, triglyceride lipase and peroxin, are inhibited in said mutant yeast strain.

A method of overexpressing the endogenous genes *DGA1, DGA2, GPD1* and *ACC1* and inhibiting the expression or activity of the endogenous genes *GUT2, TGL4* and *PEX10* in a *Y. lipolytica* strain is described in International Application WO 2014/136028.

A method of overexpressing the endogenous genes *DGA2, GPD1* and *HXK1,* and inhibiting the expression or activity of the endogenous gene *TGL4* in a *Y. lipolytica* strain is described in Lazar *et al.,* 2014.

Overexpression of an enzyme as defined above - which can be an endogenous, ortholog or heterologous enzyme - in a *Yarrowia* strain according to the present invention can be obtained in various ways by methods known *per se.*

Overexpression of an enzyme as defined in the present invention may be performed by placing one or more (preferably two or three) copies of the coding sequence (CDS) of the sequence encoding said enzyme under the control of appropriate regulatory sequences. Said regulatory sequences include promoter sequences, located upstream (at 5' position) of the ORF of the sequence encoding said enzyme, and terminator sequences, located downstream (at 3' position) of the ORF of the sequence encoding said enzyme.

Promoter sequences that can be used in yeast are well known to those skilled in the art and may correspond in particular to inducible or constitutive promoters. Examples of promoters which can be used according to the present invention, include the promoter of a *Y. lipolytica* gene which is strongly repressed by glucose and is inducible by the fatty acids or triglycerides such as the promoter of the *POX2* gene encoding the acyl-CoA oxidase 2 (AOX2) of *Y. lipolytica* and the promoter of the *LIP2* gene described in International Application WO 01/83773. One can also use the promoter of the *FBA1* gene encoding the fructose-bisphosphate aldolase (see Application US 2005/0130280), the promoter of the *GPM* gene encoding the phosphoglycerate mutase (see International Application WO 2006/0019297), the promoter of the *YAT1* gene encoding the transporter ammonium (see Application US 2006/0094102), the promoter of the *GPAT* gene encoding the O-acyltransferase glycerol-3-phosphate (see Application US 2006/0057690), the promoter of the *TEF* gene (Müller et al., 1998; Application US 2001/6265185), the hybrid promoter hp4d (described in International Application WO 96/41889), the hybrid promoter XPR2 described in Mazdak *et al.* (2000) or the hybrid promoters UAS1-TEF or UAStef-TEF described in Blazeck *et al.* (2011, 2013, 2014).

Advantageously, the promoter is the promoter of the *TEF* gene.

Terminator sequences that can be used in yeast are also well known to those skilled in the art. Example of terminator sequences which can be used according to the present invention include the terminator sequence of the *PGK1* gene and the terminator sequence of the *LIP2* gene described in International Application WO 01/83773.

The nucleotide sequence of the coding sequences of the heterologous genes can be optimized for expression in yeast by methods well known in the art (see for review Hedfalk, 2012).

Overexpression of an endogenous enzyme as defined above can be obtained by replacing the sequences controlling the expression of said endogenous enzyme by regulatory sequences allowing a stronger expression, such as those described above. The skilled person can replace the copy of the gene encoding an endogenous enzyme in the genome, as well as its own regulatory sequences, by genetically transforming the yeast strain with a linear polynucleotide comprising the ORF of the sequence coding for said endogenous enzyme under the control of regulatory sequences such as those described above. Advantageously, said polynucleotide is flanked by sequences which are homologous to sequences located on each side of said chromosomal gene encoding said endogenous enzyme. Selection markers can be inserted between the sequences ensuring recombination to allow, after transformation, to isolate the cells in which integration of the fragment occurred by identifying the corresponding markers. Advantageously also, the promoter and terminator sequences belong to a gene different from the gene encoding the endogenous enzyme to be overexpressed in order to minimize the risk of unwanted recombination into the genome of the yeast strain.

Overexpression of an endogenous enzyme as defined above can also be obtained by introducing into the *Yarrowia* strain extra copies of the gene encoding said endogenous enzyme under the control of regulatory sequences such as those described above. Said additional copies encoding said endogenous enzyme may be carried by an episomal vector, that is to say capable of replicating in *Yarrowia.* Preferably, these additional copies are carried by an integrative vector, that is to say, integrating into a given location in the *Yarrowia* genome (Madzak *et al.,* 2004). In this case, the polynucleotide comprising the gene encoding said endogenous enzyme under the control of regulatory regions is integrated by targeted integration. Said additional copies can also be carried by PCR fragments whose ends are homologous to a given locus of *Yarrowia,* allowing integrating said copies into the *Yarrowia* genome by homologous recombination. Said additional copies can also be carried by auto-cloning vectors or PCR fragments, wherein the ends have a zeta region absent from the genome of the yeast, allowing the integration of said copies into *Yarrowia* genome by random insertion as described in Application US 2012/0034652.

Targeted integration of a gene into the genome of a *Yarrowia* cell is a molecular biology technique well known to those skilled in the art: a DNA fragment is cloned into an integrating vector, introduced into the cell to be transformed, wherein said DNA fragment integrates by homologous recombination in a targeted region of the recipient genome (Orr-Weaver *et al.,* 1981).

Methods for transforming yeast are also well known to those skilled in the art and are described, *inter alia,* by Ito *et al.* (1983), Klebe *et al.,* (1983) and Gysler *et al.,* (1990).

Any gene transfer method known in the art can be used to introduce a gene encoding an enzyme. Preferably, one can use the method with lithium acetate and polyethylene glycol described by Gaillardin *et al.,* (1987) and Le Dall *et al.,* (1994).

The present invention also provides means for carrying out said overexpression.

This includes, in particular, recombinant DNA constructs for expressing the four enzymes as defined above in a *Yarrowia* cell. These DNA constructs can be obtained and introduced in said *Yarrowia* strain by the well-known techniques of recombinant DNA and genetic engineering.

Recombinant DNA constructs of the invention include in particular expression cassettes, comprising a polynucleotide encoding 1, 2, 3 or 4 of the enzymes as defined above, each polynucleotide encoding an enzyme being under the control of a promoter functional in a *Yarrowia* cell as defined above.

The expression cassettes generally also include a transcriptional terminator, such as those describes above. They may also include other regulatory sequences, such as transcription enhancer sequences.

Recombinant DNA constructs of the invention also include recombinant vectors containing expression cassettes comprising a polynucleotide encoding 1, 2, 3 or 4 of the enzymes as defined above, each polynucleotide encoding an enzyme being under transcriptional control of a suitable promoter.

Recombinant vectors of the invention may also include other sequences of interest, such as, for instance, one or more marker genes, which allow for selection of transformed *Yarrowia* cells.

The invention also comprises host cells containing a recombinant DNA construct of the invention. These host cells can be prokaryotic cells (such as bacteria cells) or eukaryotic cells, preferably yeast cells.

The invention also provides a method for obtaining a mutant *Yarrowia* strain, preferably a mutant *Y. lipolytica* strain, capable of growing on D-galactose as carbon source as defined above, comprising transforming a *Yarrowia* cell with 1, 2, 3 or 4 recombinant DNA constructs as defined above for expressing the 4 enzymes as defined above.

As used herein the term "1, 2, 3 or 4 recombinant DNA constructs for expressing the 4 enzymes" refers to:
- a single recombinant DNA construct for expressing the 4 enzymes as defined above (the coding sequences of the 4 enzymes are under the control of a suitable promoter respectively),
- two recombinant DNA constructs for expressing the 4 enzymes as defined above, including
   - a recombinant DNA construct for expressing 1 enzyme and a recombinant DNA construct for expressing the 3 other enzymes (the coding sequences of the 3 enzymes are under the control of a suitable promoter respectively), or
   - a recombinant DNA construct for expressing 2 enzymes and a recombinant DNA construct for expressing the 2 other enzymes (the coding sequences of the 2 enzymes are under the control of a suitable promoter respectively),
- three recombinant DNA constructs for expressing the 4 enzymes as defined above, including a recombinant DNA construct for expressing 1 enzyme, a recombinant DNA construct for expressing another enzyme and a recombinant DNA construct for expressing the 2 other enzymes (the coding sequences of the 2 enzymes are under the control of a suitable promoter respectively), or
- four recombinant DNA constructs for expressing the 4 enzymes respectively.

The invention also comprises a *Yarrowia* strain, preferably a *Y. lipolytica* strain, genetically transformed with 1, 2, 3 or 4 recombinant DNA constructs for expressing the 4 enzymes as defined above, and overexpressing said 4 enzymes as defined above. In said mutant (transgenic) *Yarrowia* strain 1, 2, 3 or 4 recombinant DNA constructs for expressing the 4 enzymes as defined above is/are comprised in a transgene stably integrated in the *Yarrowia* genome, so that it is passed onto successive yeast generations. Thus the mutant (transgenic) *Yarrowia* strain of the invention includes not only the *Yarrowia* cell resulting from the initial transgenesis, but also their descendants, as far as they contain 1, 2, 3 or 4 recombinant DNA constructs for expressing the 4 enzymes as defined above. The overexpression of the 4 enzymes as defined above in said *Yarrowia* strains provides them an ability to grow on D-galactose as carbon source, when compared with a *Yarrowia* strain devoid of said transgene(s).

The present invention also comprises a mutant *Yarrowia* strain as defined above, preferably a mutant *Y. lipolytica* strain, wherein a galactokinase having at least 75% identity with the polypeptide of sequence SEQ ID NO: 2 (YALI_GAL1), a galactose-1-phosphate uridyl transferase having at least 65% identity with the polypeptide of sequence SEQ ID NO: 4 (YALI_GAL7), an UDP-glucose-4 epimerase having at least 85% identity with the polypeptide of sequence SEQ ID NO: 6 (YALI_GAL10E) and a galactose mutarotase having at least 45% identity with the polypeptide of sequence SEQ ID NO: 8 (YALI_GAL10M) as defined above. This mutant *Yarrowia* strain is obtainable by a method of the invention and contains 1, 2, 3 or 4 recombinant DNA constructs for expressing the 4 enzymes of the invention.

The present invention further comprises a mutant *Yarrowia* strain as defined above, preferably a mutant *Y. lipolytica* strain, comprising, stably integrated in its genome, 1, 2, 3 or 4 recombinant DNA constructs for expressing the 4 enzymes as defined above.

The present invention also provides the use of a mutant *Yarrowia* strain, preferably a mutant *Y. lipolytica* strain, as defined above for producing lipids or citric acid from a medium comprising D-galactose.

As used herein, the term producing lipids or citric acid refers to the accumulation and optionally secretion of lipids or citric acid.

The present invention also provides a method of producing lipids or citric acid, comprising a step of growing a mutant *Yarrowia* strain, preferably a mutant *Y*. *lipolytica* strain, of the invention on D-galactose.

Methods for extracting and purifying lipids or citric acid produced by cultured yeast strains are well known to those skilled in the art (Papanikolaou *et al.,* 2001, 2002 and 2008; André *et al.,* 2009). For example, the total lipids can be extracted according to the method described by Papanikolaou *et al.,* 2001, and fractionated according to the methods described by Guo *et al.,* 2000 and Fakas *et al.,* 2006.

The present invention also provides an isolated enzyme selected from the group consisting of
- a galactokinase (E.C 2.7.1.6.) of SEQ ID NO: 2, 10, 11, 12 or 13,
- a galactose-1-phosphate uridyl transferase (E.C 2.7.7.12) of SEQ ID NO: 4, 15, 16 or 17,
- an UDP-glucose-4 epimerase (E.C 5.1.3.2) of SEQ ID NO: 6, 20, 21, 22 or 23, and
- a galactose mutarotase (E.C 5.1.3.3) of SEQ ID NO: 8, 27, 28 or 29.

The present invention will be understood more clearly from the further description which follows, which refers to non-limitative examples illustrating the overexpression of Gal11, Gal7, Gal10E and Gal10M in *Y. lipoytica,* as well as to the appended figures.

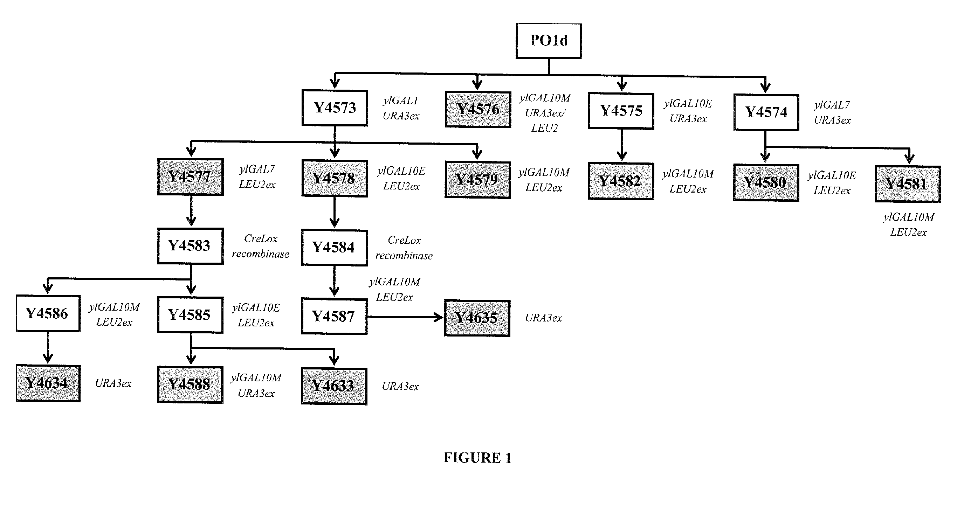

**Figure 1****:** Construction of *Y. lipolytica* strains overexpressing *ylGAL* genes. The auxotrophic PO1d strain was used as the acceptor strain. The genes were inserted one by one to create strains that overexpressed different combinations of the *ylGAL* genes; URA3ex and LEU2ex were used as selection markers. To recover prototrophy in some of the *Y. lipolytica* strains (gray squares), cassettes containing the URA3 excisable marker or the purified *Sal*I fragment of a pINA62 plasmid that contained the *LEU2* gene were inserted into the transformants' genomes (Gaillardin and Ribet, 1987). In turn, to recover URA3 and LEU2 auxotrophy, which was necessary to continue the transformation process, a JME547 plasmid containing Cre-Lox recombinase was used (Fickers *et al.,* 2003).

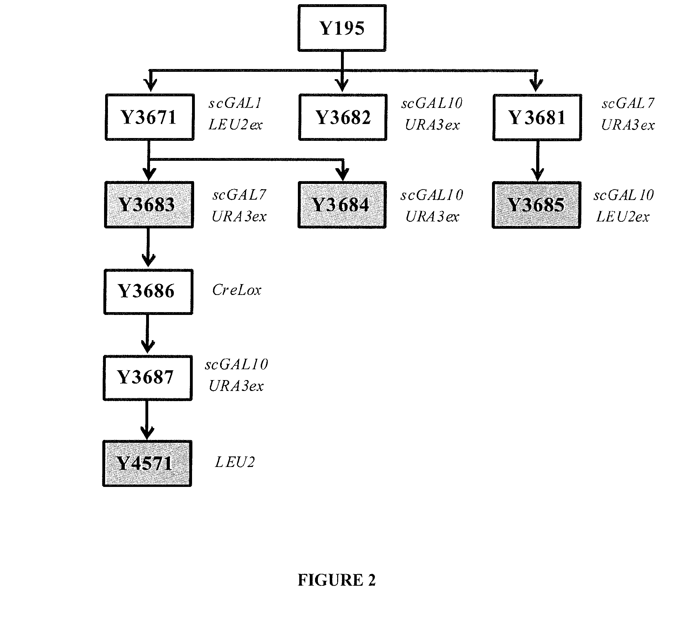

**Figure 2****:** Construction of the *Y*. *lipolytica* strains in which the *scGAL* genes were overexpressed. The auxotrophic PO1d strain was used as the acceptor strain. The genes were inserted one by one to create strains that overexpressed different combination of the *scGAL* genes; URA3ex and LEU2ex were used as selection markers. To recover auxotrophies necessary to continuing the transformation process, a JME547 plasmid containing Cre-Lox recombinase was used to transform *Y. lipolytica* Y3683, thus generating strain Y3686 (Fickers *et al.,* 2003). In turn, to recover prototrophy in the *Y. lipolytica* Y3687 strain, a purified *Sal*I fragment of the pINA62 plasmid that contained the *LEU2* gene was introduced (Gaillardin and Ribet, 1987).

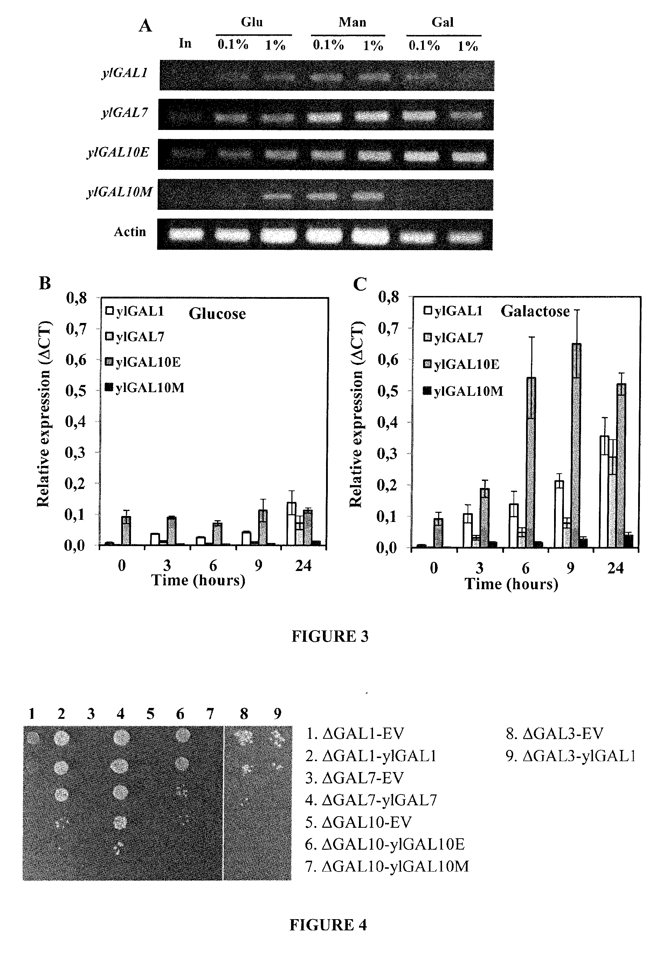

**Figure 3****:** Expression profiles of *ylGAL* genes in *Y*. *lipolytica* W29. Cells were incubated for 3 hours in YNB medium containing 0.1% or 1.0% glucose, mannose, or galactose **(A).** Kinetics of *ylGAL* gene expression in *Y*. *lipolytica* W29 grown in YNB medium containing 1% glucose **(B)** or 1% galactose (C). Gene expression levels were normalized based on the expression levels of the actin gene (ΔCT). Abbreviation: In - inoculum.
**Figure 4****:** Complementation of *GAL* gene deletion in *S. cerevisiae* using GAL homologs found in *Y. lipolytica.* EV - strains complemented for uracil deletion using a pRS426TEF empty vector.

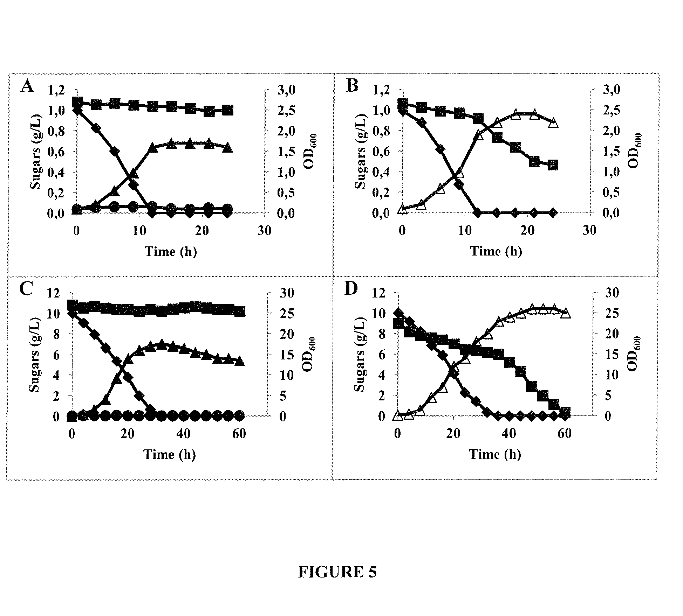

**Figure 5****:** *Y. lipolytica* W29 growth and sugar consumption over time in YNB medium containing glucose and galactose. **(A)** YNB medium containing only 0.1% glucose or 0.1 % galactose; **(B)** YNB medium containing a mixture of 0.1 % glucose and 0.1 % galactose; (C) YNB medium containing only 1% glucose or 1% galactose; **(D)** YNB medium containing a mixture of 1% glucose and 1% galactose. Symbols: glucose (◆), galactose (■), and optical density of cells growing in medium containing glucose (▲), galactose (●), or a mixture of glucose and galactose (Δ).

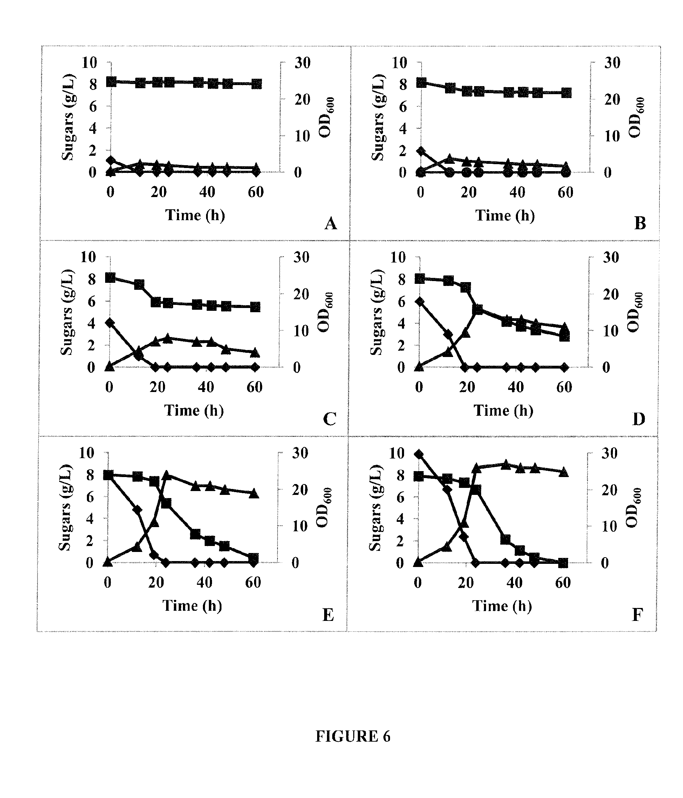

**Figure 6****:** Comparison of *Y. lipolytica* W29 growth and sugar consumption over time in YNB medium containing 1% galactose and different concentrations of glucose: 0.1% **(A);** 0.2% **(B);** 0.4% (C); 0.6% **(D);** 0.8% **(E);** 1.0% **(F).** Symbols: glucose (◆), galactose (■), and OD₆₀₀ (A).

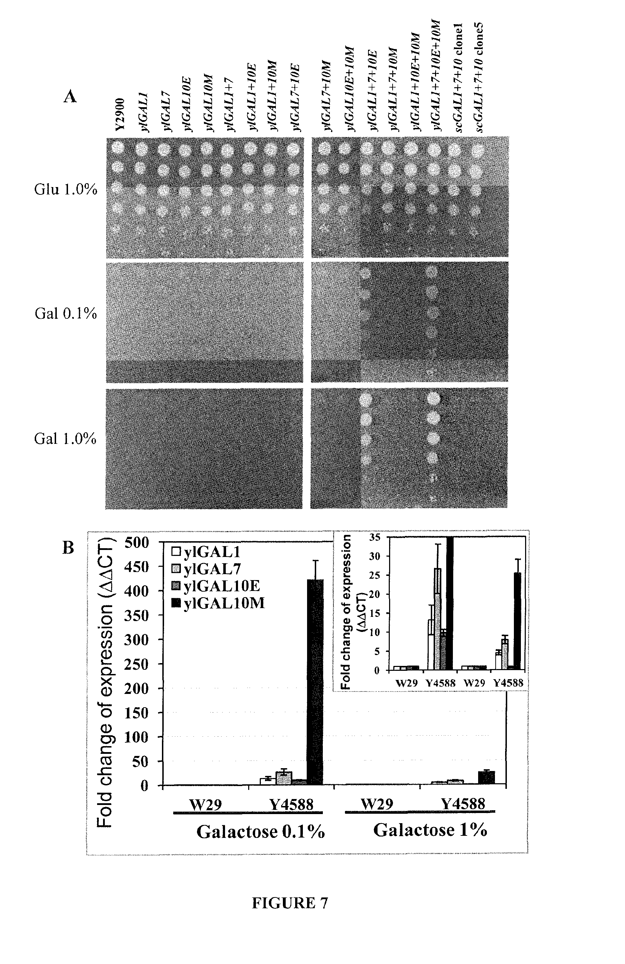

**Figure 7****:** Functional analysis of Leloir pathway overexpression in *Y. lipolytica* and fold change in the expression of the *ylGAL* genes. Growth of different *Y. lipolytica* transformants overexpressing *ylGAL* and *scGAL* genes on plates of YNB medium containing glucose or galactose **(A).** Fold change in the expression of *ylGAL* genes in the quadruple mutant, Y4588 (in which all the Leloir genes were overexpressed), relative to *Y. lipolytica* W29 (the wild-type strain) **(B);** yeast were incubated for 3 hours in YNB medium containing 0.1% or 1.0% galactose. Gene expression levels were normalized based on the expression of the actin gene.

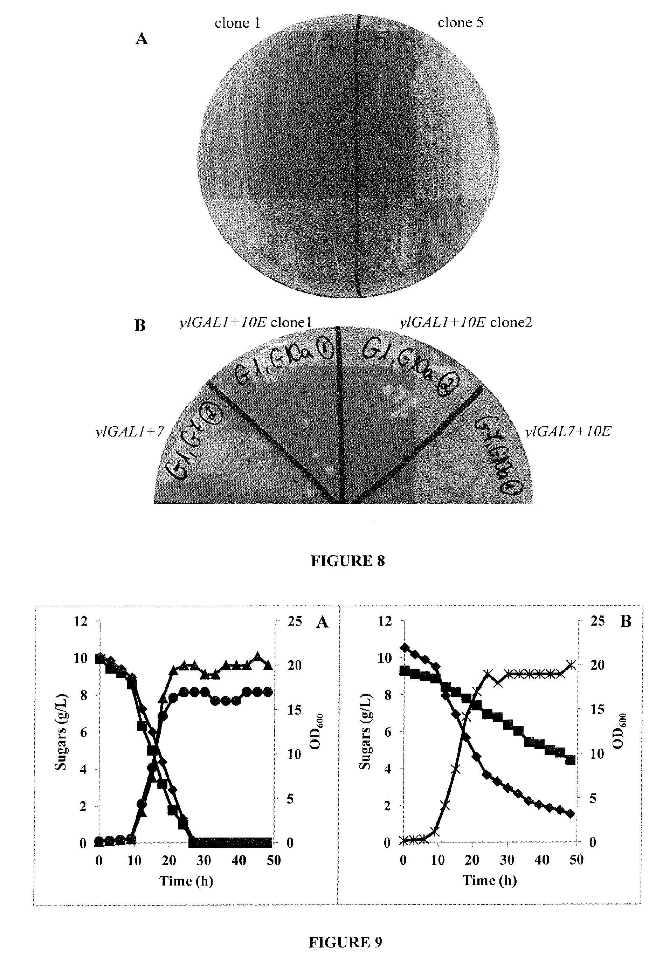

**Figure 8****:** Overexpression of *scGAL* and *ylGAL* genes in *Y. lipolytica.* Growth of *Y. lipolytica* transformants containing *scGAL* genes (*scGAL*1.7,10) on YNB medium containing 1% galactose **(A);** yeast were incubated for 1 week at 28°C. Growth of *Y. lipolytica* transformants overexpressing different combinations of *ylGAL* genes on YNB medium containing 1% galactose **(B);** yeast were incubated for 2 weeks at 28°C.
**Figure 9****:** *Y. lipolytica* Y4588 growth and sugar consumption over 48 h in YNB medium containing only 1% glucose, only 1% galactose **(A),** or a mixture of both 1% glucose and 1% galactose **(B).** Symbols: glucose (◆), galactose (■), OD₆₀₀ in glucose (A), OD₆₀₀ in galactose (●), and OD₆₀₀ in the mixture of both sugars (x).

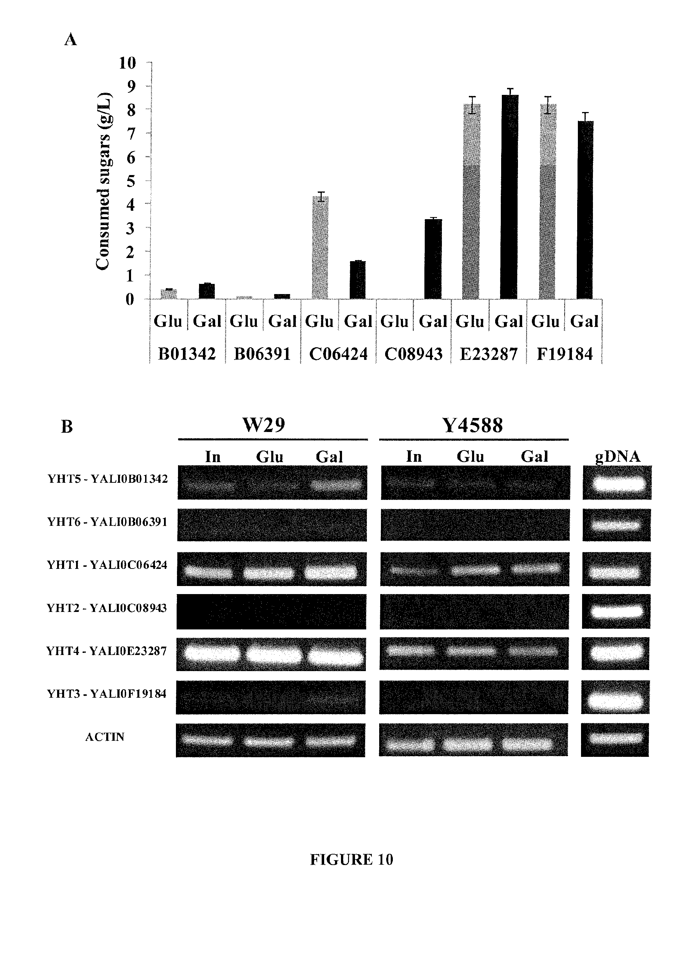

**Figure 10****:** Sugar consumption by S. *cerevisiae* null mutants expressing *Y. lipolytica* hexose transporters after 72 h of growth in YNB medium containing 1% glucose (grey square ■) or 1% galactose (black square ■) **(A).** Expression profiles of *Y. lipolytica* genes encoding hexose transporters in the W29 and Y4588 strains **(B).** Yeast were incubated for 3 hours in YNB medium containing 1.0% glucose or 1.0% galactose. The amplification of the PCR fragment in the genomic DNA served as a control for the primers' efficiency. Abbreviation: In - inoculum.

### EXEMPLE: ACTIVATION OF EFFICIENT GALACTOSE UTILIZATION BY YARROWIA LIPOLYTICA

### 1. Materials and methods

### 1.1. Yeast strains and plasmids

The strains and plasmids used in this example are listed in Table 1 below.

**Table 1: Bacteria and yeast strains used in the present example.**

| **Name** | **Relevant genotype** | **Reference** |
|---|---|---|
| ***E. coli* strains** | | |
| **JME547** | pUB4-CRE 1 | Fickers et al. (2003) |
| **JME2110** | JMP62pTEF-scGAL1-URA3ex | This study |
| **JME2111** | JMP62pTEF-scGAL1-LEU2ex | This study |
| **JME2114** | JMP62pTEF-scGAL7-URA3ex | This study |
| **JME2115** | JMP62pTEF-scGAL7-LEU2ex | This study |
| **JME2116** | JMP62pTEF-scGAL10-URA3ex | This study |
| **JME2117** | JMP62pTEF-scGAL10-LEU2ex | This study |
| **JME2542** | JMP62pTEF-ylGAL1-URA3ex | This study |
| **JME2543** | JMP62pTEF-ylGAL7-URA3ex | This study |
| **JME2544** | JMP62pTEF-ylGAL10E-URA3ex | This study |
| **JME2545** | JMP62pTEF-ylGAL10M-URA3ex | This study |
| **JME2546** | JMP62pTEF-ylGAL1-LEU2ex | This study |
| **JME2547** | JMP62pTEF-ylGAL7-LEU2ex | This study |
| **JME2548** | JMP62pTEF-ylGAL10E-LEU2ex | This study |
| **JME2549** | JMP62pTEF-ylGAL10M-LEU2ex | This study |
| **JME2735** | pRS426URA3-ylGAL1 | This study |
| **JME2736** | pRS426URA3-ylGAL7 | This study |
| **JME2738** | pRS426URA3-ylGAL10E | This study |
| **JME2739** | pRS426URA3-ylGAL10M | This study |

| ***Y***. ***lipolytica* strains** | | |
|---|---|---|
| **PO1d** | W29 *ura3-302 leu2-270 xpr2-322*+*pXPR2-SUC2* | Barth and Gaillardin (1996) |
| **W29** | *MATa ura3-302 leu2-270 xpr2-322 URA3ex LEU2* | Barth and Gaillardin (1996) |
| **Y3671** | *MATa ura3-302 leu2-270 xpr2-322 pTEF-scGAL1-LEU2ex* | This study |
| **Y3681** | *MATa ura3-302 leu2-270 xpr2-322 pTEF-scGAL7-URA3ex* | This study |
| **Y3682** | *MATa ura3-302 leu2-270 xpr2-322 pTEF-scGAL10-URA3ex* | This study |
| **Y3683** | *MATa ura3-302 leu2-270 xpr2-322 pTEF-scGAL1-LEU2ex pTEF-scGAL7-URA3ex* | This study |
| **Y3684** | *MATa ura3-302 leu2-270 xpr2-322 pTEF-scGAL1-LEU2ex pTEF-scGAL10-URA3ex* | This study |
| **Y3685** | *MATa ura3-302 leu2-270 xpr2-322 pTEF-scGAL7-URA3ex pTEF-scGAL10-LEU2ex* | This study |
| **Y3686** | *MATa ura3-302 leu2-270 xpr2-322 pTEF-scGAL1 pTEF-scGAL7* | This study |
| **Y3687** | *MATa ura3-302 leu2-270 xpr2-322 pTEF-scGAL1 pTEF-scGAL7 pTEF-scGAL10-URA3ex* | This study |
| **Y4571** | *MATa ura3-302 leu2-270 xpr2-322 pTEF*-*scGAL1 pTEF-scGAL7 pTEF-scGAL10-URA3ex LEU2* clone1 | This study |
| **Y4572** | *MATa ura3-302 leu2-270 xpr2-322 pTEF*-*scGAL1 pTEF-scGAL7 pTEF-scGAL10-URA3ex LEU2* clone5 | This study |
| **Y4573** | *MATa ura3-302 leu2-270 xpr2-322 pTEF-ylGAL1-URA3ex* | This study |
| **Y4574** | *MATa ura3-302 leu2-270 xpr2-322 pTEF-ylGAL7-URA3ex* | This study |
| **Y4575** | *MATa ura3-302 leu2-270 xpr2-322 pTEF-ylGAL10E-URA3ex* | This study |
| **Y4576** | *MATa ura3-302 leu2-270xpr2-322 pTEF-ylGAL10M-URA3ex* | This study |
| **Y4577** | *MATa ura3-302 leu2-270 xpr2-322 pTEF-ylGAL1-URA3ex pTEF-ylG4L7-LEU2ex* | This study |
| **Y4578** | *MATa ura3-302 leu2-270 xpr2-322 pTEF-ylGAL1-URA3ex pTEF-ylGAL10E-LEU2ex* | This study |
| **Y4579** | *MATa ura3-302 leu2-270 xpr2-322 pTEF-ylGAL1-URA3ex pTEF-ylGAL10M-LEU2ex* | This study |
| **Y4580** | *MATa ura3-302 leu2-270 xpr2-322 pTEF-ylGAL7-URA3ex pTEF-ylGAL10E-LEU2ex* | This study |
| **Y4581** | *MATa ura3-302 leu2-270 xpr2-322 pTEF-ylGAL7-URA3expTEF-ylGAL10M-LEU2ex* | This study |
| **Y4582** | *MATa ura3-302 leu2-270 xpr2-322 pTEF-ylGAL10a-URA3ex pTEF-ylGAL10M-LEU2ex* | This study |
| **Y4583** | *MATa ura3-302 leu2-270 xpr2-322 pTEF*-*ylGAL1 pTEF-ylGAL7* | This study |
| **Y4584** | *MATa ura3-302 leu2-270 xpr2-322 pTEF-ylGAL1 pTEF-ylGAL10E* | This study |
| **Y4585** | *MATa ura3-302 leu2-270 xpr2-322 pTEF-ylGAL1 pTEF-ylGAL7 pTEF-ylGALl10E-LEU2ex* | This study |
| **Y4586** | *MATa ura3-302 leu2-270 xpr2-322 pTEF-ylGAL1 pTEF-ylGAL7 pTEF-ylCAL10M-LEU2ex* | This study |
| **Y4587** | *MATa ura3-302 leu2-270 xpr2-322 pTEF-ylGAL1 pTEF-ylGAL10a pTEF-ylGAL10M-LEU2ex* | This study |
| **Y4588** | *MATa ura3-302 leu2-270 xpr2-322 pTEF-ylGAL1 pTEF-ylGAL7 pTEF-ylGALl10E-LEU2ex pTEF-ylGAL10M-URA3ex* | This study |
| **Y4629** | *MATa ura3-302 leu2-270 xpr2-322 pTEF-ylGAL1-URA3ex LEU2* | This study |
| **Y4630** | *MATa ura3-302 leu2-270 xpr2-322 pTEF-ylGAL7-URA3ex LEU2* | This study |
| **Y4631** | *MATa ura3-302 leu2-270 xpr2-322 pTEF-ylGAL10E-URA3ex LEU2* | This study |
| **Y4632** | *MATa ura3-302 leu2-270 xpr2-322 pTEF-ylGAL10M-URA3ex LEU2* | This study |
| **Y4633** | *MATa ura3-302 leu2-270 xpr2-322 pTEF-ylGAL1 pTEF-ylGAL7 pTEF-ylGALl10E-LEU2ex URA3ex* | This study |
| **Y4634** | *MATa ura3-302 leu2-270 xpr2-322 pTEF-ylGAL1 pTEF-ylGAL7 pTEF-ylGAL10M-LEU2ex URA3ex* | This study |
| **Y4635** | *MATa ura3-302 leu2-270 xpr2-322 pTEF-ylGAL1 pTEF-ylGAL10a pTEF-ylGAL10M-LEU2ex URA3ex* | This study |

| ***S. cerevisiae* strains** | | |
|---|---|---|
| **Y4473** | *Mat α; his3Δ1; leu2Δ0; met15Δ0; ura3Δ0; YBR018c::kanMX4 (ΔGal7)* | Euroscarf (Y13155) |
| **Y4474** | *Mat α; his3Δ1; leu2Δ0; lys2Δ0; ura3Δ0; YBR019c::kanMX4 (ΔGal10)* | Euroscarf (Y13156) |
| **Y4475** | *Mat α; his3Δ1; leu240; lys2Δ0; ura3Δ0; YBR020w::kanMX4 (ΔGal1)* | Euroscarf (Y13157) |
| **Y4846** | *Mat α; his3Δ1; leu2Δ0; lys2Δ0; ura3Δ0; YDR009w::kanMX4 (ΔGal3)* | Euroscarf (Y13949) |
| **Y4591** | *Mat α; his3Δ1; leu2Δ0; lys2Δ0; ura3Δ0; YBR020w:: kanMX4 URA3-EV(pRS426TEF)* | This study |
| **Y4592** | *Mat α; his3Δ1; leu2Δ0; lys2Δ0; ura3Δ0; YPL248c::kanMX4 URA3-EV(pRS426TEF)* | This study |
| **Y4593** | *Mat α; his3Δ1; leu2Δ0; met15Δ0; ura3Δ0; YBR018c::kanMX4 URA3-EV(pRS426TEF)* | This study |
| **Y4594** | *Mat α; his3Δ1; leu2Δ0; lys2Δ0; ura3Δ0; YBR019c::kanMX4 URA3-EV(pRS426TEF)* | This study |
| **Y4595** | *Mat α; his3Δ1; leu2Δ0; lys2Δ0; ura3Δ0; YBR020w::kanMX4 pTEF-ylGAL1-URA3* | This study |
| **Y4596** | *Mat α; his3Δ1; leu2Δ0; met15Δ0; ura3Δ0; YBR018c::kanMX4 pTEF-ylGAL7-URA3* | This study |
| **Y4597** | *Mat α; his3Δ1; leu2Δ0; lys2Δ0; ura3Δ0; YBR019c::kanMX4 pTEF-ylGAL10E-URA3* | This study |
| **Y4690** | *Mat α; his3Δ1; leu2Δ0; lys2Δ0; ura3Δ0; YBR019c::kanMX4 pTEF-ylGAL10M-URA3* | This study |
| **Y4874** | *Mat α; his3Δ1; leuΔ0; lys2Δ0; ura3Δ0; YDR009w::kanMX4 URA3-EV(pRS426TEF)* | This study |
| **Y4875** | *Mat α; his3Δ1; leu2Δ0; lys2Δ0; ura3Δ0; YDR009w::kanMX4 URA3-ylGAL1(ΔGal3)* | This study |
| **B01342** | *leu2-3,112 ura3-52 trp1-289 his31 MAL2-8C SUC2 hxt17 hxt13 hxt15 hxt16 hxt14 hxt12 hxt9 hxt11 hxt10 hxt8 hxt5-1-4 hxt2 hxt367 gal2 slt1 agt1 ydl247 yjr160c URA3-YALI0B01342* | Lazar et al., in preparation |
| **B06391** | *leu2-3,112 ura3-52 trpl-289 his31 MAL2-8CSUC2 hxt17 hxt13 hxt15 hxt16 hxt14 hxt12 hxt9 hxt11 hxt10 hxt8 hxt5-1-4 hxt2 hxt367 gal2 slt1 agt1 ydl247 yjr160c URA3-YALI0B06391* | Lazar et al., in preparation |
| **C06424 *(YHT1)*** | *leu2-3,112 ura3-52 trp1-289 his31 MAL2-8C SUC2 hxt17 hxt13 hxt15 hxt16 hxt14 hxt12 hxt9 hxt11 hxt10 hxt8 hxt5-1-4 hxt2 hxt367 gal2 slt1 agt1 ydl247 yir160c URA3-YALI0C06424* | Lazar et al., in preparation |
| **C08943 *(YHT2)*** | *leu2-3,112 ura3-52 trp1-289 his31 MAL2-8C SUC2 hxt17 hxt13 hxt15 hxt16 hxt14 hxt12 hxt9 hxt11 hxt10 hxt8 hxt5-1-4 hxt2 hxt367 gal2 sltl agt1 ydl247 yjr160c URA3-YALI0C08943* | Lazar et al., in preparation |
| **E23287 *(YHT4)*** | *leu2-3,112 ura3-52 trp1-289 his31 MAL2-8C SUC2 hxt17 hxt13 hxt15 hxt16 hxt14 hxt12 hxt9 hxt11 hxt10 hxt8 hxt5-1-4 hxt2 hxt367 gal2 slt1 agt1 ydl247 yjr160c URA3-YALI0E23287* | Lazar et al., in preparation |
| **F19184 *(YHT3)*** | *leu2-3,112 ura3-52 trp1-289 his31 MAL2-8C SUC2 hxt17 hxt13 hxt15 hxt16 hxt14 hxt12 hxt9 hxt11 hxt10 hxt8 hxt5-1-4 hxt2 hxt367 gal2 slt1 agt1 ydl247 yjr160c URA3-YALI0F19184* | Lazar et al., in preparation |

All the *Y. lipolytica* transformants were created by employing the genetic background of the W29 wild-type strain (Barth and Gaillardin, 1996). The auxotrophic strain PO1d (Ura⁻Leu⁻) was derived from W29 and was also used in this study (Barth and Gaillardin 1996). Because many different strains overexpressing *Y. lipolytica GAL* genes were created, only the quadruple overexpressing strain, Y4588, which overexpressed all four of the *ylGAL* genes, is described in detail here. Construction of the other strains is depicted in Figure 1. Plasmids containing *ylGAL* genes were constructed using URA3ex and LEU2ex selection markers. Strain Y4573, which overexpressed the *Y. lipolytica ylGAL1* gene (YALI0C13090g), was obtained by introducing the overexpression cassette from JME2542 containing the URA3ex selection marker into PO1d. Subsequently, the cassette containing the *ylGAL7* gene (YALI0F23947g) and a LEU2ex marker taken from JME2547 was introduced into the Y4573 strain, thus generating the prototrophic Y4577 strain. To excise both selection markers, Y4577 was transformed with JME547 plasmid containing Cre-Lox recombinase and hygromycin selection. This process generated the auxotrophic Y4583 strain (Ura⁻Leu⁻). Immediately afterwards, the *ylGAL10E* gene (YALI0E26829g) overexpression cassette containing the LEU2ex marker from a JME2548 plasmid was introduced into the Y4583. Then, the cassette containing the *ylGAL10M* gene (YALI0C09570g) and the URA3ex marker taken from a JME2545 plasmid were introduced, generating Y4588, which overexpressed the entire Leloir pathway.

*Y. lipolytica* strains expressing *S. cerevisiae GAL* genes were constructed as depicted in Figure 2. All the plasmids were prepared as described above.

Functional complementation analysis of the *Y. lipolytica GAL* genes was performed using *S. cerevisiae* strains from which the *GAL* genes had been deleted. The *Y. lipolytica* galactokinase (*ylGAL1*) gene was introduced into *S. cerevisiae* Δ*gal1* (Y4475) as well as into the Δ*gal3* (Y4846) strains using JME2735 plasmid, thus generating the Y4595 and Y4875 strains, respectively. To complement the Δ*gal7* strain, a JME2736 plasmid (*ylGAL7*) was introduced into Y4473, generating Y4596. Finally, Δ*gal10* was complemented separately by the *ylGAL10E* and *ylGAL10M* genes, which was accomplished by introducing JME2738 and JME2739 plasmids into the Y4474 strain, resulting in the creation of Y4597 and Y4690, respectively. All the *S. cerevisiae* deletion mutants - the control strains - were also complemented for uracil auxotrophy by the insertion of an empty pRS426TEF vector.

All the genes introduced into *Y. lipolytica* and *S. cerevisiae* were under the control of the constitutive *TEF* promoter (Mumberg *et al.,* 1995; Müller *et al.,* 1998). Transformation of *Y. lipolytica* and *S*. *cerevisiae* was performed using the lithium acetate procedure (Xuan *et al.,* 1990). Expression cassettes were integrated into the genome of *Y. lipolytica* (Mauersberger *et al.,* 2001) or were in the replicative pRS426TEF vector in *S. cerevisiae* (Mumberg *et al.,* 1995).

### 1.2. General genetic techniques and plasmid construction

Standard molecular genetics techniques were used throughout this study (as per Sambrok and Russell, 2001). Restriction enzymes were obtained from New England Biolabs (Ipswich, England). Genomic DNA from the transformants was prepared as described in Querol *et al.* (1992). PCR amplification was performed using an Eppendorf 2720 thermal cycler; both Taq (Promega, Madison, WI) and Pfu (Stratagene, La Jolla, CA) DNA polymerases were employed. PCR fragments were then purified with a QIAgen Purification Kit (Qiagen, Hilden, Germany), and DNA fragments were recovered from the agarose gels using a QIAquick Gel Extraction Kit (Qiagen, Hilden, Germany). The Staden software package was used for gene sequence analysis (Dear and Staden, 1991). The *GAL* genes were amplified using the primers listed in Table 2 below.

**Table 2: List of primers used in the present example.**

| **Primer** | **Sequence** | **Restriction site** | **Amplified gene** | **SEQ ID NO:** |
|---|---|---|---|---|
| Gal1-BamHI-fwd | gcgc**ggatcc**atgactaaatctcattcagaagaagtgattg | BamHI | *scGAL1* | 30 |
| Gal1-AvrII-rev | gcgc**cctagg**ttataattcatatagacagctgcccaatgc | AvrII | | 31 |
| ScGal7-BamHI-fwd | gcgc**ggatcc**atgactgctgaagaatttg | BamHI | *scGAL7* | 32 |
| Gal7-AvrII-rev | gcgc**cctagg**ttacagtctttgtagataatgaatc | AvrII | | 33 |
| Gal10-BamHI-fwd | gcgc**ggatcc**atgacagctcagttacaaag | BamHI | *scGAL10* | 34 |
| Gal10-AvrII-rev | gcgc**cctagg**tcaggaaaatctgtagacaatc | AvrII | | 35 |
| YLGal1Short-BamHI-fwd | gtga**ggatcc**atgtctatcagcaccctgcc | BamHI | *ylGAL1* | 36 |
| YlGal1-AvrII-rev | gcgc**cctagg**ttacaaatcaacaatagcacatccc | AvrII | | 37 |
| YlGal7-BamHI-fwd | gaga**ggatcc**atgactcttgtggcatccg | BamHI | *ylGAL7* | 38 |
| YlGal7-AvrII-rev | gcgc**cctagg**ttacaaatcatacaaccgcttgg | AvrII | | 39 |
| YlGal10E-BgIII-fwd | gaga**agatct**atgacccaggctgctgctgaac | BglII | *ylGAL10E* | 40 |
| YlGa110E-SpeI-rev | gcgc**actagt**ttacttgtcatgacggtcctttttgtaacc | Spel | | 41 |
| YlGal10M-BamHI-fwd | gaga**ggatcc**atgaccttttgcaacacgggagcaac | BamHI | *ylGAL10M* | 42 |
| YlGal10M-AvrII-rev | gcgc**cctagg**tcactcccgctccaaaacatagataatcttagc | AvrII | | 43 |
| YLGal1Short-SpeI-fwd | gcgc**actagt**atgtctatcagcaccctgcc | SpeI | *ylGAL1* | 44 |
| YlGal1-SmaI-rev | gcgc**cccggg**ttacaaatcaacaatagcacatccctc | SmaI | | 45 |
| YlGal7-SpeI-fwd | gaga**actagt**atgactcttgtggcatccg | Spel | *ylGAL7* | 46 |
| YlGal7-SmaI-rev | gcga**cccggg**ttacaaatcatacaaccgcttggc | SmaI | | 47 |
| YlGal10E-SpeI-fwd | gaga**actagt**atgacccaggctgctgctg | Spel | *ylGAL10E* | 48 |
| YlGal10E-SmaI-rev | gaga**cccggg**ttacttgtcatgacggtcctttttg | SmaI | | 49 |
| YLGal10M-SpeI-fwd | gcgc**actagt**atgaccttttgcaacacgggagcaac | SpeI | *ylGAL10M* | 50 |
| YLGal10M-EcoRI-rev | gcgc**gaattc**tcactcccgctccaaaacatagataatcttag | EcoRI | | 51 |

| Primers used for RT-PCR and qRT-PCR | | | | |
|---|---|---|---|---|
| YlGal1-RT-F | tcttcaactgctcctgtcct | *ylGAL1* | | 52 |
| YlGal1-RT-R | ctatcgtaatcgacaccatcc | | | 53 |
| YlGal7-RT-F | ccatcaaaaagctgaccaag | *ylGAL7* | | 54 |
| YlGal7-RT-R | atcgcagaagaggagggtag | | | 55 |
| YlGal10E-RT-F | tggtgacgactacccctct | *ylGAL10E* | | 56 |
| YlGal10E-RT-R | cggttcctaggttccattct | | | 57 |
| YlGal10M-RT-F | tccatctccctcaatggaac | *ylGAL10M* | | 58 |
| YlGal10M-RT-R | gccggtatagacctggaaac | | | 59 |

The restriction sites in the primer sequences enabled the genes to be cloned into JME1128 plasmids that had been digested with *Bam*HI-*Avr*II, as previously described elsewhere (Beopoulos *et al.,* 2008; Dulermo *et al.,* 2013). Auxotrophies were restored via excision using the Cre-lox recombinase system following transformation with the replicative plasmid pUB4-Cre1 (JME547) (Fickers *et al.,* 2003).

To complement the *S. cerevisiae* strains whose *GAL* genes had been deleted, the corresponding genes were cloned in a pRS426 vector using the constitutive TEF promoter and the uracil selection marker. The corresponding *ylGAL* genes were amplified and digested, as described above, with the restriction enzymes listed in Table 2 above.

### 1.3. Growth media

Media and growth conditions for *Escherichia coli* were identical to those as described by Sambrook and Russell (2001), as were those of *Y. lipolytica* (Barth and Gaillardin, 1996). Rich (YPD) medium was prepared using 20 g.L⁻¹ Bacto™ Peptone (Difco, Paris, France), 10 g.L⁻¹ yeast extract (Difco), and 20 g.L⁻¹ glucose (Merck, Fontenay-sous-Bois, France). Minimal (YNB) medium was prepared using 1.7 g.L⁻¹ yeast nitrogen base (without amino acids and ammonium sulphate, Difco), 10 g.L⁻¹ glucose (Merck), 5 g.L⁻¹ NH₄Cl, and 50 mM phosphate buffer (pH 6.8). To complement the auxotrophies, 0.1 g.L⁻¹ uracil or leucine (Difco, Paris, France) were added as necessary.

However, the YNB media on which they were grown contained the following: 10.0 g.L⁻¹ of either glucose or galactose; 6.5 g.L⁻¹ of Yeast Nitrogen Base (without amino acids and ammonium sulphate, Difco); 10.0 g.L⁻¹ of (NH₄)₂SO₄; 0.018 g.L⁻¹ of leucin; 0.0115 g.L⁻¹ of histidin; and 0.025 g.L⁻¹ of lysin.

### 1.4. Growth in microtiter plates

Precultures were obtained from frozen stock, inoculated into tubes containing 5 mL YPD medium, and cultured overnight (170 rpm, 28°C). They were then washed with sterile distilled water; cell suspensions were adjusted to an OD₆₀₀ of 0.1. Yeast strains were grown in 96-well plates in 200 µl of minimal YNB medium containing 10 g.L⁻¹ of either glucose or galactose. Culturing was repeated three times; 2-3 technical replicates were performed for each condition. Cultures were maintained at 28°C under constant agitation using a Biotek Synergy MX microtiter plate reader (Biotek Instruments, Colmar, France); each culture's optical density at 600 nm was measured every 20 min for 72 h.

### 1.5. Growth conditions in the experiments

Initial precultures were established by inoculating 50 mL of YPD medium in 250 mL Erlenmeyer flasks with the yeast strains; this was followed by an overnight shaking step at 28°C and 170 rpm. The cell suspensions were washed three times with sterile distilled water and used to inoculate 50 mL of YNB medium to an OD₆₀₀ of 0.25. The cultures were grown until all the available sugar had been consumed.

In the galactose utilization experiment, the YNB medium contained one of the following: 1.0 g.L⁻¹ of glucose; 1.0 g.L⁻¹ of galactose; 1.0 g.L⁻¹ of both glucose and galactose; or 10.0 g.L⁻¹ of both glucose and galactose. Additionally, galactose utilization was observed in YNB medium containing 10.0 g.L⁻¹ of galactose and either 1.0; 2.0; 4.0; 6.0; 8.0; or 10.0 g.L⁻¹ of glucose.

### 1.6. Analysis of growth on galactose by drop test

To analyze the different strains' growth on galactose, a drop test was performed on cultures grown on YNB plates. The *Y. lipolytica* strains were grown in 5 mL of YPD medium for 24 h. The cell suspensions were then washed twice with water and resuspended at an OD₆₀₀ of 1. Successive 10-fold dilutions were performed (10⁰-10-⁵), and 5 µl of each dilution were spotted onto YNB plates containing 10.0 g.L⁻¹ of glucose, 1.0 g.L⁻¹ of galactose, or 10.0 g.L⁻¹ of galactose. Pictures were taken after the cultures had been incubated at 28°C for 48 h. The same protocol was used for the *S. cerevisiae* strains. However, the YNB media on which they were grown contained the following: 10.0 g.L⁻¹ of either glucose or galactose; 6.5 g.L⁻¹ of Yeast Nitrogen Base (without amino acids and ammonium sulphate, Difco); 10.0 g.L⁻¹ of (NH₄)₂SO₄; 0.018 g.L⁻¹ of leucin; 0.0115 g.L⁻¹ of histidin; and 0.025 g.L⁻¹ of lysin.

### 1.7. Analysis of galactose uptake by Y. lipolytica hexose transporters

To analyze galactose uptake, *Y. lipolytica* hexose transporters were expressed one at a time in the *S. cerevisiae* hxt null mutant strain EBY.VW4000 (kindly provided by E. Boles, Goethe University, Frankfurt am Main, Germany) using a replicative pRS426 vector containing the TEF promoter. The cells were grown in 5 mL of YNB medium composed of 6.5 g.L⁻¹ of Yeast Nitrogen Base (without amino acids and ammonium sulphate, Difco); 10.0 g.L⁻¹ of (NH4)₂SO₄ as well as leucin, histidin and tryptophan to complement auxotrophies and containing 20 g.L⁻¹ of maltose; the medium was refreshed 3 times per 24 h period to increase 2µ plasmid copy number. The cell suspensions were washed three times with sterile distilled water and used to inoculate 50 mL of YNB medium that contained either 10.0 g.L⁻¹ of glucose or 10.0 g.L⁻¹ of galactose. The culture conditions were as described above (paragraph 1.5). OD₆₀₀ and sugar concentration were analyzed.

### 1.8. Conditions and media used for lipid biosynthesis from galactose in flasks and bioreactors

The precultures were prepared as described above (1.5). The main culture was grown on 50 mL of YNB medium (C/N 60) containing the following: galactose 60.0 g; YNB 1.7 g; NH₄Cl 1.5 g; 0.7 g KH₂PO₄, and 1.0 g MgSO₄×7H₂O in 1 L. The pH was kept at 6.8 using 0.05 M phosphate buffer. Tap water was used as a source of microelements. To increase the C/N ratio of the medium to 100, 1.25 g.L⁻¹ of NH₄Cl was used. Culture conditions were as described above (2.5.1).

Lipid biosynthesis was also evaluated using batch cultures (BC) that were kept in 5-L stirred-tank BIO-STAT B-PLUS bioreactors (Sartorius, Frankfurt, Germany) for 96 h under the following conditions: 2-L working volume, 28°C, 800 rpm of agitation, and 3.5-L min⁻¹ aeration rate. The production medium was prepared as described above. The pH was kept at 6.8 using a 40% (w/v) NaOH solution. The cultures were grown in 0.2 L of YPD medium in 0.5-L flasks at 170 rpm, at 28°C for 48 h. The volume of the inocula added to the bioreactor cultures was equal to 10% of the total working volume.

### 1.9. Analsyis of ylGAL gene and hexose transporter expression

For the expression experiments, the wild-type strain (W29) and the quadruple mutant (Y4588) were grown in YNB medium that had been supplemented with 10 g.L⁻¹ glucose; they were kept at 28°C for 16 h. Immediately afterwards, the cell suspensions were washed twice with distilled water and transferred into fresh YNB medium that contained either 1.0 or 10.0 g.L⁻¹ of glucose, galactose, or mannose. Samples were harvested at 3 h post inoculation; three replicates were obtained. For the kinetics experiments, the same protocol was followed, except that samples were harvested at 3, 6, 9, and 24 h post inoculation. All the samples were frozen in liquid nitrogen and stored at -80°C.

Total RNA was extracted using the RNeasy Mini Kit (Qiagen, Hilden, Germany), and 1.5 µg of each sample was treated with DNase (Ambion, Life Technologies). cDNA was synthesized using the Maxima First Strand cDNA Synthesis Kit for RT-qPCR (Thermo Scientific). PCR reactions were performed using specific primers that targeted the 3' end of the genes (Table 2). For the semi-quantitative RT-PCR analyses, PCR was performed using the GoTaq DNA Polymerase Kit (Promega). For the quantitative RT-PCR analyses, gDNA dilutions were first tested for primer efficiency and then retained if their efficiency rating was higher than 90%. Amplifications were carried out using the SsoAdvanced Universal SYBR Green Supermix Kit (BIO-RAD). The following program was used: 98°C for 3 min, followed by 40 cycles of 98°C for 15 sec, 58°C for 30 sec, and 72°C for 30 sec. Finally, melting curves were generated to confirm amplification specificity. Both ΔCT and ΔΔCT methods were used to calculate relative expression levels; a constitutive gene, actin, was utilized as the reference control (Schmittgen and Livak, 2008).

### 1.10. Analytical techniques used in this study

Lipid bodies were stained with BodiPy^{®} Lipid Probe (2.5 mg.mL⁻¹ in ethanol; Invitrogen). Cell suspension samples (OD₆₀₀ = 5) were incubated for 10 min at room temperature. Images were obtained using a Zeiss Axio Imager M2 microscope (Zeiss, Le Pecq, France) equipped with a 100× objective lens and Zeiss filter sets 45 and 46 for fluorescence microscopy. Axiovision 4.8 software (Zeiss, Le Pecq, France) was used for image acquisition.

The fatty acids occurring in 15-mg aliquots of freeze-dried cells were converted into methyl esters using the method described in Browse *et al.* (1986). They were then analyzed using a gas chromatograph (GC). GC analysis of the methyl esters was performed using a Varian 3900 instrument equipped with a flame ionization detector and a Varian FactorFour vf-23ms column, for which bleed specification at 260°C was 3 pA (30 m, 0.25 mm, 0.25 µm). Fatty acids were identified by comparing the GC patterns of their methyl esters to those of commercial methyl ester standards (FAME32; Supelco); the amounts present were quantified using the internal standard method, which involved the addition of 50 µg of commercial C17:0 (Sigma).

Citric acid, glucose, fructose, and sucrose were identified and quantified by HPLC (UltiMate 3000, Dionex-Thermo Fisher Scientific, UK) using an Aminex HPX87H column coupled with UV (210 nm) and RI detectors. The column was eluted with 0.01 N H₂SO₄ at a flow rate of 0.6 mL.min⁻¹ at room temperature. Compounds were identified and quantified via comparisons to standards. Before undergoing HPLC analysis, the samples were filtered on membranes with a pore size of 0.45 µm.

To determine dry biomass, cell pellets from the 15-mL culture samples were washed twice with distilled water, filtered on the above membranes, and dried at 105°C using a WPS 110S weight dryer (Radwag, Poznań, Poland) until a constant mass was reached.

### 2. Results

### 2.1. Y. lipolytica has all the structural Leloir pathway genes

Although *Y. lipolytica* does not normally grow on galactose, its genome contains all the genes of the Leloir pathway (Slot and Rokas, 2010).

In *Y. lipolytica,* UDP-glucose-4-epimerase and galactose mutarotase are encoded by two different genes, which gave rise to the following four structural genes: *ylGAL1* (YALI0C13090g, galactokinase), *ylGAL7* (YALI0F23947g, galactose-1-phosphate uridyl transferase), *ylGAL10E* (YALI0E26829g, UDP-glucose-4 **E**pimerase), and *ylGAL10M* (YALI0C09570g, galactose **M**utarotase) (Table 3).

**Table 3: Comparison of GAL genes among Y. lipolytica, S. cerevisiae, and H. jecorina.**

| Enzyme name | *Y. lipolytica* | | | *S. cerevisiae* | | | Identity (%) | Similarity (%) | *Hypocrea jecorina* | | | Identity (%) | Similarity (%) |
|---|---|---|---|---|---|---|---|---|---|---|---|---|---|
| | Abbreviation | Gene | Protein length (aa) | Abbreviation | Gene | Protein length (aa) | | | Abbreviation | Locus | Protein length (aa) | | |
| Galactokinase | *ylGAL1* | YALI0C13090g | 476 | *sc*GAL1 | YBR020w | 528 | 40 | 55 | Gal1 | XP_006961307 | 526 | 40 | 56 |
| Galactose-1-phosphate uridyl transferase | *ylGAL7* | YALI0F23947g | 352 | *sc*GAL7 | YBR018c | 366 | 49 | 63 | Gal7 | XP_006968597 | 382 | 49 | 61 |
| UDP-glucose-4-epimerase | *yl*GAL10E | YALI0E26829g | 369 | *sc*GAL10* | YBR019c | 699 | 62 | 76 | Gal10E | AF439323_1 | 370 | 59 | 69 |
| Galactose mutarotase | *yl*GAL10M | YALI0C09570g | 327 | | | | 26 | 42 | Gal10M | XP_006964994 | 342 | 29 | 36 |

| | | | | | | | | | | | | | |
|---|---|---|---|---|---|---|---|---|---|---|---|---|---|
| *in *S. cerevisiae,* the GAL10 gene contains the two domains epimerase and mutarotase. | | | | | | | | | | | | | |

Although all the structural genes necessary for galactose utilization are present in *Y. lipolytica's* genome, the genes known to encode regulatory proteins in *S. cerevisiae* (*i*.*e*., *scGAL3, scGAL4,* and *scGAL80)* are not. This genetic difference accounts for the differences observed between the two species in the galactose utilization pathway. In addition, *GAL4* binding sites are not present in the promoter regions of *ylGAL* genes (data not shown), which is also the case for *H. jecorina* (Seiboth *et al.,* 2002). Despite this fact, *H. jecorina* is able to grow well on galactose.

### 2.2 Y. lipolytica GAL genes are expressed

To examine *ylGAL* gene expression, *Y. lipolytica* was grown on a suite of media containing two different concentrations (0.1% and 1.0%) of three different monosaccharides (galactose, glucose, and mannose), and transcription analyses were performed (Figure 3A). Galactose was the target compound, glucose was a potential repressor, and mannose was a neutral sugar in catabolite repression. The three main *ylGAL* genes *(ylGAL1, ylGAL7, ylGAL10E*) were expressed under all of the conditions, but *ylGAL10M,* the least important gene in galactose utilization, showed very weak expression (Figure 3A).

Since the *ylGAL* genes were not repressed by glucose, a kinetics analysis of their quantitative expression on glucose and galactose was conducted (Figures 3B, C). The gene profiles show that, on glucose, expression of the UDP-glucose-4-epimerase gene (*ylGAL10E*) was constant, whereas expression of galactokinase (*ylGAL1*) and galactose-1-phosphate uridyl transferase (*ylGAL7*) genes increased over time (Figure 3B). Once again, the galactose mutarotase gene (*ylGAL10M*) had the lowest expression levels. On galactose, there was a slight increase in expression over time: it reached between 2.7 and 4.6 after 24 h (Figure 3C). Therefore, the *ylGAL* genes were upregulated in the presence of galactose.

### 2.3 Y. lipolytica GAL genes are functional

To investigate the activity of the proteins encoded by the *Y. lipolytica* GAL genes, a functional complementation test was performed using *S. cerevisiae.* The *ylGAL* genes were amplified and cloned into a multicopy pRS426 vector under the control of the scTEF promoter. The *S. cerevisiae* transformants obtained were subsequently tested to determine if they could grow when galactose was the sole carbon source (Figure 4). The three cloned *ylGAL* genes, *ylGAL1, ylGAL7,* and *ylGAL10E,* complemented their corresponding *S. cerevisiae* GAL-deletion mutants and restored growth on galactose. This result shows that the *ylGAL* genes encode functional Leloir proteins. In addition, the *ylGAL1p* gene, which complemented the *S. cerevisiae* Δ*gal1* mutant, was not able to restore normal growth on galactose for the *S. cerevisiae* Δ*gal3* mutant (Figure 4).

### 2.4 Validation of Leloir pathway functionality in Y. lipolytica

Galactose utilization by *Y. lipolytica* was investigated. Results are shown in Figure 5. The *ylGAL* genes were expressed when the yeast was grown on glucose, but galactose alone did not allow growth. As a result, growth and sugar consumption by *Y. lipolytica* W29 (the wild-type strain) in YNB medium containing a mixture of glucose and galactose (0.1% or 1.0%) were analyzed. First, a control experiment was conducted in which growth and sugar consumption on glucose versus galactose were examined. As expected, when galactose was the sole carbon source, it was not consumed, whereas 0.1% and 1.0% glucose were depleted within 12 and 32 h, respectively (Figures 5A, C). Surprisingly, when the same strain was grown in medium containing both 0.1% glucose and 0.1% galactose, it was able to use both sugars (Figure 5B). The glucose utilization rate was the same as for the glucose-only culture (0.083 g.L⁻¹.h⁻¹). In contrast, galactose was initially used at a very low rate (0.009 g.L⁻¹.h⁻¹); however, after the glucose was completely consumed, the galactose utilization rate increased (0.032 g.L⁻¹.h⁻¹). Additionally, sugar consumption was also tested using cultures containing higher concentrations of both monosaccharides (1%) to eliminate the influence of any putative sugar sensors, which could change carbon metabolism (Figure 5D). As in the previous experiment, *Y. lipolytica* W29 used both glucose and galactose from the beginning, but the glucose consumption rate was much higher than the galactose consumption rate (0.313 g.L⁻¹.h⁻¹ versus 0.083 g.L⁻¹.h⁻¹, respectively). Once all the glucose had been consumed, the galactose utilization rate climbed significantly (0.250 g.L⁻¹.h⁻¹). It is worth noting that, not only was galactose consumed by the cells, but it was also partially used for biomass production (Figures 5B, D).

The concentration of glucose that *Y. lipolytica* requires to be able to use galactose was determined. *Y. lipolytica* W29 was therefore grown in a suite of media containing 1.0% galactose and a range of glucose, from 0.1 to 1.0%. At a glucose concentration of 0.1%, growth was very weak and galactose was not consumed (Figure 6A). At higher glucose concentrations (0.2-0.4%), a small amount of galactose was also utilized; however, after the glucose was used up, galactose consumption stopped (Figures 6B, C). At even higher glucose concentrations, *Y. lipolytica* consumed galactose more efficiently, following a short delay (Figures 6D-F). Galactose consumption increased once the glucose had been drained from the medium, which is also when the cells enter into the stationary phase (Figures 6D-F).

### 2.5. Overexpression of all Y. lipolytica GAL genes induces efficient growth on galactose

All the *ylGAL* genes were placed under the control of the strong constitutive TEF promoter and introduced individually or in various combinations into the *Y. lipolytica* PO1d background. The resulting strains were then tested to see if they could grow on galactose alone. When the transformants were grown on glucose, they showed no morphological changes, suggesting that their physiology was not affected (Figure 7A). Mutants in which *ylGAL1, ylGAL7,* and *ylGAL10E* were simultaneously overexpressed could grow on 0.1% and 1.0% galactose (Figure 7A, line 12). Mutarotase activity, which is not required for galactose metabolism, nonetheless appeared to improve galactose utilization (Figure 7A, line 15). Interestingly, the overexpression of all the *S. cerevisiae* Leloir genes in *Y. lipolytica* resulted in weak, delayed growth (Figure 8A). The improper folding of *scGAL10* may have been responsible; its counterpart in *Y. lipolytica* exists as two proteins. Additional analysis of growth patterns showed that the *Y. lipolytica* transformants, with the exception of Y4585 and Y4588, took a long time to adapt to using galactose. The Y4577 strain, in which *ylGAL1* and *ylGAL7* were overexpressed, began to grow after two weeks of incubation but revealed normal morphology on plates (Figure 8B). Additionally, the Y4578 strain, in which *ylGAL1* and *ylGAL10E* were overexpressed, also began to grow after two weeks of incubation, albeit much less efficiently than Y4577 (Figure 8B). This finding underscores that *ylGAL7* expression results in functional protein biosynthesis but not normal levels for galactose utilization.

The strong increase in gene expression in the quadruple mutant (Y4588) showed that, when galactose concentrations were low, each gene was overexpressed compared to the parental strain; for instance, expression was 400 times higher for *ylGAL10M* (Figure 7B). Surprisingly, when galactose concentrations were high (1.0%), the change in gene expression levels was not so sharp, especially in the case of *ylGAL10E.* The parental strain expresses *ylGAL10E* at high levels when grown in 1% galactose (Figure 7B). Overexpressing all four of the *ylGAL* genes efficiently activated the Leloir pathway and created a strain that can use galactose as its sole carbon source.

### 2.6. Overexpression of ylGAL genes changed sugar utilization kinetics in Y. lipolytica without changing transporters expression

Glucose must be present in the culture medium for the *Y lipolytica* wild type (W29) strain to be able to utilize galactose. Overexpression of all the genes in the Leloir pathway allowed *Y. lipolytica* cells to grow on media whose sole carbon source was galactose. It was then determined if glucose had an effect on galactose utilization in the *Y. lipolytica* quadruple mutant (Y4588). To this end, its growth and sugar utilization in 1% glucose, 1% galactose, and a mixture of 1% glucose and 1% galactose were characterized. The kinetics of sugar consumption were the same when the strain was grown in the single sugar cultures, reaching 0.48-49 g.L⁻¹.h⁻¹; slightly more biomass was produced when the strain was grown on glucose alone (Figure 9A). However, sugar utilization dynamics changed in the mixed medium (Figure 9B). The galactose utilization rate maxed out at 0.11 g.L⁻¹.h⁻¹, while the glucose utilization rate declined to 0.39 g.L⁻¹.h⁻¹. This same pattern was also seen at lower concentrations of both sugars (0.1% and 0.5%; data not shown). After the lag phase, galactose utilization remained slow and constant, while glucose consumption decreased significantly when the cells reached the stationary phase (Figure 9B). Overall, growth and sugar consumption were the same on the glucose-only and mixed-sugar media; for instance, at 18 h of incubation, consumption was around 5.5-6 g.L⁻¹ for both (Figure 9B). Even though the galactose utilization pathway had clearly been activated, glucose was obviously the preferred carbon source.

The transport of galactose inside the cell is the first step in the metabolic process. It may be modified in the quadruple mutant or may be affected by the presence of glucose. In *Y. lipolytica,* six genes have been identified as coding hexose transporters from a HXT-like family; they have been named in series, YHT1 to YHT6, and have been reported to transport of galactose (Lazar *et al.,* in preparation; Young *et al.,* 2011). The uptake efficiency of both galactose and glucose was compared in *S. cerevisiae* hxt null mutants transformed with each of these six genes (Figure 10A). Four of the transporters, Yht1-Yht4, allowed significant galactose or glucose consumption; uptake efficiency, however, varied (Figure 10A). Only two of these transporter genes, *YHT1* and *YHT4,* were expressed at similarly high levels in *Y. lipolytica* W29 and Y4588 strains grown in either 1% glucose or 1% galactose (Figure 10B). These findings suggest that this set of galactose transporters is affected neither by the genetic background (WT or Y4588) nor by the carbon source.

### 2.7 Galactose is a good substrate for industrial processes using Y. lipolytica

To confirm that galactose could be effectively used in industrial applications (*e.g.*, the *Y. lipolytica* Y4588 strain could efficiently produce citric acid and lipids), batch cultures were raised in flasks and bioreactors. All the biomass, citric acid, and lipid production results are summarized in Table 4.

**Table 4: Biomass, citric acid, and lipid production. Y. lipolytica W29 and Y4588 strains were grown for 96 h in YNB medium containing 6% glucose or 6% galactose in flasks and bioreactors.**

| Parameter | | X | Y_{X/S} | CA | Y_{CA/S} | Y_{CA/X} | FA | Y_{FA/S} | Y_{FA/X} |
|---|---|---|---|---|---|---|---|---|---|
| | | g.L⁻¹ | g.g⁻¹ | g.L⁻¹ | g.g⁻¹ | g.g⁻¹ | g.L⁻¹ | g.g⁻¹ | g.g⁻¹ |
| Flasks | | | | | | | | | |
| Glu 6% (C/N 60) | W29 | 11.15 | 0.24 | 4.18 | 0.09 | 0.37 | 1.92 | 0.041 | 0.172 |
| Gal 6% (C/N 60) | Y4588 | 10.90 | 0.21 | 18.17 | 0.35 | 1.67 | 1.87 | 0.036 | 0.172 |
| Gal 6% (C/N 100) | | 11.27 | 0.19 | 12.58 | 0.22 | 1.12 | 1.98 | 0.034 | 0.176 |
| Bioreactors | | 8.45 | 0.17 | 16.96 | 0.35 | 2.01 | 1.79 | 0.037 | 0.212 |
| Gal 6% (C/N 100) | Y4588 | 19.4 | 0.34 | 29.2 | 0.51 | 1.51 | 3.22 | 0.056 | 0.166 |

| | | | | | | | | | |
|---|---|---|---|---|---|---|---|---|---|
| Symbols: X - dry biomass; FA - fatty acids; CA - citric acid; Y_{X/S} - yield of biomass from substrate consumed; Y_{CA/S} - yield of citric acid from substrate consumed; Y_{CA/X} - yield of citric acid from dry biomass; Y_{FA/S} - yield of fatty acids from substrate consumed; and Y_{FA/X} - yield of fatty acids from dry biomass. In this analysis, SD did not exceed 5%. | | | | | | | | | |

The *Y. lipolytica* wild-type (W29) and quadruple mutant (Y4588) strains produced similar amounts of biomass (∼11 g.L⁻¹) when grown in both glucose and galactose media with C/N ratio of 60. Biomass yield ranged from 0.19 to 0.24 g.g⁻¹. When the C/N ratio of the galactose medium was increased to 100, biomass yield decreased slightly (11%).

Interestingly, in glucose, citric acid biosynthesis and yield were around four times higher for Y4588 than for W29. Furthermore, Y4588 was able to produce three times more citric acid from galactose (C/N 60) than did W29 grown in glucose. Finally, when the C/N ratio was increased to 100, biomass and citric acid yield from galactose increased by 55% and 37%, respectively.

Galactose could also be used to efficiently produce lipids. In glucose with a C/N ratio of 60, W29 and Y4588 produced similar amounts of total lipids. Y4588 was able to produce comparable amounts of total lipids in galactose- and glucose-only media (Table 4). Lipid yields for W29 and Y4588 ranged between 0.034 and 0.041 g.g⁻¹. Additionally, increasing the C/N ratio to 100 improved lipid accumulation by 20%; it reached 0.212 g.g⁻¹. W29 had similar or lower yields when grown in glucose or fructose media with the same C/N ratio (Lazar *et al.,* 2014).

To study Y4588's ability to produce citric acid and lipids in large quantities, the production was scaled up by using 2-L bioreactors. Overall, biomass, citric acid, and lipid production were dramatically improved (Table 4). Up to 19.4 g.L⁻¹ of biomass was produced under these conditions, representing an increase of 100%. Citric acid production reached 29.2 g.L⁻¹ and thus the conversion of substrate to citric acid was improved by 31%. Additionally, lipid production increased from 1.79 to 3.22 g.L⁻¹, which signifies that 34% more galactose was converted into lipids. The construction of a *Y. lipolytica* strain that is able to produce citric acid and lipids from galactose is a very important step in increasing the range of carbohydrate substrates used by this yeast for industrial applications.

### REFERENCES

André, A., et al., 2009. Eng. in Life Sci. 9:468-478.
Barth G. and Gaillardin C., 1996 Yarrowia lipolytica, In Nonconventional yeasts in biotechnology, vol. 1, K. Wolf, K.D. Breunig, and G. Barth (ed.), Springer-Verlag, Berlin, Germany, 313-388
Beopoulos A., et al., 2008. Applied Environmental Microbiology. 74: 7779-7789.
Beopoulos A., et al., 2012. Applied Microbiology and Biotechnology. 93: 1523-1537.
Blazeck, J., 2011. Appl. Environ Microbiol. 77: 7905-7914.
Blazeck, J., 2013. Appl. Microbiol Biotechnol. 97:3037-3052.
Blazeck J, et al., 2014. Nature Communications. 5: 3131, DOI: 10.1038/ncomms4131.
Bouffard G.G., et al., 1994. J Mol Biol. 162: 156-159.
Browse, J., et al., 1986, Anal. Biochem. 152:141-145.
Christensen U., et al., 2011. Appl Environ Microbiol. 77: 7084-7087.
Coelho M.A.Z., et al., 2010. Yarrowia lipolytica: an industrial workhorse. In: Mendez-Villas A (ed). Current Research, Technology and Education Topics in Applied Microbiology and Microbial Biotechnology. Vol. 2. Formatex, Badajoz, p 930.
Dear S., Staden R. 1991. Nucleic Acids Res. 19: 3907-3911.
Dulermo T., et al., 2013. Biochimica and Biophysica Acta. 1831: 1486-1495.
Fakas, S., et al., 2006, Appl Microbiol Biotechnol., 73:676-683.
Fickers P., et al., 2003. Journal of Microbiological Methods. 55: 727-737.
Frey P.A., 1996. The FASEB Journal. 10: 461-470.
Gaillardin C., Ribet AM. 1987. Current Genetics. 11: 369-375.
Gruben B.S., et al., 2012. FEBS Letters. 586: 3980-3985.
Guo, X., et al., 2000, J. Appl. Microbiol. 89:107-115.
Gysler, C., et al., 1990, Biotechn Techn. 4:285-290.
Hartl L., et al., 2007. The Journal of Biological Chemistry. 282: 18654-18659.
Hedfalk, K. 2012 'Codon Optimisation for Heterologous Gene Expression in Yeast'. In Springer Protocols :Methods in Molecular Biology. Recombinant protein production in yeast :methods and protocols. Volume 866 pp. 47-55. Springer Eds.
Holden H.M., et al., 2003. The Journal of Biological Chemistry. 278: 43885-43888.
Ito, H., et al., 1983, J Bacteriol. 153:163-168.
Klebe, R.J., et al., 1983, Gene. 25:333-341.
Koivistoinen O.M., et al., 2012. FEBS Letters. 586: 378-383.
Lazar Z., et al., 2014. Metabolic Engineering. 26: 89-99.
Le Dall, M.T., et al., 1994, Curr Genet. 26:38-44.
Liu X., et al., 2014. Appl Biochem Biotechnol. DOI 10.1007/s12010-014-1430-0
Madzak C., et al., 2000, J Mol Microbiol Biotechnol. 2:207-216.
Madzak, C., et al., 2004, J Biotechnol. 109:63-81.
Majumdar S., et al., Eur. J. Biochem. 2004. 271: 753-759.
Mauersberger S., et al., 2001. Journal of Bacteriology. 183: 5102-5109.
Meyer J., et al., 1991. Mol Cell Biol. 11:5454-5461.
Michely S., et al., 2013. PLOS one. 8: e63356 (1-10).
Mirończuk A.M., et al., 2014. Journal of Industrial Microbiology and Biotechnology. 41: 57-64.
Mojzita D., et al., 2012. J Biol Chem. 287: 26010-26018.
Müller S., et al., 1998. Yeast. 14:1267-1283.
Mumberg D., et al., 1995. Gene. 156: 119-122.
Orr-Weaver, T.L., et al., 1981, Proc. Natl. Acad. Sci. USA, 78:6354-6358.
Papanikolaou, S., et al., 2001, Antonie van Leeuwenhoek, 80:215-224.
Papanikolaou, S., et al., 2002, J. Appl. Microbiol. 92:737-744.
Papanikolaou, S., et al., 2008, Bioresour. Technol. 99:2419-2428.
Querol A., et al., 1992. Applied and Environmental Microbiology, 58: 2948-2953.
Sambrook J., Russell DW. 2001. Molecular Cloning: a Laboratory Manual (3rd ed.) Cold Spring Harbor Laboratory Press, New York
Schädel C., et al., 2010. Plant Physiology and Biochemistry. 48: 1-8.
Schmittgen T.D., Livak K.J., 2008. Nature Protocols. 3: 1101-1108.
Segawa T. and Fukasawa T., 1979. J. Biol. Chem. 254: 10707-10709.
Seiboth B., et al., 2002. Gene. 295: 143-149.
Sellick C.A., et al., 2008. Int Rev Cell Mol Biol. 269: 111-50.
Schell M.A. and Wilson D.B., 1977. J. Biol. Chem. 252: 1162-1166.
Slot J.C., Rokas A., 2010. PNAS. 107: 10136-10141.
Tai M., Stephanopoulos G. 2013. Metabolic engineering. 15: 1-9.
Tatusova, T.A. and Madden, T.L., 1999. FEMS Microbiol. Lett. 174:247-250.
van der Walt J.P., von Arx JA. 1980. Antonie van Leeuwenhoek. 46: 517-521.
Wang W., et al., 2014. PLOS One, DOI: 10.1371/journal.pone.0111443
Wong T.Y., Yao X.T., 1994. Applied and environmental Microbiology, 60: 2065-2068.
Xuan J.W., et al., 1990. Molecular and Cellular Biology. 10: 4795-4806.
Young E., et al., 2011. Appl Environ Microbiol. 77: 3311-3319.

## Claims

1. A method for obtaining a *Yarrowia* strain capable of growing on D-galactose as sole carbon source, wherein said method comprises overexpressing in said strain a galactokinase (E.C 2.7.1.6) having at least 75% identity with the polypeptide of sequence SEQ ID NO: 2, a galactose-1-phosphate uridyl transferase (E.C 2.7.7.12) having at least 65% identity with the polypeptide of sequence SEQ ID NO: 4, an UDP-glucose-4 epimerase (E.C5.1.3.2) having at least 85% identity with the polypeptide of sequence SEQ ID NO: 6 and a galactose mutarotase (E.C5.1.3.3) having at least 45% identity with the polypeptide of sequence SEQ ID NO: 8.

2. The method according to claim 1, wherein 1, 2, 3 or 4 of said enzymes galactokinase, galactose-1-phosphate uridyl transferase, UDP-glucose-4 epimerase and galactose mutarotase, are from a *Yarrowia* strain.

3. The method according to claim 1 or claim 2, wherein said galactokinase has the consensus amino acid sequence SEQ ID NO: 9.

4. The method according to any one of claims 1 to 3, wherein said galactokinase is selected from the group consisting of SEQ ID NO: 2, 10, 11, 12 and 13.

5. The method according to any one of claims 1 to 4, wherein said galactose-1-phosphate uridyl transferase has the consensus amino acid sequence SEQ ID NO: 14.

6. The method according to any one of claims 1 to 5, wherein said galactose-1-phosphate uridyl transferase is selected from the group consisting of SEQ ID NO: 4, 15, 16, 17 and 18.

7. The method according to any one of claims 1 to 6, wherein said UDP-glucose-4 epimerase has the consensus amino acid sequence SEQ ID NO: 19.

8. The method according to any one of claims 1 to 7, wherein said UDP-glucose-4 epimerase is selected from the group consisting of SEQ ID NO: 6, 20, 21, 22 and 23.

9. The method according to any one of claims 1 to 8, wherein the amino acid sequence of said galactose mutarotase comprises, from the N-terminus to the C-terminus, the polypeptide fragments of SEQ ID NO: 24, 25 and 26.

10. The method according to any one of claims 1 to 9, wherein said galactose mutarotase is selected from the group consisting of SEQ ID NO: 8, 27, 28 and 29.

11. The method according to any one of claims 1 to 10, wherein said *Yarrowia* strain capable of growing on D-galactose as sole carbon source is a *Y. lipolytica* strain.

12. The method of any one of claims 1 to 11, wherein it comprises transforming a *Yarrowia* cell with 1, 2, 3 or 4 recombinant DNA constructs for expressing the 4 enzymes as defined in any one of claims 1 to 10.

13. A mutant *Yarrowia* strain, wherein a galactokinase having at least 75% identity with the polypeptide of sequence SEQ ID NO: 2, a galactose-1-phosphate uridyl transferase having at least 65% identity with the polypeptide of sequence SEQ ID NO: 4, an UDP-glucose-4 epimerase having at least 85% identity with the polypeptide of sequence SEQ ID NO: 6 and a galactose mutarotase having at least 45% identity with the polypeptide of sequence SEQ ID NO: 8 are overexpressed and wherein it is obtainable by the method of any one of claims 1 to 12.

14. Use of a mutant *Yarrowia* yeast strain as defined in claim 13 for producing lipids or citric acid from a medium comprising D-galactose.

15. A method of producing lipids or citric acid, comprising a step of growing a mutant oleaginous yeast strain as defined in claim 13 on a medium comprising D-galactose.

16. An isolated enzyme selected from the group consisting of:
- a galactokinase (E.C 2.7.1.6) of SEQ ID NO: 2, 10, 11, 12 or 13,
- a galactose-1-phosphate uridyl transferase (E.C 2.7.7.12) of SEQ ID NO: 4, 15, 16 or 17,
- an UDP-glucose-4 epimerase (E.C 5.1.3.2) of SEQ ID NO: 6, 20, 21, 22 or 23, and
- a galactose mutarotase (E.C 5.1.3.3) of SEQ ID NO: 8, 27, 28 or 29.
